# EUROPEAN PATENT SPECIFICATION

(11) **EP 2 582 862 B1**
(45) Date of publication and mention of the grant of the patent: **13.04.2016**
(21) Application number: 11796472.6
(22) Date of filing: 16.06.2011
(51) Int. Cl.: C40B 40/10, C40B 30/04, C07K 16/00, G01N 33/68

(54) **METHODS AND SYSTEMS FOR GENERATING, VALIDATING AND USING MONOCLONAL ANTIBODIES**
VERFAHREN UND SYSTEME ZUR ERZEUGUNG, VALIDIERUNG UND VERWENDUNG MONOKLONALER ANTIKÖRPER
PROCÉDÉS ET SYSTÈMES PERMETTANT DE GÉNÉRER, DE VALIDER ET D'UTILISER DES ANTICORPS MONOCLONAUX

(30) Priority: 16.06.2010 US 355329 P
(43) Date of publication of application: 24.04.2013
(73) Proprietor: CDI Laboratories Inc., Mayaguez, PR 00680 (US); Johns Hopkins University, Baltimore, MD 21201 (US)
(72) Inventor: BOEKE, Jef, D., Baltimore, MD 21210 (US); ZHU, Heng, Towson, MD 21286 (US); BLACKSHAW, Seth, Baltimore, MD 21212 (US); EICHINGER, Daniel, J., Pleasantville, NY 10570 (US); PINO, Ignacio, Mayaguez 00682 (PR)
(74) Representative: Russell, Tim
(86) International application number: PCT/US2011/040789
(87) International publication number: WO 2011/159959

(56) References cited:
- WO-A1-2010/011337
- WO-A1-2011/038138
- US-A1- 2010 034 807
- HOLT L J ET AL: "BY-PASSING SELECTION: DIRECT SCREENING FOR ANTIBODY- ANTIGEN INTERACTIONS USING PROTEIN ARRAYS", NUCLEIC ACIDS RESEARCH, OXFORD UNIVERSITY PRESS, GB, vol. 28, no. 15, 1 August 2000 (2000-08-01), pages 1-05, XP001056514, ISSN: 0305-1048, DOI: 10.1093/NAR/28.15.E72
- HOOGENBOOM H R: "Selecting and screening recombinant antibody libraries", NATURE BIOTECHNOLOGY, NATURE PUBLISHING GROUP, NEW YORK, NY, US, vol. 23, no. 9, 1 September 2005 (2005-09-01), pages 1105-1116, XP002348401, ISSN: 1087-0156, DOI: 10.1038/NBT1126
- KNAPPIK A ET AL: "Fully synthetic human combinatorial antibody libraries (HuCAL) based on modular consensus frameworks and CDRs randomized with trinucleotides", JOURNAL OF MOLECULAR BIOLOGY, ACADEMIC PRESS, UNITED KINGDOM, vol. 296, no. 1, 11 February 2000 (2000-02-11), pages 57-86, XP004461525, ISSN: 0022-2836, DOI: 10.1006/JMBI.1999.3444
- LUEKING ANGELIKA ET AL: "A nonredundant human protein chip for antibody screening and serum profiling", MOLECULAR & CELLULAR PROTEOMICS, AMERICAN SOCIETY FOR BIOCHEMISTRY AND MOLECULAR BIOLOGY, vol. 2, no. 12, 1 December 2003 (2003-12-01), pages 1342-1349, XP002610538, ISSN: 1535-9476, DOI: 10.1074/MCP.T3000010-MCP200 [retrieved on 2003-09-29]
- MICHAUD G A ET AL: "Analyzing antibody specificity with whole proteome microarrays", NATURE BIOTECHNOLOGY, NATURE PUBLISHING GROUP, NEW YORK, NY, US, vol. 21, no. 12, 9 November 2003 (2003-11-09), pages 1509-1512, XP002377918, ISSN: 1087-0156, DOI: 10.1038/NBT910
- PREDKI P F ET AL: "PROTEIN MICROARRAYS: A NEW TOOL FOR PROFILING ANTIBODY CROSS-REACTIVITY", HUMAN ANTIBODIES, IOS PRESS, AMSTERDAM, NL, vol. 14, no. 1/02, 1 January 2005 (2005-01-01), pages 7-15, XP008059638, ISSN: 1093-2607
- HU SHAOHUI ET AL: "A protein chip approach for high-throughput antigen identification and characterization.", PROTEOMICS JUN 2007, vol. 7, no. 13, June 2007 (2007-06), pages 2151-2161, XP002717370, ISSN: 1615-9853
- Johan Rockberg: "Methods for generation and characterization of monospecific antibodies", , 1 January 2008 (2008-01-01), XP055091382, Retrieved from the Internet: URL:http://urn.kb.se/resolve?urn=urn:nbn:s e:kth:diva-9338 [retrieved on 2013-12-03]
- NILSSON PETER ET AL: "Towards a human proteome atlas: High-throughput generation of mono-specific antibodies for tissue profiling", PROTEOMICS, WILEY - VCH VERLAG, WEINHEIM, DE, vol. 5, no. 17, 1 November 2005 (2005-11-01), pages 4327-4337, XP002572261, ISSN: 1615-9853, DOI: 10.1002/PMIC.200500072 [retrieved on 2005-10-20]
- MICHAUD ET AL.: 'Analyzing antibody specifictiy with whole proteome microarrays.' NATURE BIOTECHNOLOGY vol. 21, no. 12, December 2003, pages 1509 - 1512
- OLSSON ET AL.: 'Human-human hybridomas producing monoclonal antibodies of predefined antigenic specificity.' PROC. NATL. ACAD. SCI. USA vol. 77, no. 9, September 1980, pages 5429 - 5431
- SCHWEMMLE ET AL.: 'The use of peptide arrays for the characterization of monospecific antibody repertoires from polyclonal sera of psychiatric patients suspected of infection by Borna Disease Virus.' MOLECULAR DIVERSITY vol. 8, no. 3, 2004, NETHERLANDS, pages 247 - 250

## Description

### CROSS-REFERENCE

This application claims the benefit of U.S. Provisional Application No. 61/355,329, filed on June 16, 2010.

### STATEMENT AS TO FEDERALLY SPONSORED RESEARCH

This invention was made with the support of the United States government under RR020839 and GM076102 awarded by the National Institutes of Health. The government may have certain rights in the invention.

### BACKGROUND

There is an important need in the biomedical community for a reliable technology that produces the highest possible quality, reproducible antibody reagents. Ongoing improvement of this technology pipeline will directly benefit both the health research community and the larger biomedical community.

The detection or binding of an epitope, antigen or proteins is a large component of the research products industry, serving both academia and the pharmaceutical industry, as well as in the diagnostic and therapeutic industries. The use of antibodies to detect epitope, antigens or proteins can be used to identify new biomarkers and perform many assays. For example, antibodies are also widely used in diagnostic applications, such as for clinical medicine (*e.g.*, ELISA and radioimmunoassay systems). The production of monospecific antibodies has been described (see, for example, Nilsson et al. Proteomics. 2005 Nov; 5(17): 4327-37). Analysis of cells and tissues in pathology laboratories includes the use of antibodies on tissue sections and in flow cytometry analyses. Antibodies are also useful as therapeutics.

The production of antibodies can be costly and time-consuming, thus methods for the high throughput production of antibodies, in particular, highly specific antibodies, that is more cost-effective and less time-consuming is desirable. The present disclosure meets these needs, and provides related advantages.

### SUMMARY

The present invention generally relates to libraries of antibodies, including methods and systems to produce, generate, characterize and utilize antibodies. The antibodies can be highly specific. In some embodiments, the antibodies are monoclonal antibodies. The library can comprise a plurality of different antibodies, where the antibodies are produced by the same platform. The library can comprise a plurality of different antibodies, wherein within the plurality or a subset of the plurality, each antibody is a monospecific antibody; binds a native form of its target protein; is a monoclonal antibody; is an immunoprecipitating antibody; is an IgG antibody or antibody of IgG isotype; has a binding affinity for its target that is similar to that of another antibody of the plurality of antibodies (for example, within at least 1, 2, 3, 4, 5, 6, 7, 8, 9, 10, 11, 12, 13, 14, 15, 16, 17, 18, 19, or 20%); have a binding affinity of at least 10⁻⁷M (K_{D}) (for example, such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10-¹⁶M) for its target; or any combination thereof.

The libraries of antibodies can be produced with high reproducibility. For example, in a first library of antibodies and a second library of antibodies, the first and second library comprises the same set of different antibodies and each antibody of the first library has a binding affinity that is within at least 1, 2, 3, 4, 5, 6, 7, 8, 9, 10, 11, 12, 13,14,15, 16,17,18, 19, or 20% of the binding affinity of the same antibody of the second library.

Another aspect of the present invention is a library of antibodies comprising a plurality of different antibodies, wherein at least 10%, such as at least 20%, 30%, 40%, 50%, 60%, 70%, 80%, 90%, 95%, 99% or 100%, of said plurality is a monospecific antibody. Each monospecific antibody of the plurality can bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

The monospecific antibodies that comprise at least 10%, 20%, 30%, 40%, 50%, 60%, 70%, 80%, 90%, 95%, 99% or 100% of the library can comprise at least 50, 75, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, or 1000 different antibodies, wherein the antibodies or a subset of the antibodies can bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the at least 10% plurality of monospecific antibodies; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

The monospecific antibodies that comprise at least 10%, 20%, 30%, 40%, 50%, 60%, 70%, 80%, 90%, 95%, 99% or 100% of the plurality of different antibodies can bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome, wherein the antibodies or a subset of the antibodies can bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17,18, or 19%, of the binding affinity of another antibody of the at least 10% plurality of monospecific antibodies; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

The monospecific antibodies that comprise at least 10%, 20%, 30%, 40%, 50%, 60%, 70%, 80%, 90%, 95%, 99% or 100% of the plurality of different antibodies can bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5,** wherein the antibodies or a subset of the antibodies can bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

Also provided herein are methods of producing a library of antibodies comprising: (a) immunizing an animal with a plurality of antigens; (b) isolating antibody-generating cells from said animal; (c) isolating a plurality of antibodies from said antibody-generating cells; (d) screening said plurality of antibodies of step c) with a human proteome array, wherein the human proteome array can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome or at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5;** and (e) selecting an antibody that is monospecific for a proteome array. In some embodiments, the antibody selected in step (e) is added to a library, wherein the library can comprise a plurality of different antibodies, where at least 10%, 20%, 30%, 40%, 50%, 60%, 70%, 80%, 90%, 95%, 99% or 100% of the plurality of different antibodies can be produced from the same platform, be a monospecific antibody; bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies produced by the same platform; have a binding affinity of at least 10⁻⁷M (K_{D}), such as at least 10-⁸M, 10⁻⁹M, 10⁻¹⁰M, 10⁻¹¹M, 10⁻¹²M, 10⁻¹³M,10⁻¹⁴M, 10⁻¹⁵M, or 10⁻¹⁶M, for its target; or any combination thereof.

The method can produce a library of antibodies that can comprise at least 50, 75, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, or 1000 different antibodies, wherein the antibodies or a subset of the antibodies can be produced from the same platform, be a monospecific antibody; bind a native form of its target protein; not bind a denatured form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies produced by the same platform; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10-¹⁴M, 10⁻¹⁵M, or 10⁻¹⁶M, for its target; or any combination thereof.

The method can produce a library of antibodies that can bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome, wherein the antibodies or a subset of the antibodies can be produced from the same platform, be a monospecific antibody; bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies produced by the same platform; have a binding affinity of at least 10⁻⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

The method can produce a library of antibodies that can bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5,** wherein the antibodies or a subset of the antibodies can be produced from the same platform, be a monospecific antibody; bind a native form of its target protein; be a monoclonal antibody; be an immunoprecipitating antibody; be an IgG antibody or antibody of IgG isotype; have a binding affinity for its target that is within at least 20%, such as within at least 1, 2, 3, 4, 5, 6, 7, 8, 9, 10, 11, 12, 13, 14, 15, 16, 17, 18, or 19%, of the binding affinity of another antibody of the plurality of antibodies produced by the same platform; have a binding affinity of at least 10-⁷M (K_{D}), such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target; or any combination thereof.

The methods of producing the library of antibodies can further comprise pre-screening the plurality of antibodies from the antibody-generating cells prior to step c), such as by performing immunocytochemistry or determining binding of antibodies from said antibody-generating cells with a mixture comprising one or more target antigens, such as native proteins. The mixture can comprise a crude lysate, one or more cells, one or more proteins, one or peptides, or one or more nucleic acids or a biological sample, wherein the biological sample can be, but is not limited to, a cell mixture, a tissue, blood, sera, plasma, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, tears, cyst fluid, pleural fluid, peritoneal fluid, pericardial fluid, lymph, chyme, chyle, bile, interstitial fluid, menses, pus, sebum, vaginal secretion, mucosal secretion, stool water, pancreatic juice, lavage fluid from sinus cavities, bronchopulmonary aspirate, blastocyl cavity fluid, or umbilical cord blood.

The methods of producing the library of antibodies can comprise immunizing an animal with a plurality of antigens, wherein the plurality of antigens can comprise a crude lysate, one or more cells, one or more proteins, one or more peptides, or one or more nucleic acids. The plurality of antigens can also comprise a biological sample, wherein the biological sample can be, but is not limited to, a mixture of cells, a tissue, blood, sera, plasma, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, tears, cyst fluid, pleural fluid, peritoneal fluid, pericardial fluid, lymph, chyme, chyle, bile, interstitial fluid, menses, pus, sebum, vaginal secretion, mucosal secretion, stool water, pancreatic juice, lavage fluid from sinus cavities, bronchopulmonary aspirate, blastocyl cavity fluid, or umbilical cord blood. In some embodiments,the plurality of antigens comprises at least 11,000 different antigens, such as at least 12,000, 13,000, 14,000, 15,000, 16,000, 17,000, 18,000, 19,000, or 20,000 different antigens. In some embodiments, the plurality of antigens comprises at least 0.5% of the human proteome, such as at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100%, of the human proteome.

The antibody-generating cells in producing the library of antibodies can be B-cells. The method of producing a library of antibodies can further comprise immobilizing the antibody to a substrate, wherein the substrate can be planar or a particle, comprise a solid or porous material, or any combination thereof. The antibody can be reversibly or irreversibly immobilized to the substrate.

Also provided herein are methods of identifying an antibody monospecific for a human protein comprising: (a) contacting a plurality of antibodies with a human proteome array; (b) determining binding between said plurality of antibodies and the targets present on the human proteome array; and (c) identifying an antibody as monospecific when said antibody is monospecific for a single target on the proteome array, wherein the human proteome array can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome or at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5.**

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

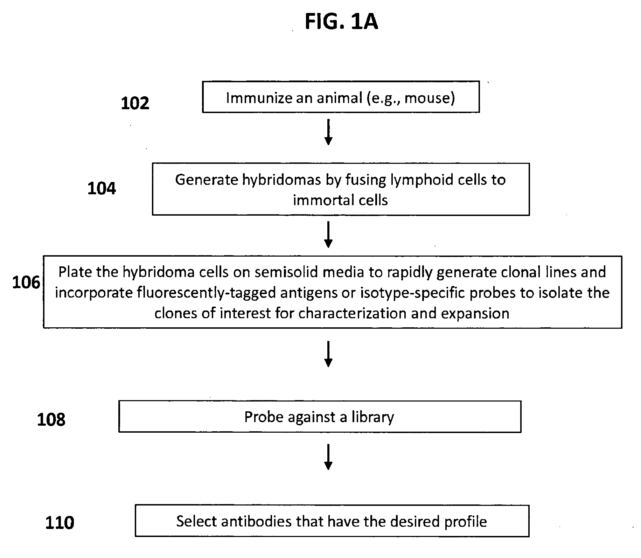

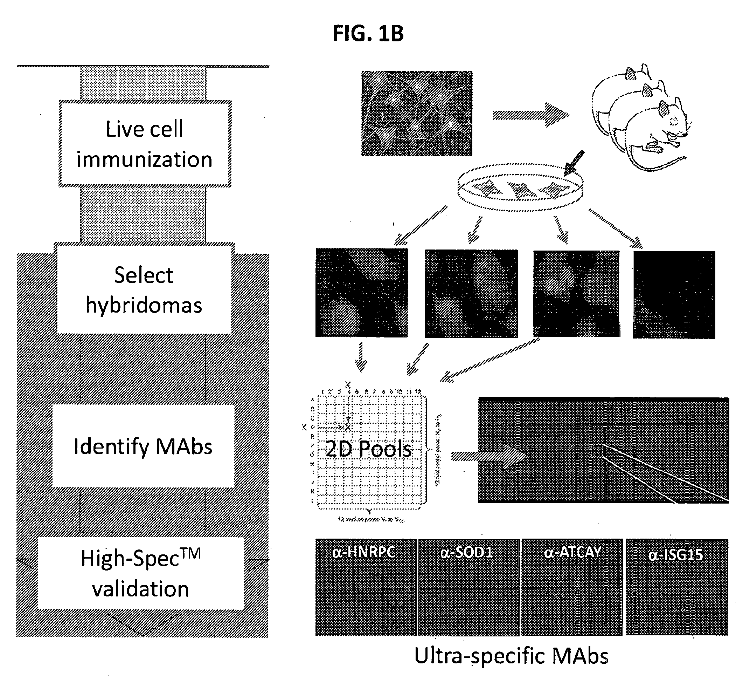

**FIG. 1** depicts **A.** a method for selecting antibodies by immunizing an animal, generating hybridomas from the antimal, plating the hybridomas, probing the antibodies produced against a library, and selecting antibodies with a desired profile; and **B.** method of producing and validating ultra-specific monoclonal antibodies (MAbs) by live cell immunization, hybridoma selection, MAb identification, and validation.

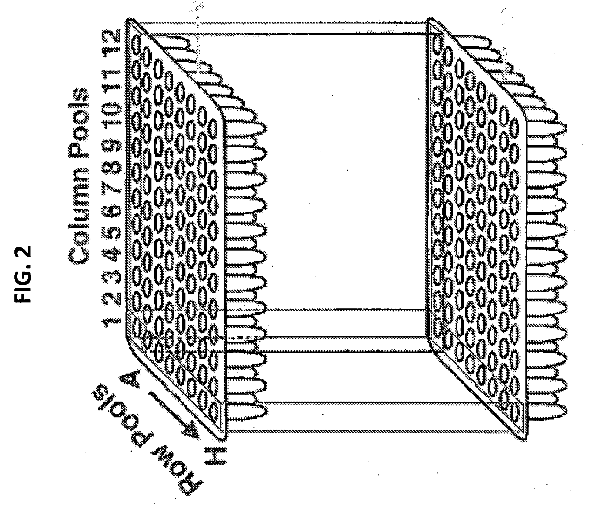

**FIG. 2** depicts a 3-D pooling strategy.

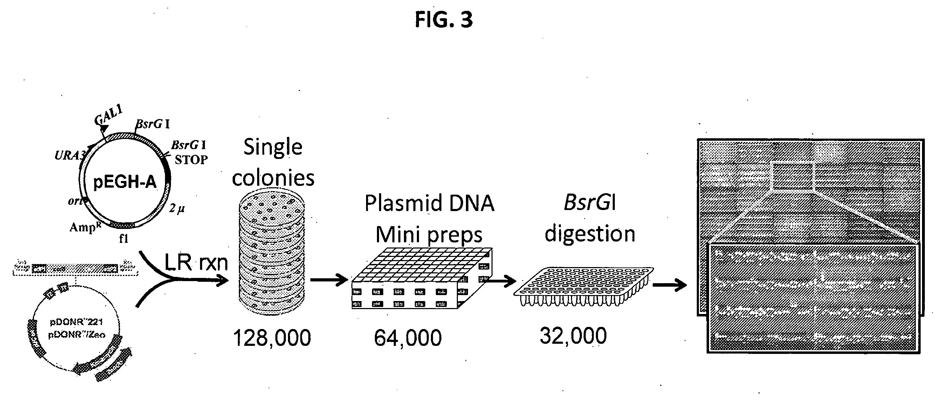

**FIG. 3** depicts a flow chart of LR recombination cloning into a destination vector, with *BsrGI* digestion of 8,064 clones.

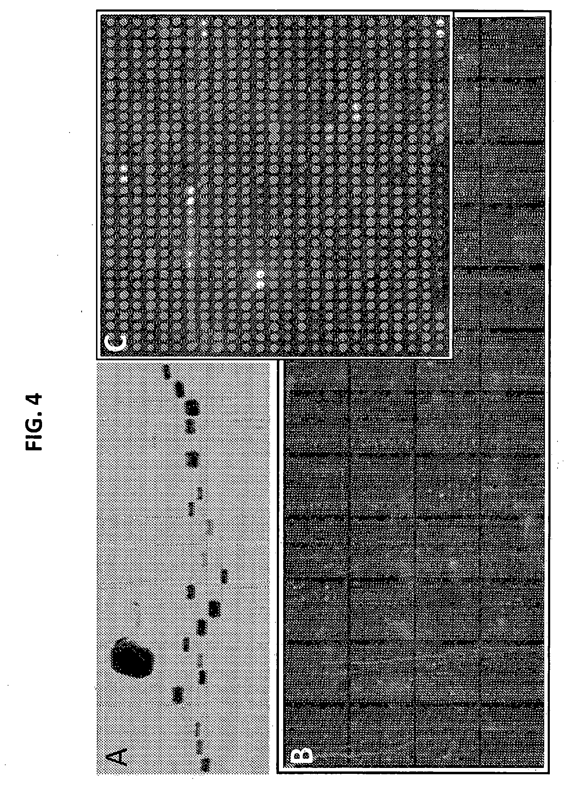

**FIG. 4** depicts fabrication of a human proteome chip. **A.** 17,000 human proteins were purified in the form of N-teminal GST-Hisx6 fusion proteins and the quality was monitored by silver staining and immunoblot analysis. **B.** 17,000 purified human proteins and controls were spotted in duplicate on a single glass slide. The immobilized proteins were visualized with anti-GST. **C.** Higher-powerview of **B.** indicates the array is of good quality.

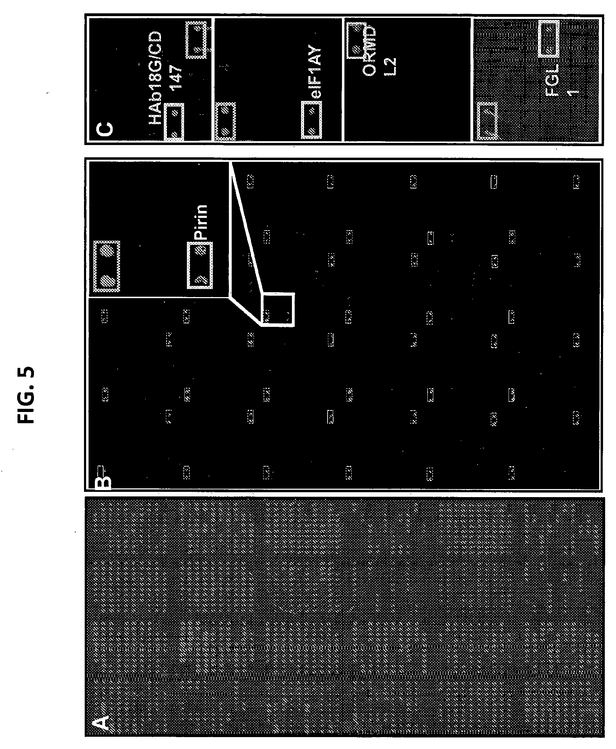

**FIG. 5** depicts monoclonal antibody (mAb) quality profiling on a human protein chip. **A.** GST image of a human protein chip of 1,058 proteins. **B.** A monoclonal antibody (mAb) generated against pirin was tested against 1,058 proteins on the chip and only recognized pirin. The purple boxes indicate the mouse IgG landmarks. **C.** Four other mAbs also showed similar specificity as the anti-pirin mAb.

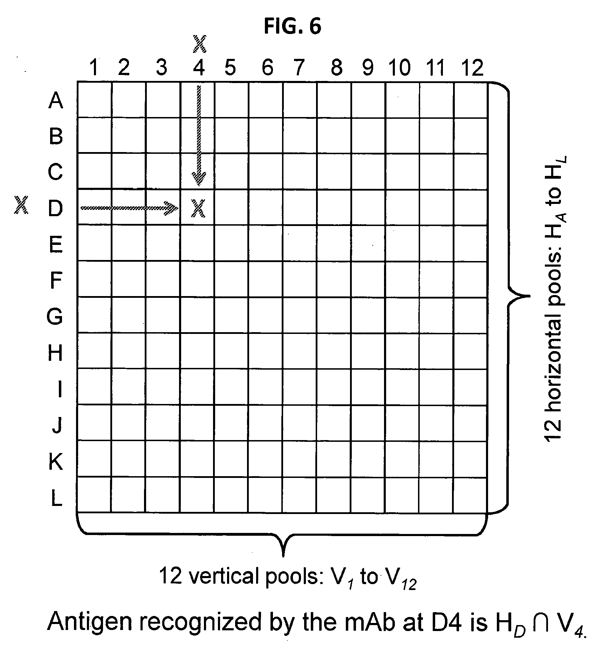

**FIG.** 6 depicts pooling strategies facility protein microarray analysis of mAb specificity. Hybridoma supernatants (A-L) are combined in "vertical" and "horizontal" pools, the binding specificity of which is then tested using the human protein microarrays. The specificity of individual mAbs is identified by determining which proteins on the microarray are bound by which combination of vertical and horizontal pools. In the example shown here, mAb pools recognizing a specific protein are indicated by Xs. The results indicate that only pools containing mAb "D" recognizes the antigen.

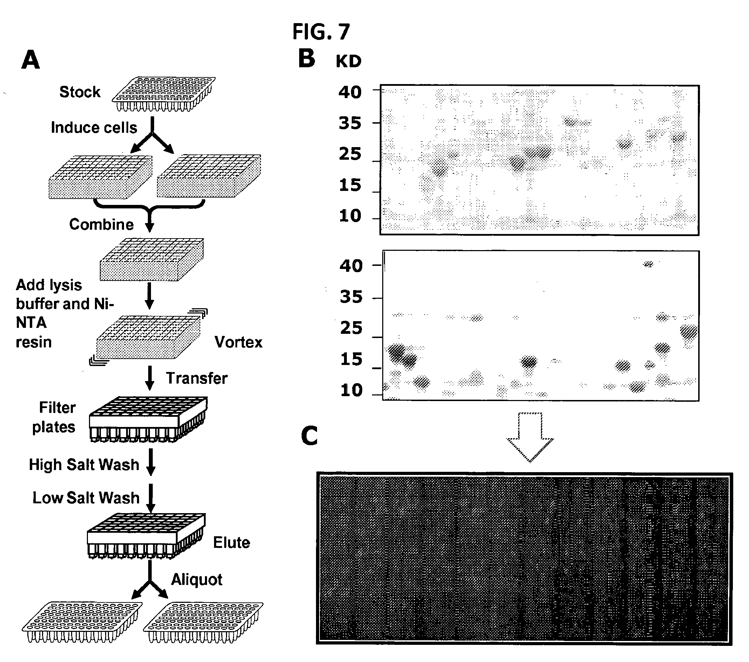

**FIG. 7** depicts a high-throughput protein puritication protocol developed for bacteria. **A.** Scheme of the protocol. **B.** Coomassie staining of purified proteins from 1.5mL *E. coli* culture. **C.** Fabrication of an *E. coli* proteome chip.

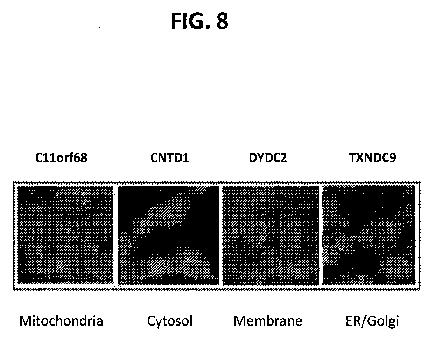

**FIG. 8** depicts subcellular localization of four human proteins using ICC. C11orf68, CNTD1, DYDC2, and TXNDC9 that were not known for their subcellular localization have now been localized to the mitochondria, cytosol, plasma membrane, and ER/Golgi, respectively.

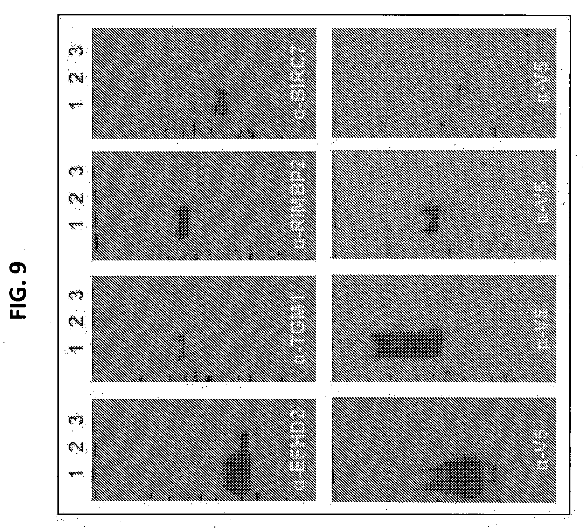

**FIG. 9** depicts validation and characterization of mMAbs (monospecific monoclonal antibodies). Cells were transfected with either V5-tagged antigen constructs (Lane 1) or empty vector (Lane 2), or co-transfected with antigen constructs with their corresponding shRNA constructs (Lane 3). Two days after transfection, cells were lysed and immuno-blotted with each corresponding mMAb (upper panel). As comparison, the same blots were stripped and re-probed with anti-V5 antibody (lower panel).

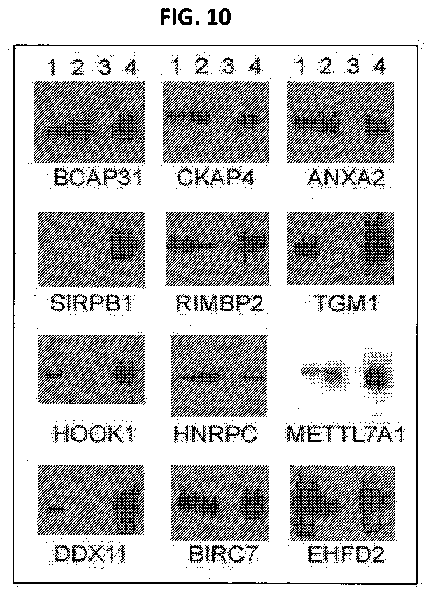

**FIG. 10** depicts many mMAbs are IP-grade. HeLa cells transfected with V5-tagged antigen constructs were immunoprecipitated with their corresponding mMAbs and immunoblotted with anti-V5 antibodies. Lane 1: Input; Lane 2: Antigens immunoprecipitated with mMAbs; Lane 3: IgG negative controls; Lane 4: Anti-V5 positive ocontrols.

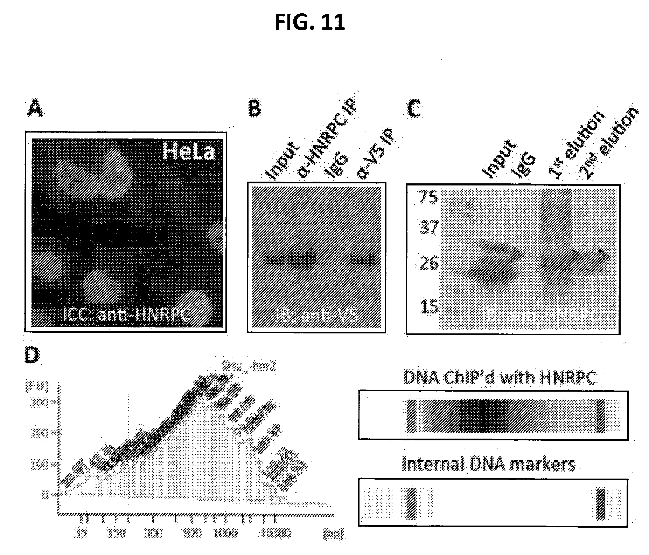

**FIG. 11** depicts characterization of anti-HNRPC mMAbs. A. ICC analysis using anti-HNRPC clearly shows a distinct nuclear localization. **B.** A single band of V5-tagged HNRPC is visualized after transfected HeLa cells were IPd with anti-HNRPC and immunoblotted with anti-V5. Anti-mouse IgG and anti-V5 antibodies were used as negative and positive controls, respectively. C. ChIP was successfully performed using the

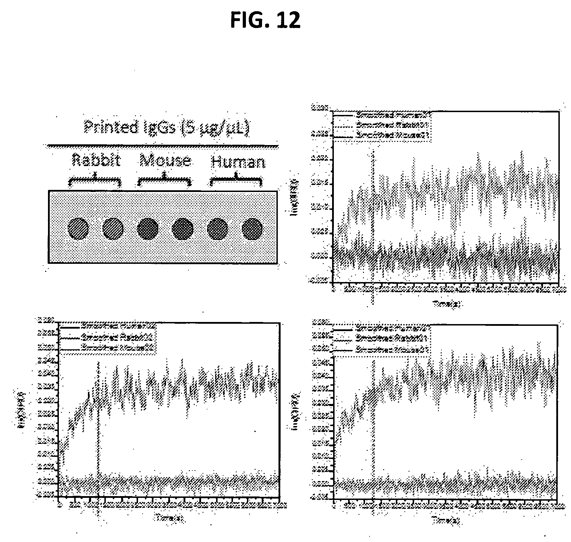

**FIG. 12** depicts sequential measurements of mAb affinity using the oblique-incidence reflectivity difference (OIRD) method. Red, blue, and black curves represent smoothened and averaged Im{OIRD} values obtained by probing a pilot IgG microarray with anti-rabbit, -mouse, and -human secondary antibodies, respectively. Dotted red lines indicate estimated starting point of the saturation phase.

### DETAILED DESCRIPTION

The present invention generally relates to libraries of antibodies, including methods and systems to produce, generate, characterize, and utilize antibodies. The antibodies can be highly specific. The library can comprise of a plurality of different antibodies, where the antibodies are produced by the same platform. The library can comprise plurality of different antibodies, wherein within the plurality or a subset of the plurality, each antibody is a monospecific antibody; binds a native form of its target protein; is a monoclonal antibody; is an immunoprecipitating antibody; is an IgG antibody or antibody of IgG isotype; has a binding affinity for its target that is similar to that of another antibody of the plurality of antibodies, have a binding affinity of at least 10⁻⁷M (K_{D}) for its target; or any combination thereof.

### Platform

The library of antibodies can comprise a plurality of different antibodies produced by the same platform. In one embodiment, the library of antibodies comprises a plurality of different antibodies wherein at least 10% of said plurality is produced by the same platform. For example, the library can comprise a plurality of different antibodies wherein at least 20%, 30%, 40%, 50%, 60%, 70%, 80%, 90%, 95%, 99% or 100% of said plurality are antibodies produced by the same platform.

Antibodies are considered to be produced by the same platform when a first antibody and second antibody is produced from the same protocol. For example, the plurality of antibodies can be produced by the platform as depicted in **FIG. 1A** and/or **B.** In one embodiment, as depicted in **FIG. 1A****,** in step **102,** an animal can be immunized with a plurality of antigens. The animal can be a non-human animal, such as a bovine, avian, canine, equine, feline, ovine, porcine, or primate animal. The animal can be a mammal, such as a mouse, rat, rabbit, cat, dog monkey, or goat.

The plurality of antigens can comprise purified or non-purified samples, such as purified or non-purified cells, proteins, peptides or nucleic acids. In another embodiment, the plurality of antigens can comprise non-purified samples, such as a crude lysate or unpurified cell, protein, peptide, or nucleic acid samples.

The plurality of antigens can comprise a biological sample. For example, the plurality of antigens can comprise a single cell or multiple cells, proteins, representative peptides, or tissues from an organism. The organism can be a human or non-human. The non-human organism can be a mammal, such as a mouse, rat, rabbit, cat, dog, monkey, or goat. The biological sample can be a tissue, blood, sera, plasma, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, tears, cyst fluid, pleural fluid, peritoneal fluid, pericardial fluid, lymph, chyme, chyle, bile, interstitial fluid, menses, pus, sebum, vaginal secretion, mucosal secretion, stool water, pancreatic juice, lavage fluid from sinus cavities, bronchopulmonary aspirate, blastocyl cavity fluid, or umbilical cord blood. In one embodiment, the biological sample can be substantially depleted of a common serum protein, such as, but not limited to, albumin or IgG. Depletion can comprise filtration, fractionation, or affinity purification.

The biological sample can comprise a cell, such as a stem cell, undifferentiated cell, differentiated cell, or cell from a diseased subject or subject with a specific condition. The disease or condition can be a cancer, inflammatory disease, immune disease, autoimmune disease, cardiovascular disease, neurological disease, infectious disease, metabolic disease, or perinatal condition. For example, the cancer can be breast cancer, ovarian cancer, lung cancer, colon cancer, colorectal cancer, prostate cancer, melanoma, pancreatic cancer, brain cancer hematological malignancy, hepatocellular carcinoma, cervical cancer, endometrial cancer, head and neck cancer, esophageal cancer, gastrointestinal stromal tumor (GIST), renal cell carcinoma (RCC) or gastric cancer.

The plurality of antigens can also comprise samples produced in bacteria (such as, but not limited to, *E. coli*)*,* yeast, mammalian, or insect cells, such as proteins being overexpressed by the organisms. The antigens, purified or non-purified, such as in the form of an overexpressing cell or cell mixture, expressing the antigens of interest, can be used to immunize the animals

The plurality of antigens can comprise at least 11,000 different antigens, such as at least 12,000, 13,000, 14,000, 15,000, 16,000, 17,000, 18,000, 19,000, or 20,000 different antigens.

In step **104,** antibody-generating cells, such as lymphoid cells, from the animal can then be isolated for producing a plurality of antibodies. The antibody-generating cell can be used to generate a hybridoma. The antibody-generating cell can be a B-cell. The B-cell can be fused to a myeloma cell to create a hybidoma. Examples of myeloma cells include, but are not limited to, NS-1, P3U1, SP2/0, AP-1 and the like cells.

In step **106,** the antibody-producing cells, such as hybridoma cells, can then be used to generate clonal lines. For example, hybridoma cells can be placed on semisolid media to rapidly generate clonal lines. Fluorescently-tagged antigens or isotype-specific probes can be used to isolate the clones of interest for characterization and expansion.

In step **108,** the clones of interest are probed against a library of antigens, to detect the antigen each antibody recognizes and at the same time gain information as to which antigens it does not react to (eliminate false positives). The library of antigens can represent at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. The library of antigens can represent a substantial portion or an entire organism's proteome, such as a bacterial, viral, fungal proteome. The library of antigens can represent a substantial portion or an entire proteome of an insect or mammal, such as a mouse, rat, rabbit, cat, dog, monkey, goat, or human. For example, the library of antigens can comprise at least 11,000, 12,000, 13,000, 14,000, 15,000, 16,000, 17,000, 18,000, 19,000, or 20,000 different antigens. The library of antigens can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5.** The library of antigens can also comprise a fusion protein library that represents an organism's proteome in unpurified form, such as a collection of overexpressing cells (E.coli, yeast, mammalian, insect, or other organism).

Probing can be performed by arraying the cells overexpressing an antigen, such as a fusion protein, or cells of different cell lines (such as cancer cell lines), and testing the hybridoma supernatants against the the arrayed cells. The arrayed cells can be permeabilized. Probing can also be performed by fluorescence flow cytometry and the target identified by PCR or sequence analysis of recombinant DNA in the cell.

In step **110,** antibodies that have the desired profile. The antibodies that are selected can be highly specific monoclonal antibodies that recognize only one target and do not cross react with the other targets in the proteome library of that organism. The cell lines that secrete these monoclonal antibodies can be expanded. The antibodies produced by the same platform or protocol can be used to produce or form a library of antibodies.

Antibodies can also be produced by the same platform when the antibodies are produced by the same method or protocol, such as described further below, in methods of producing a library.

### Monospecificity

The library of antibodies can comprise a plurality of different antibodies, each antibody having a particular binding specificity for its target. For example, the library of antibodies can comprise a plurality of different antibodies, wherein the antibodies are monospecific. In one embodiment, the library of antibodies comprises a plurality of different antibodies wherein at least 10%, 20%, 30%, 40%, 50%, 60%, 70%, 80%, 90%, 95%, 99% or 100% of the plurality of antibodies is monospecific.

An antibody is monospecific if the antibody has an A value of greater than 6 and an S value of greater than 3 for a protein or antigen when incubated on array comprising the antigens listed on **Table 5.** The number of standard deviations above mean signal intensity across the entire array for an antibody is a value termed A. The difference between the top signal and the second-highest signal on the array when the signal intensities are rank ordered for the antibody against the entire array is the S value. For example, the A and *S* value can be calculated by combining individual supernatants from antibody-producing cells or hybridomas into sets of 12x12 two-dimensional pools, and these pools are incubated on an array (such as a human proteome microarrays), and the antibodies are labeled (such as by a Cy5-coupled anti-IgG secondary antibody, or any other method of detecting an antibody). Following washing and scanning, the signal intensity for each spot (representing antibody binding to a protein or antigen on the array) as the ratio of foreground to background signal. The number of standard deviations above mean signal intensity across the entire array is a value termed A. Duplicate spots (for each duplicate pair of proteins or antigens) for which A >3 are flagged and results deconvoluted to identify proteins or antigens that are present at the intersection of a single horizontal and single vertical pool, and thus recognized by an individual monoclonal antibody. Each candidate highly specific monoclonal antibody (ie. A >3) is tested individually against the entire array, and A measured for each spotted protein. The signal intensities are then rank ordered and the difference between the top signal and the second-highest signal on the array is calculated, giving the S value. Monoclonal antibodies in which A>6 and S>3 are identified as monospecific monoclonal antibodies (mMAbs). Dispecific monoclonal antibodies (dMAbs) bind intensely to two different proteins on the array and have A>6 and *S<3.*

In some embodiments, the mMAbs can have an A value of greater than 6, 7, 8, 9, 10, 20, 30, 40, 50, 60, 70, 80, 90, or 100 and/or an S value of greater than 3, 4, 5, 6, 7, 8, 9, 10, 20, 30, 40, 50, 60, 70, 80, 90, or 100.

In one embodiment, the array for determining the monospecificity of an antibody is comprises an organism's proteome library, such as at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. The library of antigens can represent a substantial portion or an entire organism's proteome, such as a bacterial, viral, fungal proteome. The library of antigens can represent a substantial portion or an entire proteome of an insect or mammal, such as a mouse, rat, rabbit, cat, dog, monkey, goat, or human. For example, an organism's proteome library can comprise at least 11,000, 12,000, 13,000, 14,000, 15,000, 16,000, 17,000, 18,000, 19,000, or 20,000 different antigens. The organism's proteome library can comprise at least 0.5%, 1%, 2%, 3%, 4%, 5%, 6%, 7%, 8%, 9%, 10%, 11%, 12%, 13%, 14%, 15%, 16%, 17%, 18%. 19%, 20%, 25%, 30%, 35%, 40%, 45%, 50%, 60%, 70%, 80%, 90%, or 100% of the human proteins listed in **Table 5.**

### Binding Affinity

The library of antibodies can comprise a plurality of different antibodies have a particular binding affinity its target. For example, the library can comprise a plurality of different antibodies wherein at least 10%, 20%, 30%, 40%, 50%, 60%, 70%, 80%, 90%, 95%, 99% or 100% of the plurality has a particular binding affinity. For example, the plurality of antibodies can have a binding affinity as determined by its dissociation constant (K_{D}), of at least 10⁻⁷M, such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M, for its target protein.

The plurality of antibodies can have a binding affinity as measured by its association rate constant (kₒₙ), wherein the plurality of antibodies have a binding affinity of at least 10⁴ M⁻¹s⁻¹, at least 5 X 10⁴ M⁻¹s⁻¹, at least 10⁵ M⁻¹s⁻¹, at least 5 X 10⁵ M⁻¹s⁻¹ at least 10⁶ M⁻¹s⁻¹, at least 5 X 10⁶ M⁻¹s⁻¹, at least 10⁷ M⁻¹s⁻¹, at least 5 X 10⁷ M⁻¹s⁻, or at least 10⁸ M⁻¹s⁻¹. The plurality of antibodies can have a binding affinity as measured by its dissociation rate constant (k_{off}), wherein the plurality of antibodies have a binding affinity of less than 10³ M⁻¹s⁻¹, less than 5 X 10³ M⁻¹s⁻¹, less than 10⁴ M⁻¹s⁻¹, less than 5 X 10⁴ M⁻¹s⁻¹, less than 10⁵ M⁻¹s⁻¹, less than 5 X 10⁵ M⁻¹s⁻¹, less than 10⁶ M⁻¹s⁻¹, less than 5 X 10⁶ M⁻¹s⁻¹, less than 10⁷ M⁻¹s⁻¹, less than 5 X 10⁷ M⁻¹s⁻, or less than 10⁸ M⁻¹s⁻¹. less than 5 X 10⁷ M⁻¹s⁻ , less than 10⁸ M⁻¹s⁻¹, less than 5 X 10⁸ M⁻¹s⁻, less than 10⁹ M⁻¹s⁻¹, less than 5 X 10⁹ M⁻¹s⁻, or less than 10¹⁰ M⁻¹s⁻¹.

The binding affinity can be determined by surface plasmon resonance, chromatography or any other methods known in the art. Binding affinity can also be determined by optically, such as by using real-time and/or label-free methods of detecting biomolecule interactions. In one embodiment, oblique-incidence reflectivity different (OIRD) is used.

The library of antibodies can comprise a plurality of different antibodies that binds native form of its target protein. For example, the library can comprise a plurality of different antibodies wherein at least 10%, 20%, 30%, 40%, 50%, 60%, 70%, 80%, 90%, 95%, 99% or 100% of the plurality binds a native form of its target protein. In one embodiment, the plurality of antibodies that binds a native form of its target protein does not bind a denatured form of its target protein under the same binding conditions.

The library of antibodies can comprise a plurality of different antibodies, wherein one or more antibodies of the library have a binding affinity for its target that is similar to the binding affinity of another antibody of the plurality. For example, the library of antibodies can comprise a first antibody and a second antibody, wherein the first antibody has a binding affinity for a first protein that similar to the binding affinity of the second antibody to a second protein. One antibody of the library can have a binding affinity for its target that is within at least 1%, 2%, 3%, 4%, 5%, 6%, 7%, 8%, 9%, 10%, 11%, 12%, 13%, 14%, 15%, 16%, 17%, 18%, 19%, or 20% of the binding affinity of one or more other antibodies of the library. The first antibody of the library can have a binding affinity for its target that is within at least 20% of the binding affinity of one or more other antibodies of the library. In one embodiment, the library of antibodies can comprise a plurality of different antibodies, wherein at least 10%, 20%, 30%, 40%, 50%, 60%, 70%, 80%, 90%, 95%, 99% or 100% of the different antibodies have a binding affinity for its target proteins that is within at least 20% of the binding affinity of the rest of the plurality of different antibodies to their respective target protein.

### Proteome

Also provided herein is a library of antibodies comprising a plurality of different antibodies that binds a portion of an organism's proteome. The plurality of different antibodies can bind at least 0.5%, 1 %, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. For example, the plurality of different antibodies can bind at least 11,000, 12,000, 13,000, 14,000, 15,000, 16,000, 17,000, 18,000, 19,000, or 20,000 proteins of an organism. The proteome can be a bacterial, viral, fungal proteome. The proteome can be of an insect or mammal, such as a mouse, rat, rabbit, cat, dog, monkey, goat, or human. In some embodiments, the proteome is a human proteome.

For example, the plurality of different antibodies can bind at least 0.5% of the human proteome. In one embodiment, the plurality of different antibodies can bind at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome. The plurality of different antibodies can bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5.**

### Other Antibody Characteristics

The library of antibodies can also comprise a plurality of different antibodies, wherein the antibodies are IgG antibodies (e.g. antibodies of IgG isotype). For example, a library of antibodies can comprise a plurality of different antibodies, wherein at least 10%, 20%, 30%, 40%, 50%, 60%, 70%, 80%, 90%, 95%, 99% or 100% of the antibodies is an IgG antibody or antibody of IgG isotype.

The library of antibodies can also comprise a plurality of different antibodies, wherein the antibodies are immunoprecipitating antibodies. For example, a library of antibodies can comprise a plurality of different antibodies, wherein at least 10%, 20%, 30%, 40%, 50%, 60%, 70%, 80%, 90%, 95%, 99% or 100% of the antibodies is an immunoprecipicating antibody. An immunoprecipicating antibody is an antibody that can immunoprecipitate a target protein from a cell homogenate as compared to a no antibody negative control and an anti-V5 antibody positive control. Detection of the imnmnoprecipitated target can be by Western blot. The immunopreiciptation can be carried out with cleared cell lysate and approximately 2µg of antibody, followed by incubation for 2 hours at 4°C, then addition of a substrate to bind any antibody-protein complex (such as protein-G Dynabead), and then incubation for an additional 2 hours at 4°C. After incubation the substrate is washed, such as with ice cold TBST twice, the substrate transferred to a new reaction vessel, washed again with ice cold TBST, before being subjected to SDS-PAGE and Western blot analysis.

### Biological Pathways

In one aspect, the present invention relates to a library of antibodies comprising a plurality of antibodies that are specific to antigens of a common pathway. Antigens belong to a common pathway when they share one or more attributes in common in a gene ontology, a collection that assigns defined characteristics to a set of genes and their products. The ontology administered by the Gene Ontology ("GO") Consortium is particularly useful in this regard. Antigens belonging to common pathways can be identified by searching a gene ontology, such as GO, for genes sharing one or more attributes. The common attribute could be, for example, a common structural feature, a common location, a common biological process or a common molecular function.

The wealth of information that exists in published, peer-reviewed literature concerning the function of human genes and proteins has been organized and curated using a coordinated system of controlled vocabulary that is administered by the Gene Ontology (GO) Consortium (http://www.geneontology.org/). Of the approximately 40,000 transcribed units in the human genome, approximately 20,000 of those code for annotated proteins, and approximately 14,000 of those proteins have a functional annotation in the GO database. The functional annotations contained in the GO database are organized in a hierarchical manner, and it is possible to access this information from the GO database and search for all of the genes in the human genome that are annotated to be involved in the same biological process, reside in the same cellular component, or perform the same molecular function.

In some embodiments, antigens in a common pathway are the expression product of genes involved in the same biological process or molecular function as annotated by a gene ontology. One example of this are genes involved in the response to DNA damage. Another example is gene products of transcription factors, such as of a particular tissue, cell type or organ. One example is gene products of transcription factors of the brain.

In some embodiments, antigens in a common pathway are gene products of genes which are all bound by the same transcription factor protein, complex of transcription factor proteins, other nucleic acid binding proteins, or other molecule. These interactions may occur in a living cell (in vivo) or in a solution of purified molecules (in vitro). For instance, all of the gene products of the genes bound by the hypoxia inducible transcription factor protein.

In some embodiments, antigens in a common pathway are the gene products of genes whose transcript levels or proteins levels change upon treatment or exposure to the same stimulus and are thus coregulated. For example all of the antigens that are induced or repressed upon treatment to UV radiation.

In some embodiments, antigens in a common pathway are antigens that contain similar sequence features. These features may be a DNA sequence motif, collection of DNA sequence motifs, or enrichment of higher order sequence features that are distinguishable from a background model of random genomic sequences. As used herein, a sequence motif is a string of 2 or more nucleic acid bases (A, T, C, or G). A DNA sequence motif can either be defined by a consensus sequence or a probability matrix where the identity of each base at each position of a motif is defined as a probability.

In some embodiments, antigens in a common pathway could be the gene products of genes whose sequences, transcripts or proteins are connected via metabolic transformations and/or physical protein-protein, protein-DNA and protein-compound interactions. Enzymes catalyze these reactions, and often require dietary minerals, vitamins and other cofactors in order to function properly. Because of the many chemicals that may be involved, pathways can be quite elaborate.

In some embodiments, the members of the pathway share a common structural or functional attribute. For example, the proteins could share a common sequence motif, such as a zinc finger or a transmembrane region.

In some embodiments, the antigens in a common pathway belong to the same signal transduction pathway. Typically, in biology signal transduction refers to any process by which a cell converts one kind of signal or stimulus into another, most often involving ordered sequences of biochemical reactions inside the cell that are carried out by enzymes, activated by second messengers resulting in what is thought of as a signal transduction pathway. Usually, signal transduction involves the binding of extracellular signaling molecules (or ligands) to cell-surface receptors that face outwards from the plasma membrane and trigger events inside the cell. Additionally, intracellular signaling cascades can be triggered through cell-substratum interactions, as in the case of integrins which bind ligands found within the extracellular matrix. Steroids represent another example of extracellular signaling molecules that may cross the plasma membrane due to their lipophilic or hydrophobic nature. Many steroids, but not all, have receptors within the cytoplasm and usually act by stimulating the binding of their receptors to the promoter region of steroid responsive genes. Within multicellular organisms there are a diverse number of small molecules and polypeptides that serve to coordinate a cell's individual biological activity within the context of the organism as a whole. Examples of these molecules include hormones (e.g. melatonin), growth factors (e.g. epidermal growth factor), extra-cellular matrix components (e.g. fibronectin), cytokines (e.g. interferon-gamma), chemokines (e.g. RANTES), neurotransmitters (e.g. acetylcholine), and neurotrophins (e.g. nerve growth factor).

In addition to many of the regular signal transduction stimuli listed above, in complex organisms, there are also examples of additional environmental stimuli that initiate signal transduction processes. Environmental stimuli may also be molecular in nature or more physical, such as, light striking cells in the retina of the eye, odorants binding to odorant receptors in the nasal epithelium, bitter and sweet tastes stimulating taste receptors in the taste buds, UV light altering DNA in a cell, and hypoxia activating a series of events in cells. Certain microbial molecules e.g. viral nucleotides, bacterial lipopolysaccharides, or protein antigens are able to elicit an immune system response against invading pathogens, mediated via signal transduction processes.

Activation of genes, alterations in metabolism, the continued proliferation and death of the cell, and the stimulation or suppression of locomotion, are some of the cellular responses to extracellular stimulation that require signal transduction. Gene activation leads to further cellular effects, since the protein products of many of the responding genes include enzymes and transcription factors themselves. Transcription factors produced as a result of a signal transduction cascade can in turn activate yet more genes. Therefore an initial stimulus can trigger the expression of an entire cohort of genes, and this in turn can lead to the activation of any number of complex physiological events. These events include, for example, the increased uptake of glucose from the blood stream stimulated by insulin and the migration of neutrophils to sites of infection stimulated by bacterial products.

Most mammalian cells require stimulation to control not only cell division, but also survival. In the absence of growth factor stimulation, programmed cell death ensues in most cells. Such requirements for extra-cellular stimulation are necessary for controlling cell behavior in both the context of unicellular and multi-cellular organisms. Signal transduction pathways are so central to biological processes that it is not surprising that a large number of diseases have been attributed to their dysregulation.

In some embodiments, the invention provides methods and compositions including a library comprising a plurality of antibodies that are specific to antigens that are part of an oncology pathway. Antigens in an oncology pathway are those gene products of genes involve in the development of hyperplasia, neoplasia and/or cancer. Examples of oncology pathways include, but are not limited to, hypoxia, DNA damage, apoptosis, cell cycle, and p53 pathway.

In some embodiments, the invention provides methods and compositions including a library comprising a plurality of antibodies that are specific to antigens that that are part of a membrane pathway. Examples of membrane pathways include, but are not limited to, transport proteins, G-coupled receptors, ion channels, cell adhesion proteins and receptors pathways.

In some embodiments, the invention provides methods and compositions including a library comprising a plurality of antibodies that are specific to antigens that are part of a nuclear receptor pathway. Examples of antigens in a nuclear receptor pathway include, but are not limited to, gene products that are regulated by the glucocorticoid receptor protein, estrogen receptor protein, peroxisome proliferator-activated receptor protein, androgen receptor protein and transporter protein pathways, including ABC and SLC transporters.

In some embodiments, the invention provides methods and compositions including a library comprising a plurality of antibodies that are specific to antigens that are part of a neuronal pathway. Examples of antigens in a neuronal pathway include, but not limited to, gene products of genes expressed in neurons such as neurotransmitters and cell adhesion proteins.

In some embodiments, the invention provides methods and compositions including a library comprising a plurality of antibodies that are specific to antigens that are part of a vascular pathway. Examples of antigens in a vascular pathway include, but not limited to, antigens involved in angiogenesis, lipid metabolism, and inflammation.

In some embodiments, the invention provides methods and compositions including a library comprising a plurality of antibodies that specific to antigens that are part of a signaling pathway. Examples of antigens in a signaling pathway include, but are not limited to, gene products involved in cell-to-cell signaling, hormones, hormone receptors, cAMP response, and cytokines.

In some embodiments, the invention provides methods and compositions including a library comprising a plurality of antibodies that specific to antigens that are part of an enzymatic pathway. Examples of antigens in a enzymatic pathway include, but are not limited to, gene products of genes involved in glycolysis, anaerobic respiration, Krebs cycle / Citric acid cycle, Oxidative phosphorylation, fatty acid oxidation (β-oxidation), gluconeogenesis, HMG-CoA reductase pathway, pentose phosphate pathway, porphyrin synthesis (or heme synthesis) pathway, urea cycle, photosynthesis (plants, algae, cyanobacteria) and chemosynthesis (some bacteria).

The present invention also provides a library comprising a plurality of antibodies specific to a plurality of antigens in which at least 5%, 10%, 20%, 30%, 40%, 50%, 60%, 70%, 80%, 90%, 99% or 100% of the antiges are part of a common pathway.

In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of an oncology pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a hypoxia pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a DNA-damage pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of an apoptosis pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a cell cycle pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a p53 pathway. In some embodiments, the inventions provides a library comprising a plurality of antibodies specific to a plurality of antigens in which at least 5%, 10%, 20%, 30%, 40%, 50%, 60%, 70%, 80%, 90%, 99% or 100% of the antigens are differently selected from the group consisting of hypoxia pathway, DNA-damage pathway, apoptosis pathway, cell cycle pathway, and p53 pathway.

In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a membrane bound pathway.

In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a nuclear receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a glucocorticoid receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a peroxisome proliferator-activated receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of an estrogen receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of an androgen receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a cytochrome P450 receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a transporter receptor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are differently selected from the group consisting of glucocorticoid receptor pathway, peroxisome proliferator-activated receptor pathway, estrogen receptor pathway, androgen receptor pathway, cytochrome P450 pathway, and transporter pathways

In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a vascular pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a neuronal pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a transcription factor pathway. In some embodiments, the invention provides a library comprising a plurality of antibodies specific to a plurality of antigens in which at least 5%, 10%, 20%, 30%, 40%, 50%, 60%, 70%, 80%, 90%, 99% or 100% of the antigens are part of a signaling pathway.

The present invention also provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a common pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of an oncology pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a hypoxia pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a DNA-damage pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of an apoptosis pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens are part of a cell cycle pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a p53 pathway in the genome.

In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a membrane bound pathway in the genome.

In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens are part of a nuclear receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a glucocorticoid receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a peroxisome proliferator-activated receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a estrogen receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of an androgen receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a cytochrome P450 receptor pathway in the genome. In some embodiments, the invention provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a transporter receptor pathway in the genome.

The present invention also provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a neuronal pathway in the genome. The present invention also provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a signaling pathway in the genome. The present invention also provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a vascular pathway in the genome. The present invention also provides a library comprising a plurality of antibodies specific to a plurality of antigens in which the library represents at least 5%, 10%, 20%, 30%, 40%, 50%, 60%, 70%, 80%, 90%, 99% or 100% of all the antigens that are part of a transcription factor pathway in the genome.

### Method of Producing a Library of Antibodies

Also provided herein are methods of producing a library of antibodies. In one embodiment, the method comprises (a) immunizing an animal with a plurality of antigens; (b) isolating antibody-generating cells from the animal; (c) isolating a plurality of antibodies from the antibody-generating cells; (d) screening the plurality of antibodies of step c) with a protein array (such as a human proteome array); (e) selecting an antibody that is monospecific for for a single target on the proteome array; and (f) adding the antibody from (e) to the library. In one embodiment, the method can further comprise pre-screening the plurality of antibodies from the antibody-generating cells prior to step c).

The animal can be a non-human animal, such as a bovine, avian, canine, equine, feline, ovine, porcine, or primate animal. The animal can be a mammal, such as a mouse, rat, rabbit, cat, dog monkey, or goat.

The animal can be immunized with a plurality of antigens that can comprise purified or non-purified samples, such as purified or non-purified cells, proteins, peptides or nucleic acids. In another embodiment, the plurality of antigens can comprise non-purified samples, such as a crude lysate or unpurified cell samples, protein samples, peptide samples, or nucleic acid samples. The plurality of antigens can comprise a biological sample. For example, the plurality of antigens can comprise a single cell or multiple cells, proteins, representative peptides, or tissues from an organism, such as a bovine, avian, canine, equine, feline, ovine, porcine, or primate animal. The organism can be a human or non-human. The non-human organism can be a mammal, such as a mouse, rat, rabbit, cat, dog, monkey, or goat.

The biological sample can be a tissue, blood, sera, plasma, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, tears, cyst fluid, pleural fluid, peritoneal fluid, pericardial fluid, lymph, chyme, chyle, bile, interstitial fluid, menses, pus, sebum, vaginal secretion, mucosal secretion, stool water, pancreatic juice, lavage fluid from sinus cavities, bronchopulmonary aspirate, blastocyl cavity fluid, or umbilical cord blood. In one embodiment, the biological sample can be substantially depleted of a common serum protein, such as, but not limited to, albumin or IgG. Depletion can comprise filtration, fractionation, or affinity purification.

The biological sample can comprise a cell, such as a stem cell, undifferentiated cell, differentiated cell, or cell from a diseased subject or subject with a specific condition. The disease or condition can be a cancer, inflammatory disease, immune disease, autoimmune disease, cardiovascular disease, neurological disease, infectious disease, metabolic disease, or perinatal condition. For example, the cancer can be breast cancer, ovarian cancer, lung cancer, colon cancer, colorectal cancer, prostate cancer, melanoma, pancreatic cancer, brain cancer hematological malignancy, hepatocellular carcinoma, cervical cancer, endometrial cancer, head and neck cancer, esophageal cancer, gastrointestinal stromal tumor (GIST), renal cell carcinoma (RCC) or gastric cancer.

The plurality of antigens can also comprise samples produced in bacteria (such as, but not limited to, *E. coli*)*,* yeast, mammalian, or insect cells, such as proteins being overexpressed by the organisms. The antigens, purified or non-purified, such as in the form of an overexpressing cell or cell mixture, expressing the antigens of interest, can be used to immunize the animals

The plurality of antigens can comprise at least 11,000 different antigens, such as at least 12,000, 13,000, 14,000, 15,000, 16,000, 17,000, 18,000, 19,000, or 20,000 different antigens. In some embodiments, said plurality of antigens comprises at least 0.5% of the human proteome. The plurality of antigens can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. The plurality of antigens can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome. The plurality of antigens can comprise at least 0.5%, 1%, 2%, 3%. 4%, 5%, 6%. 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of proteins listed in **Table 5.** The library of antigens can also comprise a fusion protein library that represents an organism's proteome in unpurified form, such as a collection of overexpressing cells (E.coli, yeast, mammalian, insect, or other organism).

The antibody-generating cells from the animal can be lymphoid cells, such as B-cells. The antibody-generating cell can be used to generate a hybridoma, for example, fused to an immortal cell such as a myeloma cell, to create a hybidoma.

In one embodiment, a pre-screening step is performed, wherein a plurality of antibodies from the antibody-generating cells are screened prior to isolating a plurality of antibodies from the antibody-generating cells. Pre-screening can be performed by using the serum or supernatant of the antibody-producing cells to determine binding of antibodies from said antibody-generating cells with a mixture comprising one or more target antigens, such as native antigens or proteins. The pre-screening can be performed using immunohistochemistry, immunocytochemistry, ELISA, chromatography, or any other suitable methods known in the art to determine binding. Pre-screening can be used to select the antibody-generating cells (which can include antibody-secreting cells that have been fused to immortalized cells, such as hybridomas), for further screening, such as by a proteome array as described herein.

The mixture can comprise a crude lysate, cell, protein, peptide, or nucleic acid. The mixture can comprise a biological sample, such as a tissue, blood, sera, plasma, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, Cowper's fluid, pre-ejaculatory fluid, female ejaculate, sweat, tears, cyst fluid, pleural fluid, peritoneal fluid, pericardial fluid, lymph, chyme, chyle, bile, interstitial fluid, menses, pus, sebum, vaginal secretion, mucosal secretion, stool water, pancreatic juice, lavage fluid from sinus cavities, bronchopulmonary aspirate, blastocyl cavity fluid, or umbilical cord blood. In one embodiment, the biological sample can be substantially depleted of a common serum protein, such as, but not limited to, albumin or IgG. Depletion can comprise filtration, fractionation, or affinity purification. The biological sample can comprise a cell, such as a stem cell, undifferentiated cell, differentiated cell, or cell from a diseased subject or subject with a specific condition

A plurality of antibodies from the antibody-generating cells can be isolated, with or without a previous pre-screening step, before being subjected to a screening with an entire, or portion of, a proteome of an organism. For example, the isolated antibodies can be screened with at least 0.5%, 1%, 2%, 3%, 4%, 5%, 6%, 7%, 8%,9%,10%,11%,12%,13%,14%,15%,16%,17%,18%,19%,20%,25%, 30%, 35%, 40%, 45%, 50%, 60%, 70%1,80%, 90%, or 100% of an organism's proteome. For example, the isolated antibodies can be screened with at least 11,000, 12,000, 13,000, 14,000, 15,000, 16,000, 17,000, 18,000, 19,000, or 20,000 proteins of an organism. The proteome can be a bacterial, viral, fungal proteome. The proteome can be of an insect or mammal, such as a mouse, rat, rabbit, cat, dog, monkey, goat, or human. In some embodiments, the proteome is of a human.

For example, the isolated antibodies can be screened with at least 0.5% of the human proteome. In one embodiment, the isolated antibodies is screened with at least 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome. The isolated antibodies can be screened with at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5.** The proteome, or portion thereof, that is used to screen the antibodies can be present on an array.

After screening, an antibody can be selected based on its binding profile. For example, an antibody can be selected if it binds to a single target of the proteome, such as to a single target of a human proteome array, for a library described herein.

The method can be used to perform high-through production of antibodies. For example antigens to immunize mice can be produced in a high-throughput manner. This can be done by: 1) directed production of recombinant proteins from a gene expression library, where proteins of interest can be expressed as needed or through the use of whole heterologous cells, tissues, or crude or enriched cell extracts from the target species (an undirected approach), or whole mammalian cells engineered to express a heterologous protein (a directed approach). The extracts for injection can include subcellular components, such as cell and intracellular membranes, organelles, particles, or protein complexes and may be prepared using differential centrifugation or other well-known methods. These immunizations with complex mixtures of proteins may eventually produce antibodies against the most highly immunogenic proteins; in this event, the antigen sources can be immunodepleted using previously isolated antibodies to remove any especially antigenic proteins. In addition, both soluble and membrane proteins can be chromatographed in either their native or denatured state(s), by size-exclusion, ion-exchange, hydrophobic or affinity chromatography or by using individually collected small fractions to immunize with dozens to hundreds rather than thousands of proteins. Similarly, simple ammonium sulfate fractionation of extracts will provide a wide variety of antigen subsets. Specific techniques to target specific classes of proteins can be used. For example, proteins involved in SNO signaling may be purified by Immobilized Metal Affinity Chromatography (IMAC) and eluted metal-free proteins may then be used as antigens for immunization, or phosphoproteins may be bound to Ti_{O2} affinity matrices and thereby greatly enriched. Native proteins isolated or obtained directly from living or fixed cells can be preferred sources of immunogens.

The high-throughput method can also comprise a short timescale immunization to produce antibody-secreting lymphoid cells. For example, animals can be immunized with multiple antigens and adjuvant preparations by way of the rear footpads. Popliteal lymph nodes are collected from 7 to 21 days post-immunization. Lymph nodes in immunized animals are directly revealed by injection with Evans blue at the footpad.

The high-throughput method can also comprise generating the antibodies in GST-tolerant mice. Since many antigens are expressed as GST fusions, a line of GST-expressing mice (mice that actively express GST from *S. japonicum*) can be used to increase the yield of specific antibodies raised against the non-GST components of the fusion proteins and eliminate generation of anti-GST antibody.

A high-throughput method for producing antibodies can also comprise creation of cell fusions/hybridomas for antibody production. Primed lymphoid cells and myeloma cells are fused and fusion products are either plated onto semisolid medium (methylcellulose) containing fluorescently tagged antibodies that recognize the desired subclass of immunoglobulin molecules, or into semisolid media containing fluorescently tagged antigens that will mark colonies secreting the desired antibodies. A combination of the above methods may also be used. Fluorescently marked colonies are rescued from semisolid medium to liquid medium, using an inverted fluorescence microscope in combination with hand-picking using Drummond microcapillary and Drummond WireTrol devices, followed by outgrowth of the individual clones.

For the monoclonal antibodies generated in the high-throughput method, using either recombinant or native proteins, the responsible antigen can be identified by protein microarray deconvolution. Single-step deconvolution can be performed.

For example, when whole cells or cell lysates are used to generate hybridomas, a two-dimensional pooling strategy is employed to reveal identity of antigens that each monoclonal antibody would recognize. Supernatants of hybridomas are arrayed in two-dimensional grids and pooled both horizontally and vertically in 3-by-3 to 100-by-100 pool sizes. Three-dimensional pooling strategies can also be performed in which hybridomas are arrayed in plate sets to generate plate pools and horizontal and vertical pools in 3-by-3-by-3 to 100-by-100-by-100 format. The resulting pools are hybridized individually to the protein microarrays and scored using microarray analysis software. The shared positives (hits) of each horizontal and vertical pair or those at each 3-way intersection of plate, horizontal and vertical pools (called a trio) in two- and three-dimensional designs, respectively, are identified as the antigens recognized by the monoclonal antibody at the intersection of the same pairs or trios. When necessary, the identified antigens are validated by probing the microarrays with the corresponding antibodies. The pooling strategy can reduce the cost of characterizing or producing antibodies because the cost of the arrays is high, and use of a *e.g.* 10 by 10 pool reduces the number of arrays needed to analyze 100 clones from 100 to 20; for a 20 X 20 pool it reduces the number of arrays needed from 400 to 40. The 3-dimensional pools, for example, can screen a pool of 4096 hybridomas, while a 16-by-16-by-16 3-D strategy requires only 48 arrays. Using the 10-by-10 strategy, 820 arrays would can be used. A 3-D pooling strategy (12-by-8-by-12) based on 96-well plates is depicted in **FIG. 2****.**

Characterization of the monoclonal antibodies generated in the high-throughput method can be performed using whole proteome microarrays. The microarrays can be used to determine the specific antigens to which a given monoclonal antibody binds. Critical quality information about monoclonal antibody affinity, potential cross reactivity or lack of crossreactivity with other antigens are all provided by an array analysis.

Production of monoclonal antibody in the high-throughput method can be from fusion clones. Monoclonal antibodies are produced in vitro or in vivo in the desired quantities. These can be purified using various well-established methods.

On the basis of the characterization of monoclonal antibodies using protein microarrays, monoclonal antibodies of high quality (*e.g.,* high affinity and low cross reactivity) can be selected and used to produce antibody arrays. The antibodies are selected and their concentrations normalized to a similar titer. They can be arrayed in a multiwell format *(e.g.* 96- 384- or 1562-well) with proper positive *(e.g.,* diluted human IgG) and negative (e.g., human IgM and BSA) controls to fabricate antibody microarrays using a microarrays robot (e.g., Nanoprint, Array It, Inc.). The arrangement of the monoclonal antibodies in different microarray configurations may be customized to facilitate a range of proteome-wide studies.

### Method of Using a Library of Antibodies

A method of identifying an antibody for a target comprises contacting a target with a library of antibodies, determining binding between the target and the plurality of antibodies; and identifying an antibody for the target when the target binds to an antibody of the library. A method of identifying a target comprises contacting a target with a library of antibodies, determining binding between the target and the plurality of antibodies; and identifying the target antibody when the target binds to an antibody of the library. The library of antibodies can be attached to a substrate such that the target is contacted with an array comprising the library of antibodies.

The library can comprise of a plurality of different antibodies, such as described above. For example, the library of antibodies can comprise antibodies that are produced by the same platform. The library can comprise a plurality of different antibodies, wherein within the plurality or a subset of the plurality (such as at least 10%, 20%, 30%, 40%, 50%, 60%, 70%, 80%, 90%, 95%, 99% or 100% of the plurality), the antibodies are produced by the same platform, are monospecific, bind a native form of its target protein; are monoclonal; are immunoprecipitating antibodies; are IgG antibodies (e.g. antibodies of IgG isotype); have a binding affinity for its target that is similar to that of another antibody of the plurality of antibodies (for example, within at least 1, 2, 3, 4, 5, 6, 7, 8, 9, 10, 11, 12, 13, 14, 15, 16, 17, 18, 19, or 20%); each antibody is a have a binding affinity of at least 10⁻⁷M (K_{D}) (for example, such as at least 10⁻⁸M, 10⁻⁹M, 10⁻¹⁰M, 10⁻¹¹M, 10⁻¹²M, 10⁻¹³M, 10⁻¹⁴M, 10⁻¹⁵M, or 10⁻¹⁶M) for its target; or any combination thereof. The library may comprise at least 50, 75, 100, 125, 150, 175, 200, 225, 250, 275, 300, 325, 350, 375, 400, 425, 450, 475, 500, 525, 550, 575, 600, 625, 650, 675, 700, 725, 750, 775, 800, 825, 850, 875, 900, or 1000 different monoclonal antibodies, bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome (e.g. a human proteome), bind at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5,** or any combination thereof.

Also provided herein is a method of identifying an antibody monospecific for a protein, such as a human protein, comprising: contacting a plurality of antibodies with a proteome array, such as a human proteome array; determining binding between the plurality of antibodies and the targets present on the proteome array; and identifying an antibody as monospecific. The array can comprise a plurality of antigens or proteins that comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. For example, the proteome array can comprise at least 11,000, 12,000, 13,000, 14,000, 15,000, 16,000, 17,000, 18,000, 19,000, or 20,000 proteins of an organism. The organism can be a bacterium, virus, or fungus. The organism can be of an insect or mammal, such as a mouse, rat, rabbit, cat, dog, monkey, goat, or human. For example, the proteome can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteome. The proteome array can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in Table 5.

Binding can be detected by techniques known in the art (e.g., radioimmunoassay, ELISA (enzyme-linked immunosorbant assay), "sandwich" immunoassays, immunoradiometric assays, gel diffusion precipitation reactions, immunodiffusion assays, in situ immunoassays (e.g., using colloidal gold, enzyme or radioisotope labels, for example), Western blots, precipitation reactions, agglutination assays (e.g., gel agglutination assays, hemagglutination assays, etc.), complement fixation assays, immunofluorescence assays, protein A assays, and immunoelectrophoresis assays.

Antibody binding can be detected by detecting a label on the primary antibody. Alternatively, the primary antibody is detected by detecting binding of a secondary antibody or reagent to the primary antibody. For example, the secondary antibody can be labeled. In some embodiments, an automated detection assay or high-throughput system is utilized. For example, in a capture micro-enzyme-linked immunosorbent assay (ELISA), an antibody/antigen reaction is made measurable by immobilization of the antibody and subsequent direct or indirect colorimetric, fluorescent, luminescent or radioactive detection of bound, labeled antigens. For example, the antigen can be labeled by biotin or other labels, which will allow downstream detection.

The immobilized antibodies will generally bind to a single antigenic determinant present. The antigenic determinant can be labeled, such as through labeling of the biomarker comprising the antigenic determinant. The specificity of this reaction will permit quantification in the ELISA measurements. The ELISA reaction can be used in a high throughput format to screen all hybridoma supernatants via the following steps. Screening assays built on other principles than an ELISA can be deployed (e.g., antibody microarrays, high-throughput screening based on MALDI/MS and/or multi-channel capillary electrophoresis). ELISA or microarray data are evaluated, e.g., by published methods. The goal of the data analysis process is the selection of hybridoma supernatants that show the best collection with an important clinical parameter and are specific to one of the analyte groups.

### Microarray Substrate

An array (or microarray) comprises a library of antibodies and a substrate. In some embodiments, each antibody is immobilized on a substrate. The antibody may be reversibly or irreversibly immobilized on the substrate. The substrate can be planar or a particle and can comprise a solid or porous material. The substrate may be either organic or inorganic, biological or non-biological, or any combination of these materials.

In one embodiment, the substrate is transparent or translucent. The portion of the surface of the substrate on which the patches reside is preferably flat and firm or semi-firm. Numerous materials are suitable for use as a substrate. The substrate can comprise silicon, silica, glass, or a polymer. For instance, the substrate can comprise a material selected from a group consisting of silicon, silica, quartz, glass, controlled pore glass, carbon, alumina, titanium dioxide, germanium, silicon nitride, zeolites, and gallium arsenide. Many metals such as gold, platinum, aluminum, copper, titanium, and their alloys are also options for substrates of the array. In addition, many ceramics and polymers may also be used as substrates. Polymers which may be used as substrates include, but are not limited to, the following: polystyrene; poly(tetra)fluorethylene; (poly)vinylidenedifluoride; polycarbonate; polymethylmethacrylate; polyvinylethylene; polyethyleneimine; poly(etherether)ketone; polyoxymethylene (POM); polyvinylphenol; polylactides; polymethacrylimide (PMI); polyalkenesulfone (PAS); polyhydroxyethylmethacrylate; polydimethylsiloxane; polyacrylamide; polyimide; co-block-polymers; and Eupergit®. Photoresists, polymerized Langmuir-Blodgett films, and LIGA structures may also serve as substrates.

In one embodiment, a microarray of distinct antibodies is bound on a glass slide coated with a polycationic polymer. A substrate may be formed and intended for use in detecting binding of target molecule to one or more distinct antibodies. In one embodiment, the substrate includes a glass substrate having formed on its surface, a coating of a polycationic polymer, preferably a cationic polypeptide, such as poly-lysine or poly-arginine. Formed on the polycationic coating is a microarray of distinct biopolymers, each localized at known selected array regions, such as regions.

The slide may be coated by placing a uniform-thickness film of a polycationic polymer, e.g., poly-1-lysine, on the surface of a slide and drying the film to form a dried coating. The amount of polycationic polymer added is sufficient to form at least a monolayer of polymers on the glass surface. The polymer film is bound to surface via electrostatic binding between negative silyl-OH groups on the surface and charged amine groups in the polymers. Poly-1-lysine coated glass slides may be obtained commercially, e.g., from Sigma Chemical Co. (St. Louis, Mo.).

A suitable microarray substrate is also made through chemical derivatization of glass. Silane compounds with appropriate leaving groups on a terminal Si will covalently bond to glass surfaces. A derivatization molecule can be designed to confer the desired chemistry to the surface of the glass substrate. An example of such a bifunctional reagent is amino-propyl-tri(ethoxy)silane, which reacts with glass surfaces at the tri(ethoxy)silane portion of the molecule while leaving the amino portion of the molecule free. Surfaces having terminal amino groups are suitable for adsorption of biopolymers in the same manner as poly-lysine coated slides. The identity of the terminal surface group can be modified by further chemical reaction. For example, reaction of the terminal amine in the above example with glutaraldehyde results in a terminal aldehyde group. Further layers of modification may be applied to achieve the desired reactivity before spotting the microarray, such as by application of a Protein A or Protein G solution to the silynated glass. Additional surfaces that bind polypeptides are nitrocellulose-coated glass slides, available commercially from Schleicher and Schuell, and protein-binding plastics such as polystyrene.

The spotted antibodies may be attached by either adsorption or covalent bonding. Adsorption occurs through electrostatic, hydrophobic, Van der Waals, or hydrogen-bonding interactions between the spotted polypeptide and the array substrate. Simple application of the polypeptide solution to the surface in an aqueous environment is sufficient to adsorb the polypeptide. Covalent attachment is achieved by reaction of functional groups on the polypeptide with a chemically activated surface. For example, if the surface has been activated with a highly reactive electrophilic group such as an aldehyde or succinimide group, unmodified polypeptides react at amine groups, as at lysine residues or the terminal amine, to form a covalent bond.

To form the microarray, defined volumes of distinct biopolymers are deposited on the polymer-coated slide using any suitable method known in the art. According to an important feature of the substrate, the deposited antibodies remain bound to the coated slide surface non-covalently when an aqueous sample is applied to the substrate under conditions that allow binding of labeled ligands in the sample to cognate binding partners in the substrate array.

In some embodiments, each microarray contains at least 50, 60, 70, 80, 90, 100, 150, 200, 250, 300, 350, 400, 450, 500, 600, 700, 800, 900, or 1000 distinct antibodies per surface area of less than about 1 cm². In one embodiment, the microarray contains 50, 60, 70, 80, 90, 100, 150, 200, 250, 300, 350 or 400 regions in an area of about 16 mm², or 2.5 x 10³ regions/cm². Also in a preferred embodiment, the antibodies in each microarray region are present in a defined amount between about 0.1 femtomoles and 100 nanomoles.

Also in a preferred embodiment, the biopolymers have lengths of at least about 50 units, e.g. amino acids, nucleotides, etc., i.e., substantially longer than polymers which can be formed in high-density arrays by various in situ synthesis schemes.

### Uses of the Microarrays

Microarrays can be used in medical diagnostics, drug discovery, molecular biology, immunology and toxicology.

Microarrays of immobilized antibodies prepared in accordance with the invention can be used for large scale binding assays in numerous diagnostic and screening applications. The multiplexed measurement of quantitative variation in levels of large numbers of targets (e.g. proteins) allows the recognition of patterns defined by several to many different targets (e.g., proteins). One can simultaneously assess many physiological parameters and disease-specific patterns.

One embodiment involves the separation, identification and characterization of proteins present in a biological sample. For example, by comparison of disease and control samples, it is possible to identify "disease specific proteins". These proteins may be used as targets for drug development or as molecular markers of disease.

Antibody arrays can be used to monitor the expression levels of proteins in a sample where such samples may include biopsy of a tissue of interest, cultured cells, microbial cell populations, biological fluids, including blood, plasma, lymph, synovial fluid, cerebrospinal fluid, cell lysates, culture supernatants, amniotic fluid, etc., and derivatives thereof. Of particular interest are clinical samples of biological fluids, including blood and derivatives thereof, cerebrospinal fluid, urine, saliva, lymph, synovial fluids, etc. Such measurements may be quantitative, semi-quantitative, or qualitative. Where the assay is to be quantitative or semi-quantitative, it will preferably comprise a competition-type format, for example between labeled and unlabeled samples, or between samples that are differentially labeled.

Assays to detect the presence of target molecules to the immobilized polypeptides may be performed as follows, although the methods need not be limited to those set forth herein and include any suitable method known in the art.

Samples, fractions or aliquots thereof are added to a microarray comprising the antibodies. Samples may comprise a wide variety of biological fluids or extracts as described above. Preferably, a series of standards, containing known concentrations of control ligand(s) is assayed in parallel with the samples or aliquots thereof to serve as controls. The incubation time should be sufficient for target molecules to bind the polypeptides. Generally, from about 0.1 to 3 hr, usually 1 hr, but could be as long as one day or longer.

After incubation, the insoluble support is generally washed of non-bound components. Generally, a dilute non-ionic detergent medium at an appropriate pH, generally 7-8, is used as a wash medium. From one to six washes may be employed, with sufficient volume to thoroughly wash non-specifically bound proteins present in the sample.

In order to detect the presence of bound target, a variety of methods may be used. These fall into three general groups. The target itself may be labeled with a detectable label, and the amount of bound label directly measured. Alternatively, the labeled sample may be mixed with a differentially labeled, or unlabeled sample in a competition assay. In yet another embodiment, the sample itself is not labeled, but a second stage labeled reagent is added in order to quantitate the amount of ligand present.

Examples of labels that permit direct measurement of ligand binding include radiolabels, such as ³H or ¹²⁵I, fluorescers, dyes, beads, chemilumninescers, colloidal particles, and the like. Suitable fluorescent dyes are known in the art, including fluorescein isothiocyanate (FITC); rhodamine and rhodamine derivatives; Texas Red; phycoerythrin; allophycocyanin; 6-carboxyfluorescein (6-FAM); 2',7'-dimethoxy-4',5'-dichloro-6-carboxyfluorescein (JOE); 6-carboxy-X-rhodamine (ROX); 6-carboxy-2',4',7',4,7-hexachlorofluorescein (HEX); 5-carboxyfluorescein (5-FAM); N,N,N',N'-tetramethyl-6-carboxyrhodamine (TAMRA); sulfonated rhodamine; Cy3; Cy5; etc. Preferably the compound to be labeled is combined with an activated dye that reacts with a group present on the ligand, e.g. amine groups, thiol groups, aldehyde groups, etc.

Particularly where a second stage detection is performed, for example by the addition of labeled antibodies that recognize the target, the label can be a covalently bound enzyme capable of providing a detectable product signal after addition of suitable substrate. Examples of suitable enzymes for use in conjugates include horseradish peroxidase, alkaline phosphatase, malate dehydrogenase and the like. Where not commercially available, such antibody-enzyme conjugates are readily produced by techniques known to those skilled in the art. The second stage binding reagent may be any compound that binds the target molecules with sufficient specificity such that it can be distinguished from other components present. In a preferred embodiment, second stage binding reagents are antibodies specific for the ligand, either monoclonal or polyclonal sera, e.g. mouse anti-human antibodies, etc.

For an amplification of signal, the ligand may be labeled with an agent such as biotin, digoxigenin, etc., where the second stage reagent will comprise avidin, streptavidin, anti-digoxigenin antibodies, etc. as appropriate for the label.

Microarrays can be scanned to detect binding of the ligands, e.g. by using a scanning laser microscope, by fluorimetry, a modified ELISA plate reader, etc. For example, a scanning laser microscope may perform a separate scan, using the appropriate excitation line, for each of the fluorophores used. The digital images generated from the scan are then combined for subsequent analysis. For any particular array element, the ratio of the fluorescent signal with one label is compared to the fluorescent signal from the other label DNA, and the relative abundance determined.

The microarray and methods of detecting target molecules may be used for a number of screening, investigative and diagnostic assays. In one application, an array of antibodies is bound to total protein from an organism to monitor protein expression for research or diagnostic purposes. Labeling total protein from a normal cell with one color fluorophore and total protein from a diseased cell with another color fluorophore and simultaneously binding the two samples to the same array allows for differential protein expression to be measured as the ratio of the two fluorophore intensities. This two-color experiment can be used to monitor expression in different tissue types, disease states, response to drugs, or response to environmental factors.

In screening assays, for example to determine whether a protein or proteins are implicated in a disease pathway or are correlated with a disease-specific phenotype, measurements may be made from cultured cells. Such cells may be experimentally manipulated by the addition of pharmacologically active agents that act on a target or pathway of interest. This application is important for elucidation of biological function or discovery of therapeutic targets.

For many diagnostic and investigative purposes it is useful to measurement levels of target molecules, e.g. proteins, in blood or serum. This application is important for the discovery and diagnosis of clinically useful markers that correlate with a particular diagnosis or prognosis. For example, by monitoring a range of antibody or T cell receptor specificities in parallel, one may determine the levels and kinetics of antibodies during the course of autoimmune disease, during infection, through graft rejection, etc. Alternatively, novel protein markers associated with a disease of interest may be developed through comparisons of normal and diseased blood sample, or by comparing clinical samples at different stages of disease.

Information on the protein expression in a genome of an organism can have a wide variety of applications, including but not limited to diagnosis and treatment of diseases in a personalized manner (also known as "personalized medicine") by association with phenotype such as onset, development of disease, disease resistance, disease susceptibility or drug response. Identification and characterization of the proteins relevant to biological pathways in a genome of an organism in terms of cell- or tissue-specificity can also aid in the design of transgenic expression constructs for therapy with enhanced therapeutic efficacy and reduced side effects. Identification and characterization of protein expression in terms of cell- or tissue-specificity can also aid in the development of function markers for diagnosis, prevention and treatment of diseases. "Disease" includes but is not limited to any condition, trait or characteristic of an organism that it is desirable to change. For example, the condition may be physical, physiological or psychological and may be symptomatic or asymptomatic.

In another embodiment, the antibody arrays are used to detect post-translational modifications in proteins, which is important in studying signaling pathways and cellular regulation. Post-translational modifications can be detected using antibodies specific for a particular state of a protein, such as phosphorylated, glycosylated, farnesylated, etc.

The detection of these interactions between ligands and polypeptides can lead to a medical diagnosis. For example, the identity of a pathogenic microorganism can be established unambiguously by binding a sample of the unknown pathogen to an array containing many types of antibodies specific for known pathogenic antigens.

### Kits

A kit can comprise a library of antibodies. In some embodiments the library of antibodies is arrayed in a support, e.g., 96 or 384 wells. In one embodiment, a kit comprises a microarray of antibodies. A kit may further include: reporter assay substrates; reagents for induction or repression of a particular biological pathway (cytokines or other purified proteins, small molecules, cDNAs, siRNAs, etc.), and/or data analysis software.

In addition, kits can comprise reagents and instructions for performing methods of the present invention, or for performing tests or assays utilizing any of the compositions, libraries, arrays, or assemblies of articles of the present invention. A kits may further comprise buffers, enzymes, adaptors, labels, secondary antibodies and instructions necessary for use of the kits, optionally including troubleshooting information.

In yet another embodiment, a kit may comprise a library of antibodies, such as described herein, and a library of antigens, such as a proteome of an organism. The kit may comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of an organism's proteome. The library of antigens can represent a substantial portion or an entire organism's proteome, such as a bacterial, viral, fungal proteome. The library of antigens can represent a substantial portion or an entire proteome of an insect or manmmal, such as a mouse, rat, rabbit, cat, dog, monkey, goat, or human. For example, an organism's proteome library can comprise at least 11,000, 12,000, 13,000, 14,000, 15,000, 16,000, 17,000, 18,000, 19,000, or 20,000 different antigens. The organism's proteome library can comprise at least 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100% of the human proteins listed in **Table 5.**

### EXAMPLES

### Example 1: Subcloning ∼17,000 Full-Length Human ORFs into an Expression Vectors.

A Gateway® ORF collection of ∼16,000 unique ORFs (Invitrogen, CA) was obtained along with an additional 1,000 full-length human ORFs were subcloned. A Gateway® -compatible expression vector for yeast (pEGH-A) was constructed that upon galactose induction produces N-terminal 6xHis6::glutathione-S-transferase (GST) fusion proteins in yeast **(****FIG. 3****).** Subsequently, all ∼17,000 human ORFs was subcloned at a success rate of 99% as determined by restriction digestion. Specifically, three replicates of the collection were made, from one of which the Entry DNAs were extracted, and the quality of the plasmid DNA was checked on agarose gels. The resulting recombinants were then transformed into bacteria and single colonies were selected on Amp-containing plates. For each LR recombination, four single colonies were picked to generate glycerol stocks, two of which were further processed to extract plasmid DNAs in a 96-well format. The extracted plasmid DNAs were digested with a restriction enzyme to release the inserts, and run on agarose gels to examine the vector and insert sizes as an indicator of successful subcloning (**FIG**. **3****).** Each restriction event was scored based on expected insert sizes and the success rate was determined as 99.3%.

Sequencing of >200 random expression clones showed 100% of the sequences were correct. The confirmed expression constructs were rearrayed to generate a master set of expression clones for yeast. Similar large scale cloning was completed in a bacterial expression vector.

### Example 2: Design and fabrication of a 5000 human antigen microarray.

A pilot experiment was performed to test the ability to rapidly purify correctly folded recombinant proteins for microarray production. These proteins fall into five different functional categories: transcription factors and transcription co-regulators, RNA binding proteins, protein kinases, chromatin-associated and chromatin-modifying proteins, and mitochondrial proteins. Proteins were placed in these categories on the basis of primary sequence, literature, and Gene Ontology annotation. The ORFs expressed represented as many as 85% (in the case of transcription factors) of all human proteins in the relevant functional category. Over 90% of expressed proteins were purified at sufficient levels for array construction (Ho, S.W., et al., Linking DNA-binding proteins to their recognition sequences by using protein microarrays. Proc Natl Acad Sci USA, 2006. 103(26): p. 9940-5).

Several functional tests were performed to confirm that the expressed proteins were functional after being immobilized on solid surfaces. Autophosphorylation assays were performed on a kinase chip containing 119 individually purified protein kinases from yeast and observed that approximately 85% of the kinases showed detectable kinase activity (Zhu, H., et al., Analysis of yeast protein kinases using protein chips. Nat Genet, 2000. 26(3) p. 283-9.). In spite of being covalently linked to a solid surface, most of these protein kinases clearly maintained their enzymatic activity. Specific interactions between various DNA motifs and transcription factors was identified and profiled using a protein chip composed of ∼300 yeast transcription factors (Ho, S.W., et al., Linking DNA-binding proteins to their recognition sequences by using protein microarrays. Proc Natl Acad Sci USA, 2006. 103(26): p. 9940-5.). The interactions between DNA motifs and human transcription factors was also studied, in which a pilot protein chip composed of∼1,000 human transcription factors was used to show that the known DNA motifs specifically bound to their documented transcription factors; conversely, and point mutations in these motifs dramatically reduced their binding affinity (Hu, S., et al., Profiling the human protein-DNA interactome reveals ERK2 as a transcriptional repressor of interferon signaling. Cell, 2009. 139(3): p. 610-22).

### Example 3: Fabrication of a Human Antigen Protein Chip.

To purify the complete set of 17,000 human protein antigens from yeast cells, the entire master set of human ORFs was cloned in pEGH-A into yeast, single colonies were picked and glycerol stocks prepared. The yeast cells were induced for recombinant protein production and stored at -80°C. To monitor the quality of induced culture, 24 random strains were inoculated in duplicate for each batch of culture preparation and let them go through the protein purification step first. Using immunoblot and silver staining techniques, the success rate of culture induction was estimated **(****FIG. 4A****).** Success was indicated only when at least 85% of the purified proteins showed a major band at the expected MW range in both immunoblot and silver staining analyses. Otherwise, culture preparation for the failed batch was repeated. Using this standard, all 17,000 antigen proteins were purified at a success rate of 85%. The purified human antigen proteins were spotted using a microarrayer onto various glass surfaces (e.g., FAST, Ni-NTA and FullMoon) to produce the human antigen chips. The quality of the chips was monitored by probing the slides with anti-GST antibodies and Cy3-labeled secondary antibodies. The chips were scanned and signals acquired and analyzed using GenePix software. The results indicated that the chips were of high quality and >90% of the printed proteins produced signals significantly higher than background (**FIG**. **4B**, C).

### Example 4: Antibody quality profiling on an antigen microarray

Monoclonal antibodies against human proteins expressed in liver was performed using traditional methods (Hu, S., et al., A protein chip approach for high-throughput antigen identification and characterization. Proteomics, 2007. 7(13): p. 2151-61). Large amounts of monoclonal were purified from ascites and diluted 10,000-fold in PBS containing 1% BSA. To evaluate the quality (e.g., affinity and specificity) of the mAbs, a human liver protein chip was used. On the basis of a literature/database search, 1,058 full-length cDNAs that are known to be expressed in human liver were cloned into an expression vector that expresses these proteins as N-terminal GST fusion proteins. To fabricate the human liver protein chips, these genes were expressed and purified from yeast cells using glutathione affinity chromatography and spotted onto glass slides. Mouse IgG was also spotted on the chips to serve as a landmark. The amount of immobilized human liver proteins on the antigen microarray was visualized and quantified using anti-GST antibodies **(****FIG. 5A****).** The conclusion was 98.5% 1,042/1,058) of the proteins showed significant signals above the background and that the protein chips are of high quality.

To evaluate the specificity of several monoclonal antibodies generated against different human liver proteins, the chips were blocked with 1% BSA in PBS buffer at room temperature (RT) for a minimum of 1 hr with gentle shaking in a homemade, humidified chamber. Meanwhile, the monoclonal antibodies purified from ascites were diluted 20,000-fold in phosphate-buffered saline (PBS) buffer. After blocking, the diluted mAbs were added to different chips and incubated at RT for 30 min with gentle shaking. The chips were then washed three times in PBST (1% Tween 20) buffer for 10 min at 42° C with shaking. Anti-mouse IgG antibodies labeled with Cy3 (Jackson Laboratories, USA, 11,000 dilution in PBS) were added to the chips and incubated in the dark at RT for 1 hr. The chips were then washed 3 times with pre-warmed PBS+0.1% Triton for 10 min each at 42° C with gentle shaking. After rinsing twice in filter-sterilized, double-deionized water, chips were spun to dryness. To visualize the binding profiles, we scanned the chips with a microarray scanner and further analyzed the binding signals with the GenePix software. As shown in **FIG. 5B****,** the specificity of the mAb generated against pirin was tested against 1,058 proteins spotted on a slide, and this mAb only recognized pirin. Because the amount of each spotted protein does not vary dramatically, this result indicated that the antibody is highly specific. Similar results were also obtained with mAbs generated against four other human proteins **(****FIG. 5C****).**

### Example 5: Generation and characterization of monospecific antibodies.

Over 2,000 strong IgG-secreting monoclonal antibodies in response to immunization with both recombinant human proteins and live human cancer cell lines have been generated. Protein microarrays that contains 17,000 human proteins has been used to analyze the binding specificity of 88 of these monoclonal antibodies.

To reduce the number of microarrays used for this analysis, equal mixtures of supernatants from up to seven different hybridomas were used an intersectional analysis described schematically in **FIG. 6** to interrogate the specificity of these pooled antibodies. Eleven of the 88 monoclonal antibodies are truly monospecific, in that only a single protein on the microarray is recognized by these monoclonal antibodies. Seven other monoclonal antibodies bound to only three different proteins on the array, showing highly restricted specificity. For four of the monospecific monoclonal antibodies, no commercially antibodies of any sort (polyclonal or monoclonal) are available, while it is not know whether any of the commercially available antibodies to the other seven are monospecific. A list of these monospecific monoclonal antibodies, and the commercially available antibodies which recognize them, is shown on **Table 1.** Antigens recognized by the 11 monospecific monoclonal antibodies are listed by Gene Symbol ID. The number of all commercially-available antibodies (both monoclonal and polyclonal) to that protein is also listed, along with the monoclonal antibodies to that antigen in parentheses.

**Table 1: Antigens recognized by monospecific mAbs generated.**

| Antigen recognized by monospecific mAb | Number of all commercially-available Abs to antigen (mAbs to antigen) |
|---|---|
| ZG16B | 0 |
| CAPZA3 | 16 (7) |
| C1orf123 | 0 |
| LOC284361 | 0 |
| COASY | 37 (17) |
| CA1 | 64 (19) |
| TES | 11 (3) |
| LSM6 | 7 (3) |
| PRKCQ | 7 (5) |
| C19orf47 | 0 |
| SGCG | 3 (2) |

### Example 6: Monoclonal antibodies against human brain-expressed transcription factors.

### Antigen Production.

Human proteins are expressed from a proprietary expression vector in *E. coli.* As described previously, the human proteins are expressed and purified as N-terminus-tagged GST: His6 fusion proteins. As some human proteins are much easier to express and purify in large quantities than others from the bacterial host, a large-scale screening to determine which human chromosomal proteins fall in this category is performed. An extremely high-throughput protein purification protocol that allows purification of >4,000 individual proteins from *E. coli* within ten hours can be performed as depicted in **FIG**. **7** (Chen, C.S., et al., A proteome chip approach reveals new DNA damage recognition activities in Escherichia coli. Nat Methods, 2008. 5(1): p. 69-74.). Using this protocol, the proteins of the ∼200 candidates that are easier to purify will be identified. Approximately 30% of the strains should yield 2 mg protein per 250 mL of culture, 60% should yield 0.5 mg protein/250 mL culture, and 40% should yield <0.1 mg protein/250 mL culture.

The "easier" higher yield proteins from larger bacterial culture is purified. In brief, the strains are activated in smaller overnight culture and inoculated into 250 mL media to a final OD₆₀₀ of ∼0.1 the next morning. After growing the cells to an OD₆₀₀ of 0.7-0.9 at 37°C with shaking at 250 rpm, protein expression is induced with isopropyl β-D-thiogalactoside (IPTG) at a final concentration of 1 mM for∼3.5 h. The cells are collected by centrifugation and resuspended at 4°C in lysis buffer containing 50 mM NaH₂PO₄, pH 8, with 300 mM NaCl, 20 mM imidazole, CelLytic B (Sigma), lysozyme at 1 mg/ml, benzonase at 50 units/ml, proteinase inhibitor cocktail (Sigma), and 1 mM PMSF. After incubation on ice for 30 min, the cell debris is removed by centrifugation and pre-washed Ni-NTA Superflow (QIAGEN) is added to the cell lysates. After 40 min incubation on ice, the nickel resin is collected and washed three times with wash buffer I (50 mM NaH₂PO₄ with 300 mM NaCl, 10% glycerol, 20 mM imidazole, and 0.01% Triton X-100, pH 8) and three times with wash buffer II (50 mM NaH₂PO₄ with 150 mM NaCl, 25% glycerol, 20 mM imidazole, and 0.01% Triton X-100, pH 8) in a cold room. The purified proteins are eluted by 3C protease cleavage to remove the GST and His6 tags. The eluted proteins are combined and aliquoted; a small fraction of these is used to determine the quality and quantity of the protein on a PAGE gel using Coomassie staining.

For the proteins falling in the mediocre yield category, the culture volume is increased to 1 L to reach the 2 mg yield. For those that do not express well in bacteria, the proteins are purified from a yeast culture using the GST affinity tag. As described previously, the expression of the human proteins can be induced in yeast by adding galactose. To purify these tough ones, the proteins are synthesized using a commercially available in vitro transcription/translation system (e.g., the Roche rabbit reticulocyte system). No additional subcloning is required as a T7 promoter is already been built into the vector system being used. Taken together, at least 85% of the human proteins are expected to yield ∼2 mg. This amount of antigen is more than sufficient for immunization, monoclonal antibody production and screening purposes.

### Antibody Production

Rapid immunizations at multiple sites (RIMMS), which uses a single round of up to four dorsal subcutaneous injections of antigen, followed 14 days later by an intraperitoneal (IP) boost and 3-4 days later by spleen cell harvest (Bynum, J., et al., Development of class-switched, affinity-matured monoclonal antibodies following a 7-day immunization schedule. Hybridoma, 1999. 18(5): p. 407-11) or a single round of injection of antigen/adjuvant into the rear footpads, followed by harvesting of draining (popliteal and inguinal) lymph nodes 7-14 days later, without the need for a booster injection (Mirza, I.H., et al., A comparison of spleen and lymph node cells as fusion partners for the raising of monoclonal antibodies after different routes of immunisation. J Immunol Methods, 1987. 105(2): p. 235-43) is used to immunize the animals.

Hybridomas are generated by fusing two cell types, immune B cells and a culture-stable myeloma cell line. This is carried out in the presence of a fusogenic compound such as PEG. The desired hybrid cell products can be selected from among the unfused cells by taking advantage of the presence of two metabolic routes of pyrimidine/purine synthesis, the de novo and scavenging pathways. The myeloma cell lines commonly used are deficient in the salvage pathway, as they have been selected for resistance to 8-azaguanine or 6-thioguanine and are thus hypoxanthine-guanine phosphoribosyl transferase (HGPRT) deficient. Fusion reactions are thus plated in HAT medium to eliminate unfused immune cells and myeloma cells but is permissive for hybridoma outgrowth. The myeloma cell lines that is used is X63-Ag8.653, NSO/1 or Sp2/0-Ag-14.

Screening to identify the desired hybridomas is performed by analyzing aliquots of each microtiter well culture supernatant from the dispersed cell fusion reaction. An ELISA type of assay, in which antigen is immobilized onto a 96 well polyvinyl plate and then incubated with supernatants from all of the colony-containing wells is devised for each individual antigen. The cells in the culture wells producing antigen-binding antibody are harvested and replated in diluted form for isolation of clonal colonies. This limiting dilution cloning step takes up to approximately 2 weeks of incubation for individual cells to grow into assayable colonies. Commonly built into the ELISA assay is a second screen, which reveals the class of antibody being secreted by the hybridoma.

The two final steps in the isolation of monoclonal antibodies involve the establishment of the clonality of the hybridoma lines, and then the expansion of those lines to produce useful amounts of antibody. When the standard methods have been used to produce a hybridoma, the cell line is cloned to eliminate the presence of contaminating hybridomas that may be producing unrelated antibodies. This is typically carried out by limiting dilution plating of the cells into microtiter wells, followed by a rescreening of the supernatant with an antigen-specific ELISA. Limiting dilution cloning can be bypassed if the original fusion reaction is plated in semi-solid medium (methylcellulose) in Petri dishes (Davis, J.M., et al., A simple, single-step technique for selecting and cloning hybridomas for the production of monoclonal antibodies. J Immunol Methods, 1982. 50(2): p. 161-71), since the cells grow out as defined clones from the beginning. Clones that are expressing antibodies to the antigen can be identified by including antigen in the semisolid medium, in effect carrying out an Ouchterlony reaction that will precipitate antigen:antibody complexes in the vicinity of the clone. Clones are then picked with capillaries into 12 well plates as the first step of expansion. Small scale cultures from either type of cloning process are then expanded using culture vessels of increasing volume. Useful amounts of antibody are harvested by maintaining the hybridomas in large volume culture systems, (e.g., large stationary flasks, spinner flasks, hollow fiber reactors), which typically yield 20-100 mg/1, or by injection of the cells into mice for ascites production, which can yield 5-10 mg/ml, but substantially smaller total volumes.

### Fabrication of human antigen microarrays.

To provide sufficient human antigen chips, the ∼17,000 human antigens is first purified as N-terminal GST-His6 fusion proteins from yeast cells, using an established high-throughput protocol (discussed above). First, the yeast strains on SC-Ura agar plates and culture in 800 µl of SC-Ura medium overnight in a 96-well. Each saturated culture will then be inoculated to 12 ml SC-Ura and induced by 2% galactose for 4 hours after the culture reaches O.D. 0.7 The induced yeast cells are collected and stored at -80°C. To monitor the quality of the induced culture, 24 random strains for each batch of culture preparation is double-inoculated and run through the protein purification step first to confirm adequate induction levels. Using immunoblot and silver staining techniques, the success rate of culture induction is estimated and only those batches that show >85% success rate will be subjected to large-scale purification. To produce the human antigen chips, the purified human antigen proteins and control proteins, including dilution series of BSA and GST-His6 as negative controls and dilution series of histones, human IgG and IgM, and EBNA1 as positive controls, is spotted in duplicate on FullMoon slides (FullMoon Biosystems, USA) using two microarrayers, ChipWriter Pro (Bio-Rad) and NanoPrint (TeleChem, USA), at a throughput of 150 chips/day. The quality of each batch of the chips and the amount of immobilized proteins on the surface will be monitored by probing the chips with anti-GST antibody, followed by Cy3-labeled secondary antibodies. Success rate of printing is expected to reach >90%.

### Validation of antibodies against chromosomal proteins using human antigen microarrays.

*Determining mAb Specificity:* To evaluate the specificity of mAbs generated against human chromosomal proteins, mAbs purified from ascites is diluted 1000-fold in PBS as a working stock. The chips (microarrays) are blocked with 1% BSA in PBS buffer at room temperature (RT) for a minimum of 1 hr with gentle shaking in a homemade, humidified chamber. Properly diluted mAbs in PBS buffer is incubated on chips at RT for 30 min with gentle shaking. The chips are then subjected to 3-10 min washes in PBST (1% Tween 20) buffer at 42 °C with shaking. Anti-mouse IgG antibodies labeled with Cy-5 (Jackson Laboratories, USA, 1:1,000 dilution in PBS) are added to the chips and incubated in the dark at RT for 1 hr. After the same washing step, the chips are briefly rinsed in filter-sterilized, double-deionized water, and spun to dryness. To visualize the binding profiles, the chips are scanned with a microarray scanner and the binding signals with the GenePix software is are analyzed. As a negative control experiment, Cy5-labeled secondary antibodies is probed to the chips to identify non-specific binding activities. About 150 or so proteins (e.g., MGMT and PCBP1) showed binding activities to Cy5-labeled secondary antibody against mouse IgG when tested against the 17,000 protein microarrays. The non-specific interactors are excluded from further analysis.

*Pooling Analysis:* Based on the preliminary data, a pooling approach is used to rapidly identify mAbs that show monospecificity. Supernatants of hybridomas that secrete high levels of IgG-positive mAbs in "horizontal" and "vertical" pools as shown schematically in **FIG. 6****.** Antibodies are classified as monospecific if a protein on the microarray is recognized by only a single horizontal and vertical pool that both contain supernatant from the hybridoma in question. It is expected that roughly 10% of all hybridomas screened will i show monospecificity, and that a large number of such mAbs can be isolated. A total of 14 microarrays can be used to analyze the specificity of 49 different hybridomas, substantially increasing the throughput of analysis.

*Secondary Screening:* Antibodies determined to be monospecific by the analysis of pooled supernatants is then subject to a round of secondary screening using the protein microarrays, to further characterize both their affinity and specificity. Antibodies that recognize no more than three different proteins on the array will also be set aside for further analysis in this manner, although they will be given lower priority than the monospecific mAbs, as mAbs with highly selective (though not necessarily monospecific) protein recognition properties may also be useful in a range of applications. For these experiments, one human proteome chip is probed with each mAb at 10,000-fold dilution in the course of secondary screening. If a mAb can specifically recognize its corresponding target protein, meaning no obvious binding signals to any other proteins (excluding those non-specific ones as described above) on the chips is observed, the mAb is probed to a chip again at 50,000-fold dilution to help define an optimal dilution. If no binding signals is observed, the titer is increased to 100-fold and the chip is probed again. If binding signals occur, a 1000-fold dilution is added. Those showing no binding signals at 100-fold dilution are considered as failures. Therefore, for a typical antigen, 6 chips per antigen (test 3 mAbs per antigen, using 2 chips for each) is used for this second round of microarray-based screening.

The primary tool for evaluation of specificity is reaction with a single protein on the 17,000 antigen microarray as outlined above. Antibodies that show high specificity on the antigen microarray-based screen is tested further via a battery of tests that is consolidated into a testing workflow referred to as an application testing pipeline. The immunoblotting test checks the specificity of each antibody against the cognate antigen, and against at least three human cell extracts from diverse tissue culture cell types. These are tested with and without a mixed-in cognate antigen (since the antigen is GST tagged, it can be distinguished from the native antigen).

Several different neuroblastoma cell lines are used to carry out these additional studies of antibody specificity. A large quantity of gene expression data is publicly available at the Gene Expression Omnibus (GEO) website, which allows the ready determine of whether both the transcription factor in question and its putative target gene are expressed in human cell lines that are can be readily cultured and analyzed. As shown in **Table 2,** previously published microarray data has been analyzed to determine which readily available cell lines show robust levels of mRNA expression for each of the antigens used to generate monoclonal antibodies.

**Table 2: Antigens to be used for monoclonal antibody generation.**

| Gene | Associated disease | Antibody used for ChIP | Monoclonal available | IP grade | References |
|---|---|---|---|---|---|
| Arnt2 | PWS | No | 4 | 0 | |
| Arx | Austism, nsMR | No | 30 | 0 | Stromme, P., et al., Nat Genet, 2002. 30(4): p. 441-5; Bienvenu, T., et al.,. Hum Mol Genet, 2002.11(8): p. 981-91. |
| ATF4 | SZ | No | 10 | 0 | Qu, M., et al., Am J Med Genet B Neuropsychiatr Genet, 2008. 147B(6): p. 732-6 |
| Cux2 | MD | None | 0 | 0 | Glaser, B., et al., Am J Med Genet B Neuropsychiatr Genet, 2005. 132B(1). p. 38-45. |
| DIx2 | Autism | Rabbit polyclonal* | 27 | 0 | Liu, X., et al., Eur J Hum Genet, 2009. 17(2): p. 228-35 |
| Ent | Autism | No | 4 | 0 | Yang, P , et al., Neuropsychobiology, 2008. 57(1-2): p. 3-8; Gharani, N., et al., Mol Psychiatry, 2004. 9(5): p. 474-84; Benayed, R., et al.. Am J Hum Genet, 2005. 77(5): p. 851-68 |
| Foxg1 | RS | No | 1 | 0 | Ariani, F., et al., Am J Hum Genet, 2008. 83(1): p. 89-93 |
| Foxp2 | Autism | Goat polyclonal | 3 | 0 | MacDermot, K.D., et al., Am J Hum Genet, 2005. 76(6): p. 1074-80; Lai, C.S., et al., Nature, 2001. 413(6855): p. 519-23 |
| GTF2I | WBS | Goat polyclonal | 15 | 0 | Chimge, N.O., et al.,. Proc Natl Acad Sci U S A, 2008. 105(26): p. 9006-10Perez Jurado, L.A., et al., Hum Mol Genet, 1998. 7(3): p. 325-34 |
| Hoxal | Autism | No | 0 | 0 | Tischfield, M.A., et al., Nat Genet, 2005. 37(10): p. 1035-7; Ingram, J.L., et al., Teratology, 2000. 62(6): p. 393-405 |
| Isl1 | NI | No | 19 | 0 | Elshatory, Y. and L. Gan, J Neurosci, 2008. 28(13): p. 3291-7; Thaler, J.P., et al., Neuron, 2004. 41(3): p. 337-50 |
| Isl2 | NI | No | 5 | 0 | Thaler, J.P., et al., Neuron, 2004. 41(3): p. 337-50 |
| Lhxl | NI | No | 39 | 0 | Huang, K., et al., Hum Genet, 2008. 122(6): p. 659-60 |
| Lhx2 | NI | Rabbit polyclonal* | 3 | 0 | Mangale, V.S., et al., Science, 2008. 319(5861): p. 304-9 |
| Lhx3 | NI | Rabbit polyclonal* | 5 | 0 | Sharma, K., et al., Cell, 1998. 95(6): p. 817-28 |
| Lhx4 | NI | No | 34 | 1 | Sharma, K., et al., Cell, 1998.95(6): p. 81.7-28 |
| Lhx5 | NI | No | 18 | 0 | Huang, K., et al., Hum Genet, 2008. 122(6): p. 659-60. |
| Lhx6 | NI | No | 18 | 0 | Ariani, F., et al., Am J Hum Genet, 2008. 83(1): p. 89-93; Nobrega-Pereira, S., et al., Neuron, 2008. 59(5): p. 733-45; Choi, G.B., et al., Neuron, 2005. 46(4): p. 647-60 |
| Lhx9 | NI | No | 2 | 0 | Kumar, R.A., et al., Am J Med Genet B Neuropsychiatr Genet, 2008. 147B(6): p. 880-9 |
| Mecp2 | Autism, RS, PWS/AS | Rabbit polyclonal | 20 | 0 | Yasui, D.H., et al., Proc Natl Acad Sci U S A, 2007. 104(49): p. 19416-21; Chahrour, M., et al., Science, 2008. 320(5880) p. 1224-9; Watson, P., et al., J Med Genet, 2001 38(4): p. 224-8; Dotti, M.T., et al., Neurology, 2002. 58(2): p. 226-30; Amir, R.E., et al., Nat Genet, 1999. 23(2): p. 185-8; Innessaaudene, B., et al., |
| | | | | | J Med Genet, 2001. 38(3): p. 171-4; Urdinguio, R.G., et al., PLoS ONE, 2008. 3(11). p. e3669; Vernes, S.C., et al., Am J Hum Genet, 2007. 81(6): p. 1232-50; , M., et al., Am J Hum Genet, 1999. 65(6): p. 1520-9 |
| Mef2C | Autism | No | 0 | 0 | Li, H., et al. Proc Natl Acad Sci USA, 2008. 105(27): p. 9397-402. |
| NDN | PWS/AS | Rabbit polyclonal | 17 | 0 | Miller, N.L., R. Wevriclc, and P.L. Mellon, Hum Mol Genet, 2009.18(2). p. 248-60; Kurita, M., et al., J Neurosci, 2006. 26(46): p. 12003-13 |
| NEURO | SZ | No | 18 | 0 | Ho, B.C., et al., Schizophr Res, 2008. 106(2-3): p. 192-9; Yang, P, et al., Neuropsychobiology, 2008. 57(1-2): p. 3-8 |
| NPAS3 | SZ | No | 0 | 0 | Pickard, B.S., et al., Am J Med Genet B Neuropsychiatr Genet, 2005. 136B(1): p. 26-32.; Pickard, B.S., et al., Mol Psychiatry, 2008. |
| NR2E1 | MD | None | 18 | 3 | Kumar, R.A., et al.,. Am J Med Genet B Neuropsychiatr Genet, 2008. 147B(6): p. 880-9 |
| NURR1 | SZ | No | 43 | 3 | Rojas, P., et al., Mol Psychiatry, 2007. 12(8): p. 756-66; Buervenich, S., et al., Am J Med Genet, 2000. 96(6): p. 808-13 |
| Olig2 | SZ | No | 14 | 0 | Huang, K., et al., Hum Genet, 2008. 122(6): p. 659-60; Georgieva, L., et al., Proc Natl Acad Sci USA, 2006. 103(33) p. 12469-74 |
| PHF6 | BFLS | No | 0 | 0 | Lower, K.M., et al., Nat Genet, 2002. 32(4): p. 661-5; Mangelsdorf, M., et al., J Child Neural, 2009 |
| Pitxl | Autism | No | 10 | 0 | Philippi, A., et al., BMC Med Genet, 2007. 8: p. 74 |
| PMX2B | SZ | No | 0 | 0 | Toyota, T., et al., Hum Mol Genet, 2004. 13(5): p. 551-61 |
| RAI1 | SMS | No | 0 | 0 | Slager, R.E., et al., Nat Genet, 2003. 33(4): p. 466-8; Seranski, P., et al., Genomics, 1999. 56(1): p. 1-11 |
| Rax | Autism | No | 30 | 0 | Voronina, V.A., et al., Hum Mal Genet, 2004. 13(3): p. 315-22. |
| REST | NSMR | Rabbit polyclonal | 1 | 0 | Tahiliani, M., et al., Nature, 2007. 447(7144): p. 601-5 |
| Sim1 | PWS | No | 0 | 0 | Faivre, L., et al., J Med Genet, 2002. 39(8): p. 594-6; Fyffe, S.L., et al., Neuron, 2008. 59(6) p. 947-58 |
| SOX10 | SZ | No | 4 | 0 | Maeno, N., et al., Psychiatr Genet, 2007. 17(4): p. 227-31 |
| Sox3 | NSMR | No | 8 | 0 | Laumonnier, F., et al., Am J Hum Genet, 2002. 71(6): p. 1450-5; Hamel, B.C., et al., Am J Med Genet, 1996. 64(1). p. 35-41 |
| SREBF1 | SZ | No | 16 | 0 | Le Hellard, S., et al., Mol Psychiatry, 2008 |
| SREBF2 | SZ | No | 0 | 0 | Le Hellard, S., et al., Mol Psychiatry, 2008 |
| Tbx1 | SZ | No | 0 | 0 | Paylor, R., et al., Proc Natl Acad Sci USA, 2006. 103(20) p. 7729-34 |
| TGIF | SZ | No | 43 | 3 | Chavarria-Siles, I., et al., Mol Psychiatry, 2007. 12(11): p. 1033¬41 |
| Xbp1 | MD | None | 12 | 0 | Grunebaum, M.P., et al., Int J Neuropsychopharmacol, 2009. 12(2) p. 281-3; Kakiuchi, C., et alNat Genet, 2003. 35(2): p. 171-5 |
| ZBTB16 | NSMR | No | 5 | 0 | Fischer, S., et al., J Med Genet, 2008. 45(11). p. 731-7. |
| ZNF41 | NSMR | No | 31 | 0 | Shoichet, S.A., et al., Am J Hum Genet, 2003. 73(6). p. 1341-54 |
| ZNF674 | NSMR | No | 0 | 0 | Lugtenberg, D., et., Am J Hum Genet, 2006 78(2): p. 265-78. |
| ZNF74 | SZ | No | 0 | 0 | Takase, K., et al., Schizophr Res, 2001 52(3): p. 161-5. |
| ZNF804 | SZ | No | 0 | 0 | O'Donovan, M.G., et al., Nat Genet, 2008. 40(9): p. 1053-5. |

| | | | | | |
|---|---|---|---|---|---|
| Abbreviations: BFLS = Borjenson-Forssman-Lehmann Syndrome MD = mood disorder, NI = none identified, NSMR = nonsyndromic mental retardation, PWS/AS = Prader-Willi Syndrome/Angelman Syndrome, RS = Rett Syndrome, SZ = schizophrenia, WBS = Williams-Beuren Syndrome. * indicates ChIP grade antibody is not commercially available. | | | | | |

For most antigens of interest, the well-characterized SH-SY5Y neuroblastoma cell line is used. In other cases, however, the mRNA corresponding to the antigen of interest is significantly more highly expressed, for example in a different readily cultured neuroblastoma or nonneuronal cell line. These are, indicated in **Table 3.**

**Table 3: Cell lines to be used for immunohistochemical and/or chromatin immunoprecipitation (ChIP)-based analysis of antibodies generated. Target genes to be analyzed for ChIP are also listed when known.**

| Gene | Cell line to be used | Reference | Target gene(s) | Reference |
|---|---|---|---|---|
| Arnt2 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p.8213-21 | Trb2 | Liu, C., et al., J Biol Chem, 2003. 278(45): p. 44857-67 |
| Arx | SK-N-MC | Staege, M.S., et al., Cancer Res, 2004. 64(22): p. 8213-21 | ND | |
| ATF4 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | CHOP | Cherasse, Y., et al., Nucleic Acids Res, 2007. 35(17): p. 5954-65 |
| Cux2 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | Neurod1, p27 | lulianella, A., et al., Development, 2008. 135(4): p. 729-41 |
| DIx1 | SH-SY5Y | Peddada, S., D.H. Yasui, and J.M. LaSalle, Hum Mol Genet, 2006. 15(12): p. 2003-14 | DIx5/6 | Zhou, Q.P., et al., Nucleic Acids Res, 2004. 32(3): p. 884-92. |
| DIx2 | SK-N-MC | Staege, M.S., et al., Cancer Res, 2004. 64(22): p. 8213-21 | Arx, DIx5/6 | Zhou, Q.P., et al., Nucleic Acids Res, 2004. 32(3): p. 884-92; Colasante, G., et al., J Neurosci, 2008. 28(42): p. 10674-86. |
| Ent | SH-SY5Y | Peddada, S., D.H. Yasui, and J.M. LaSalle, Hum Mol Genet, 2006. 15(12): p. 2003-14 | ND | |
| Foxg1 | IMR-32 | Peng, J., et al., Mol Cell Biochem, 2007. 294(1-2)- p. 45-54. | ND | |
| Foxp2 | SH-SY5Y | Peddada, S., D.H. Yasui, and J.M. LaSalle, Hum Mol Genet, 2006. 15(12): p. 2003-14 | Neurod2*, Pax3* | Vernes, S.C., et al., Am J Hum Genet, 2007. 81(6): p. 1232-50. |
| GTF2I | SH-SY5Y | Peddada, S., D.H. Yasui, and J.M. LaSalle, Hum Mol Genet, 2006. 15(12): p. 2003-14 | HDAC1 | Chimge, N.O., et al., Proc Natl Acad Sci USA, 2008. 105(26): p. 9006-10 |
| Hoxal | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | ND | |
| Isl1 | SH-SY5Y | Peddada, S., D.H. Yasui, and J.M. LaSalle, Hum Mol Genet, 2006. 15(12): p. 2003-14 | ISL1, SHH | Ariani, F., et al., Am J Hum Genet, 2008. 83(1): p. 89-93 |
| Is12 | SK-N-SH | Wang, Z., et al., J Biol Chem, 2006. 281(30): p. 21377-86. | PLXNA4 | Miyashita, T., et al., Development, 2004. 131(15): p. 3705-15 |
| Lhx1 | IMR-32 | Peng, J., et al., Mol Cell Biochem, 2007. 294(1-2)- p. 45-54. | ND | |
| Lhx2 | IMR-32 | Peng, J., et al., Mol Cell Biochem, 2007. 294(1-2)-p. 45-54. | SIX3 | Tetreault, N., M.P. Champagne, and G. Bemier, Dev Biol, 2009. 327(2): p. 541-50 |
| Lhx3 | IMR-32 | Peng, J., et al., Mol Cell Biochem, 2007. 294(1-2)- p. 45-54. | HB9 | Savage, J.J., et al., Gene, 2007. 400(1-2): p. 44-51 |
| Lhx4 | IMR-32 | Peng, J., et al., Mol Cell Biochem, 2007. 294(1-2)-p. 45-54. | ND | |
| Lhx5 | HepG2 | Nguyen, H., S. Sankaran, and S. Dandekar, Virology, 2006. 354(1): p. 58-68 | ND | |
| Lhx6 | EFTHALNP | Staege, M.S., et al., Cancer Res, 2004. 64(22): p. 8213-21 | ND | |
| Lhx9 | IMR-32 | Peng, J., et al., Mol Cell Biochem, 2007. 294(1-2)- p. 45-54. | ND | |
| Mecp2 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | EGR2 | Swanberg, S.E., et al., Hum Mol Genet, 2009. 18(3): p. 525-34 |
| Mef2C | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | Asc11 | Skerjanc, I.S. and S. Wilton, FEBS Lett, 2000. 472(1): p. 53-6. |
| NDN | SH-SY5Y | Peddada, S., D.H. Yasui, and J.M. LaSalle, Hum Mol Genet, 2006. 15(12): p. 2003-14 | CDC2 | Kurita, M., et al., J Neurosci, 2006. 26(46): p. 12003-13 |
| NEUR OG1 | SH-SY5Y | Peddada, S., D.H. Yasui, and J.M. LaSalle, Hum Mol Genet, 2006. 15(12): p. 2003-14 | Pk2 | Huang, K., et al., Hum Genet, 2008. 122(6): p. 659-60 |
| NPAS3 | SH-SY5Y | Peddada, S., D.H. Yasui, and J.M. LaSalle, Hum Mol Genet, 2006. 15(12): p. 2003-14 | ND | |
| NR2E1 | SH-SY5Y | Peddada, S., D.H. Yasui, and J.M. LaSalle, Hum Mol Genet, 2006. 15(12): p. 2003-14 | p21 | Sun, G., et al., Proc Natl Acad Sci U S A, 2007. **104**(39): p. 15282-7. |
| NURR 1 | CHP-126 | Staege, M.S., et al., Cancer Res, 2004. 64(22): p. 8213-21 | Bdnf | Volpicelli, F., et al., J Neurochem, 2007. 102(2): p. 441-53 |
| Olig2 | IMR-32 | Peng, J., et al., Mol Cell Biochem, 2007. 294(1-2)-p. 45-54. | Simi | Borodovsky, N., et al., Dev Dyn, 2009. |
| PHF6 | IMR-32 | Peng, J., et al., Mol Cell Biochem, 2007. 294(1-2)- p. 45-54. | ND | |
| Pitx1 | SH-SY5Y | Peddada, S., D.H. Yasui, and J.M. LaSalle, Hum Mol Genet, 2006. 15(12): p. 2003-14 | p53 | Liu, D.X. and P.E. Lobie, Cell Death Differ, 2007. 14(11): p. 1893-907 |
| PMX2B | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | ND | |
| RAI1 | IMR-32 | Peng, J., et al., Mol Cell Biochem, 2007. 294(1-2)- p. 45-54. | ND | |
| Rax | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | Hmgb3 | Terada, K., et al., Dev Biol, 2006. 291(2) p. 398-412 |
| REST | IMR-32 | Peng, J., et al., Mol Cell Biochem, 2007. 294(1-2)- p. 45-54. | Scn2a, Syn1 | Tahiliani, M., et al., Nature, 2007. 447(7144): p. 601-5. |
| Sim1 | SK-N-SH | Chen, J.Y., et al., Ai Zheng, 2006. 25(2): p. 170-4. | Trb2 | Liu, C., et al., J Biol Chem, 2003. 278(45): p. 44857-67 |
| SOX10 | SH-SY5Y | Peddada, S., D.H. Yasui, and J.M. LaSalle, Hum Mol Genet, 2006. 15(12): p. 2003-14 | Sox10 | Lee, K.E., et al., BMC Genomics, 2008. 9: p. 408 |
| Sox3 | MCF-7 | Stitziel, N.O., et al., Cancer Res, 2004. 64(23): p. 8682-7 | ND | |
| SREBF1 | SH-SY5Y | Peddada, S., D.H. Yasui, and J.M. LaSalle, Hum Mol Genet, 2006. 15(12): p. 2003-14 | Fas | Furuta, E., et al., Cancer Res, 2008. 68(4): p. 1003-11 |
| SREBF2 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | ABCA1 | Zeng, L., et al., J Biol Chem, 2004. 279(47): p. 48801-7 |
| Tbx1 | SK-N-MC | Staege, M.S., et al., Cancer Res, 2004. 64(22): p. 8213-21 | ND | |
| TGIF | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | RARB | Bartholin, L., et al., Mol Cell Biol, 2006. 26(3): p. 990-1001 |
| Xbp1 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | Wfs1* | Kakiuchi, C., et al., J Neu rochem, 2006. 97(2). p. 545-55 |
| ZBTB16 | SK-N-MC | Staege, M.S., et al., Cancer Res, 2004. 64(22): p. 8213-21 | CDC6 | Petrie, K., et al., Oncogene, 2008. **27**(39). p. 5260-6. |
| ZNF41 | IMR-32 | Peng, J., et al., Mol Cell Biochem, 2007. 294(1-2)- p. 45-54. | ND | |
| ZNF674 | IMR-32 | Peng, J., et al., Mol Cell Biochem, 2007. 294(1-2)- p. 45-54. | ND | |
| ZNF74 | SH-SY5Y | Peddada, S., D.H. Yasui, and J.M. LaSalle, Hum Mol Genet, 2006. 15(12): p. 2003-14; Staege, M.S., et al., Cancer Res, 2004. 64(22): p. 8213-21 | ND | |
| ZNF804A | SH-SY5Y | Peddada, S., D.H. Yasui, and J.M. LaSalle, Hum Mol Genet, 2006. 15(12): p. 2003-14; Staege, M.S., et al.. Cancer Res, 2004. 64(22): p. 8213-21 | ND | |

| | | | | |
|---|---|---|---|---|
| * indicates target gene is inferred from loss of function analysis rather than directly verified by ChIP, luciferase analysis, etc | | | | |

If this is the case, these other cell lines are used for immunocytochemical analysis, and for chromatin immunoprecipitation, as described later. To determine whether monoclonals generated in the course of the screen can detect endogenous protein via immunoblot, the following test panel is used: Antigen alone, line 1-3 extracts, line 1-3 extracts + antigen mixed in at 1 µGm/mL. For the immunoprecipitation (IP) test, the same starting materials is used, except the final readout is an immunoblot (IB) with anti-GST to detect the antigen. To examine expression of endogenous protein via immunohistochemistry (IHC), indirect immunofluorescence-based detection is conducted to screen neuroblastoma cells grown on poly-D-lysine coated coverslips. As a negative control, a mouse fibroblast cell line (3T3) is screened.

A limited number of antibodies that are confirmed as specific by both these tests is also screened for their ability to successfully mediate chromatin immunoprecipitation (ChIP). To this end, a quantitative ChIP protocol (Onishi, A., et al., Pias3-dependent SUMOylation directs rod photoreceptor development. Neuron, 2009. 61(2): p. 234-46; Peng, G.H. and S. Chen, Chromatin immunoprecipitation identifies photoreceptor transcription factor targets in mouse models of retinal degeneration, new findings and challenges. Vis Neurosci, 2005. 22(5): p. 575-86.) is used. Briefly, 1 microgram of each antibody is used to immunoprecipitate a same amount of formaldehyde-crosslinked, and sonication-sheared chromatin from cultured cells (5 x 10⁶ cells). Specific genomic regions enriched in the IP samples versus input controls will be detected and quantified using real-time qPCR with gene specific primers. Non-specific mouse IgG will be used as a negative control. The IP/Input ratios in various genomic regions of a gene will be used to compare different antibodies made to a same protein for their specificity and effectiveness. The cell lines that express the target antigens at high levels, as indicated in **Table 3,** is employed for this analysis.

Several criteria is taken into account when selecting genomic regions for amplification and analysis. In the case of monoclonals directed against antigens for which ChIP-qualified polyclonal antibodies are already available (as indicated in **Table 2),** genomic regions and cell types in which express the protein in question and where the target genes of that transcription factor are already known are chosen, so that the effectiveness of the new reagents can be gauged. In the case of a number of these antigens, ChIP analysis of SH-SY5Y cells has already been conducted using polyclonal antibodies, and in these cases genomic regions already shown to be bound by these factors in this cell type is analyzed. For other proteins, ChIP analysis has been conducted in other cell or tissue types, although usually with an antibody that is not commercially available. Table 3 lists both known and proposed direct target genes for the antibodies to be generated. In cases where ChIP has already been performed for target genes of these factors, previously tested primer sets are used. In other cases, bioinformatic tools such as TRANSFACdb is used to identify consensus binding sites for these factors, and then design primer pairs that flank evolutionarily conserved sequences corresponding to sites that are found in putative regulatory regions for these transcripts, or have been characterized using high-throughput protein microarray-based analysis of protein-DNA interaction. By combining this bioinformatic data with previously published gene expression data, a list of high-probability direct targets for transcription factors under investigation is developed, and primer sets are used to for ChIP analysis on the antibodies

### Example 7: Characterization of monospecific monoclonal antibodies (mMAbs):

*Monospecific MAb ICC validation.* To further characterize the mMAbs generated using the shotgun approach, high resolution immunocytochemistry (ICC) was carried out to assess the ability of the mMAbs to bind to proteins in fixed cells. Permeabilized HeLa, HepG2, or HCT-116 cells were fixed in paraformaldehyde and blocked in 3% BSA + 3% FBS. Each mMAb was diluted to 10 µg/mL and incubated overnight at 4¹/⁴ C. DyLight™ conjugated Goat anti-Mouse IgG antibody (Rockland) was used as the secondary antibody. Stained cells were observed and images taken using a fluorescent microscope. Five simple categories, including nucleus, mitochondria, ER/Golgi, cytosol, and plasma membrane, were used to classify subcellular localization of a given antigen detected by the mMAbs.

As expected given that HeLa cells were the source antigen, of the 59 mMAbs tested 52 produced distinguishable patterns in HeLa cells,whereas 6 and 1 mMAbs correctly recognized their corresponding antigens in HCT-116 and HepG2 cells, respectively. The distribution of subcellular localization of these 59 human proteins is summarized in **Table 4.**

**Table 4. Summary of antigen subcellular localization in cells**

| | Nucleus | Mitochondria | ER/Golgi | Cytosol | Membrane |
|---|---|---|---|---|---|
| % of total | 17.8% | 6.6% | 13.3% | 37.8% | 24.4% |

By comparing the ICC results with the literature and databases (e.g., NCBI), 39 of these matched known patterns, whereas 16 were mismatched. This discrepancy likely reflects differences in the cell lines/tissues tested and/or differences in underlying biology. In addition, four antigens whose subcellular location was unknown, namely C11orf68, CNTD1, DYDC2, andTXNDC9, have now been localized to the mitochondria, cytosol, plasma membrane, and ER/Golgi, respectively (**Fig. 8****).** Therefore, the mMAbs generated not only showed a 100% ICC success rate, but also helped determine localization of proteins for which that information was previously unknown.

*Validation* / *characterization of mMAbs using immunoblot and shRNA-based gene knockdown.* We are constructing a human tet-regulated plasmid expression vector. Forty-two human ORFs have been subcloned into a commercial mammalian expression vector, pcDNA3.1-v5 (Invitrogen), via the Gateway® LR recombination, in order to overexpress them as V5-tagged fusion proteins in HeLa. In brief, all plasmids were amplified in *E. coli* DH5α and isolated by alkaline lysis. Following purification, all clones were verified by restriction digestion. To transiently transfect human cells, HeLa cells plated at ∼0.6 x 10⁴ cells/well in 6-well tissue culture plates were transfected with the corresponding ORF expression constructs. Two days after transfection, cells were lysed and a portion of each lysate was then subjected to immunoblot analysis using the mMAbs (1:1000 dilution).

Also included in the immunoblot analysis were cells transfected with the empty vector DNA as a negative control. In some cases, a given mMAb detect ed two bands in transfected cells. For example, α-EFHD2 recognized two bands in cells transfected with V5-tagged EFHD2 but only one in vector-transfected cells (**FIG. 9****,** Lanes 1, 2, upper left panel). When the same blot was stripped and probed with anti-V5 antibody, the faster migrating bands disappeared in both cells, indicating that α-EFHD2 faithfully detected both V5-tagged and native EFHD2 in cells. Of the 42 mMAbs tested, 30 (71%) were confirmed as correctly detecting the corresponding proteins, by comparing the mMAb with the V5 blots (**FIG. 9****,** Lanes 1, 2 in upper and lower panels).

To further confirm that these mMAbs were highly specific, gene knockdown analysis using shRNAs was performed. One microgram of expression construct was co-transfected with 3µg of shRNA expression construct in HeLa cells. Cells were harvested 24∼36 hr post-transfection, lysed and subjected to immunoblot analysis using the corresponding mMAbs and anti-V5 antibody (**FIG**. 9, Lanes 3 in upper and lower panels). In all six cases tested (4 of these are shown in **FIG. 9****),** the corresponding shRNAs effectively knocked down expression of the antigens, as determined by both mMAb and anti-V5 immunoblotting. These results directly confirm a very stringent test that the mMAbs produced in the pipeline are very likely to be highly specific, and thereby highly useful for immunoblot analysis. Moreover, they provide the ultimate validation that throughout the entire pipeline, from making the arrays to interpreting the array images, the correct identity of the proteins is maintained, as the shRNA knockdown reagents come from a completely distinct collection (Sigma) than the ORF collection (Invitrogen) used to express the proteins that are put on the array.

### Characterization of mMAbs using Immunoprecipitation

An immunoprecipitation (IP) validation protocol for human cells was performed. HeLa cells were transfected with a plasmid expressing the antigen of interest in the form of an N-terminally V5 tagged protein and lysed. Antigens were immunoprecipitated using corresponding mAAbs and analyzed using immunoblotting with an anti-V5 antibody. Anti-mouse IgG and anti-V5 antibodies were used in the IP as negative and positive controls, respectively. Sixteen of 27 tested mMAbs were shown to effectively IP their corresponding antigens in HeLa cells, an overall success rate of 59% (examples shown in **FIG. 10****).** Therefore, a high proportion of the mMAbs is IP-grade.

### Characterization of mMAbs using chromatin immunoprecipitation.

Many unconventional DNA-binding proteins (uDBPs) show sequence-specific binding property, including an RNA-binding protein HNRPC, one of the hnRNPs (heterogeneous nuclear ribonucleoproteins) (Hu et al, *Cell* 2009). The hnRNPs are known to complex with heterogeneous nuclear RNAs (hnRNAs). They are also associated with pre-mRNAs in the nucleus and appear to influence pre-mRNA processing and other aspects of mRNA metabolism and transport. Although the hnRNP proteins have distinct nucleic acid binding properties, such as forming the 40S hnRNP particle, recent studies using the protein binding microarray (PBM) showed that it also binds to both double-and single-stranded DNAsin a sequence-dependent fashion. Because a highly specific mMAb against HNRPC (Isotype IgG2b) was generated in the CDI pipeline, we decided to fully characterize this antibody using a variety of standard assays.

As illustrated in **FIG. 11A****,** ICC staining with this antibody in HeLa cells showed a clear cut nuclear localization of HNRPC, consistent with its known subcellular localization (i.e., nucleoplasm and pronucleus). Since this mMAb could detect native HNRPC in HeLa cells, testing to determine whether it is useful for IP analysis was performed, before testing if it could specifically immunoprecipitate HNRPC after crosslink to DNA.

Two million HeLa cells overexpressing V5-tagged HNRPC were lysed, and 20 µg of the anti-HNRPC mMAb was added to the lysate and allowed to incubate overnight. Protein G- conjugated beads were added to capture the antibody, followed by extensive washes. Anti-mouse IgG and anti-V5 antibodies were also used in parallel IP experiments as negative and positive controls, respectively. The supernatants of boiled beads were then immunoblotted with anti-V5 antibody. As shown in **FIG. 11B****,** a single band of V5- tagged HNRPC is detected at the same molecular weights as those observed in both the input and anti-V5 lanes, indicating the success of the HNRPC IP using this mMAb. As expected, the IgG negative control did not show any signals.

We next performed chromatin immunoprecipitation (ChIP) against endogenously expressed HNRPC, to determine whether this mMAb is capable of ChIP of HNRPC and, if successful, what specific DNA sequences HNRPC proteinis associated with *in vivo*. Briefly, 5X10⁷ of HeLa cells were crosslinked, quenched, harvested, and washed. After cell lysis under mild conditions, nuclear pellets were collected, washed, and sheared by sonication. To ChIP HNRPC, 10 µg anti-HNRPC antibody (or 10 µg mouse IgG as a negative control) was added and incubated at 4°C overnight. Protein G bound Dynabeads were added to capture DNA-HNRPC complexes, followed by elution. ∼10% of the eluates were used for immunobloting. As shown in **FIG. 11C****,** endogenously expressed HNRPC could be ChIPd as observed in both 1^{st} and 2^{nd} elution, while the IgG control showed no detectable signals.
To determine whether any DNA fragments were ChIPd with the antibody, the ChIPd complex was reverse-crosslinked at 65°C overnight. Because HNRPC is a known RNA-binding protein, the eluates were treated with RNase, followed by deproteinization. The quality and concentration of the concentrated DNA samples were determined using the Agilent 2100 Bioanalyzer. As demonstrated in **FIG. 11D****,** a high yield of high quality DNA was obtained.

### Sequential Measurement of MAb Affinity

Dynamics of three antigen-antibody interactions is simultaneously monitored in real-time using unlabeled antibodies as shown in **FIG. 12****.** The three antibodies were sequentially injected to the flow chamber covering the IgG microarray and the binding signals appeared to reach saturation around 20 min as indicated with the red lines in **FIG. 12****.** This data demonstrates the feasibility of this method of sequential measuring of avidity of MAbs in a high-throughput system.

**Example 8:** Platform for production and characterization of monospecific monoclonal antibodies (mMAbs):

A protein microarray comprised of 17,263 unique full-length human proteins comprising over 70% of the annotated human proteome was constructed. To express and purify these proteins, the Invitrogen Ultimate ORF collection along with 400 additional ORFs was subcloned into a yeast expression vector which allows galactose-dependent overexpression of N-terminal GST- and 6x-His tagged recombinant proteins. The quality of the purified proteins was determined by both GST immunobloting and silver staining of a random subset of recombinant proteins. Based on these results, approximately 85% of all proteins could be purified at high yield to at least 80% purity. A microarray comprised of these recombinant proteins was fabricated and tested its quality by probing with anti-GST antibody. Over 85% of spotted proteins produced significant signals above background.

Highly complex mixtures of antigens, such as whole cells or tissues, to generate monoclonal antibodies (mAbs) work well for immunocytochemistry (ICC). Generating mAbs in this "shotgun" manner increases the likelihood that the mAbs will recognize native protein epitopes, thus increasing the range of potential applications. However, the major limitation of this approach has been the difficulty in identifying the antigen preferentially recognized by a given mAb, which has typically only been possible via affinity purification coupled with mass spectrometry. By directly screening mAbs against human proteome microarrays, it is possible to directly identify the corresponding antigen recognized by a given mAb generated by immunization with complex biological samples.

Using this shotgun approach, mice were immunized with a variety of different cancer cell lines. Live human cells were injected directly into the footpad, and three weeks later, politeal lymph nodes were collected and hybridomas generated. To enrich for useful mAbs, a high-throughput ICC prescreening step against one or more cell lines was conducted using supernatants from !2200 hybridomas. ICC-positive hybridomas were then grown further and isotyped, and IgG isotype-positive supernatants selected for microarray analysis.

Individual supernatants were combined into sets of 12x12 two-dimensional pools, and these pools were then incubated on the human proteome microarrays, and incubated with a Cy5-coupled anti-IgG secondary antibody. Following washing and scanning, the signal intensity for each spot was expressed as the ratio of foreground to background signal. The number of standard deviations above mean signal intensity across the entire array for each duplicate pair of proteins, a value which we termed A, was then measured. Duplicate spots for which A>3 were then flagged, and results deconvoluted to identify proteins that were present at the intersection of a single horizontal and single vertical pool, and were thus recognized by an individual mAb. To confirm the specificity of these mAbs, each candidate highly specific mAb was then tested individually against the entire array, and A measured for each spotted protein. The signal intensities were then rank ordered and the difference between the top signal and the second-highest signal on the array calculated, which we expressed as S. A number of mAbs were obtained where A>6 and S>3 were identified, which we termed monospecific mAbs (mMAbs). In many cases, mMAbs showed extremely high selectivity (i.e. S>100). A number of mAbs bound intensely to two different proteins on the array (A>6, S<3), which were termed dispecific mAbs (dMAbs).

To further characterize the utility and specificity of the mMAbs and dMAbs, a variety of different assays were conducted. First the ICC analysis on human cancer cell lines was repeated and images collected to assess the subcellular expression pattern of the target antigen. Confirmation of the observed specificity of individual mAbs comes from the finding that 85% of all ICC patterns obtained in this study matched those previously reported for their target proteins when this data was available. The subcellular distribution of the proteins for which high-quality mAbs were obtained showed no significant preference for any particular cellular compartment, confirming that shotgun immunization can generate mAbs to a wide range of cellular proteins.

Next, the ability to detect their top target protein using inmmnoblot analysis was analyzed. Individual target proteins were overexpressed in HeLa cells as N-terminal V5-tagged fusion proteins, and expression was confirmed via immunoblotting to V5. Out of 56 mAbs tested, 31 (55%) recognized their target protein, while 18 (33%) recognized endogenous untagged protein in these cells. To further confirm the accuracy of these results, the V5-tagged ORF was cotransfected with individual shRNAs targeting this gene. In all 9 cases tested, a substantial reduction in immunoblot signal was observed, but control shRNAs gave no corresponding signal reduction.

Next, these mAbs were tested to determine if they would work effectively for immunoprecipitation, and thus recognize native proteins in cell homogenates. Out of 51 mAbs tested, 28 (55%) could efficiently precipitate transfected protein, which was then detected via immunoblotting to V5. A considerable fraction of mAbs work effectively in both immunoblotting and immunoprecipitating applications. Taken together, the protein microarray-based shotgun approach is a rapid and effective way to generate highly specific mAbs useful for a broad range of applications.

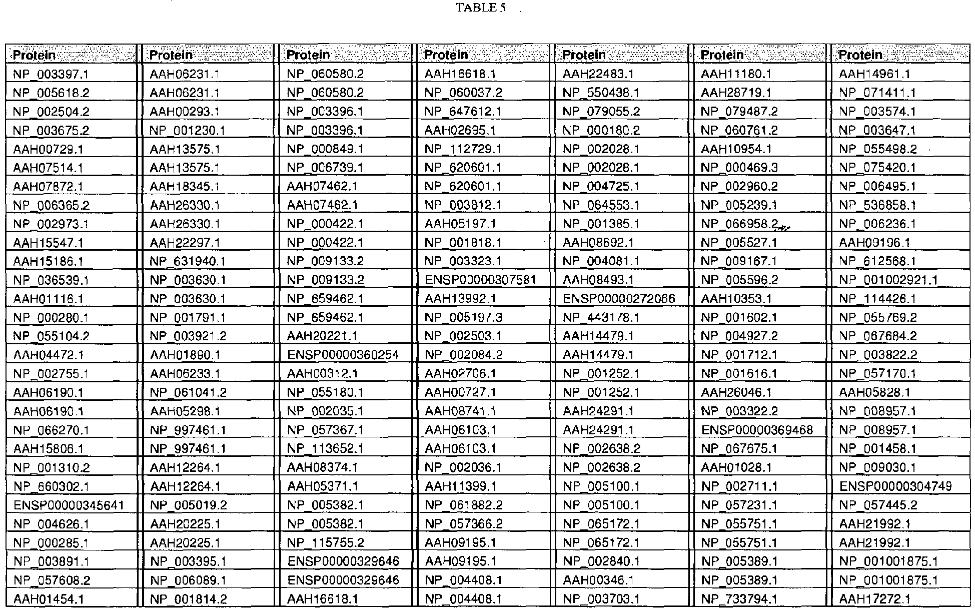

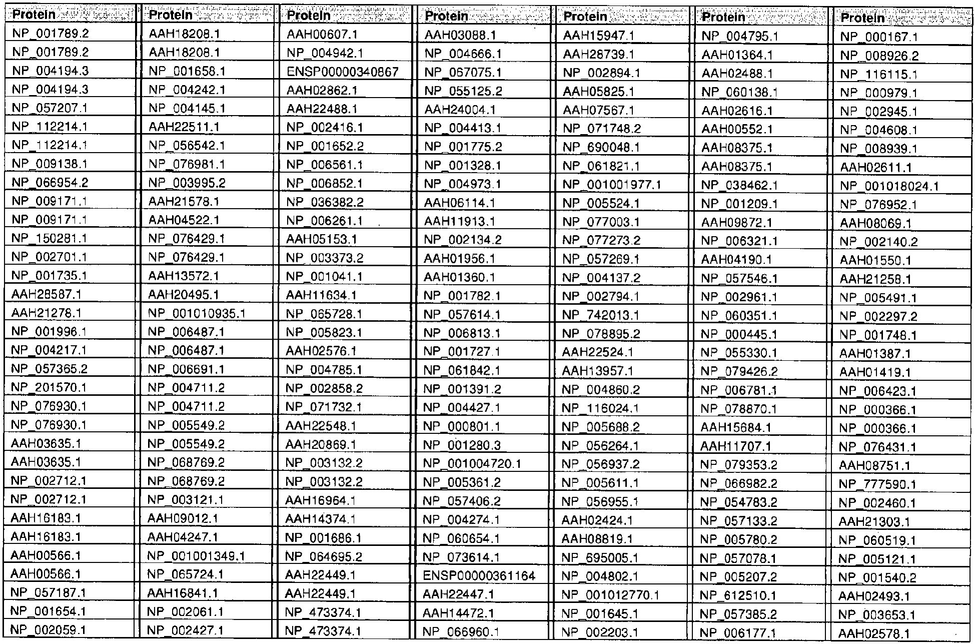

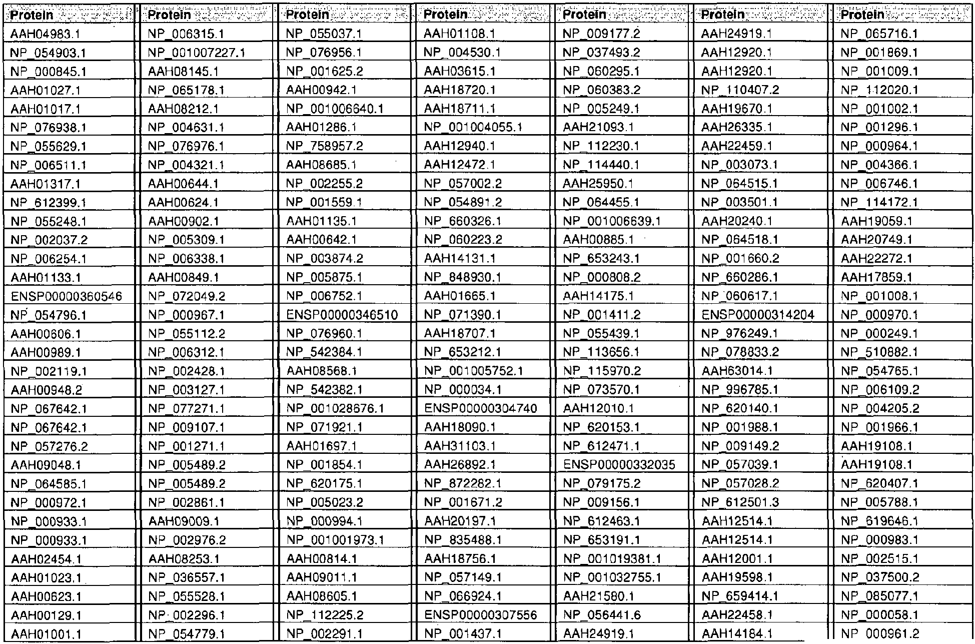

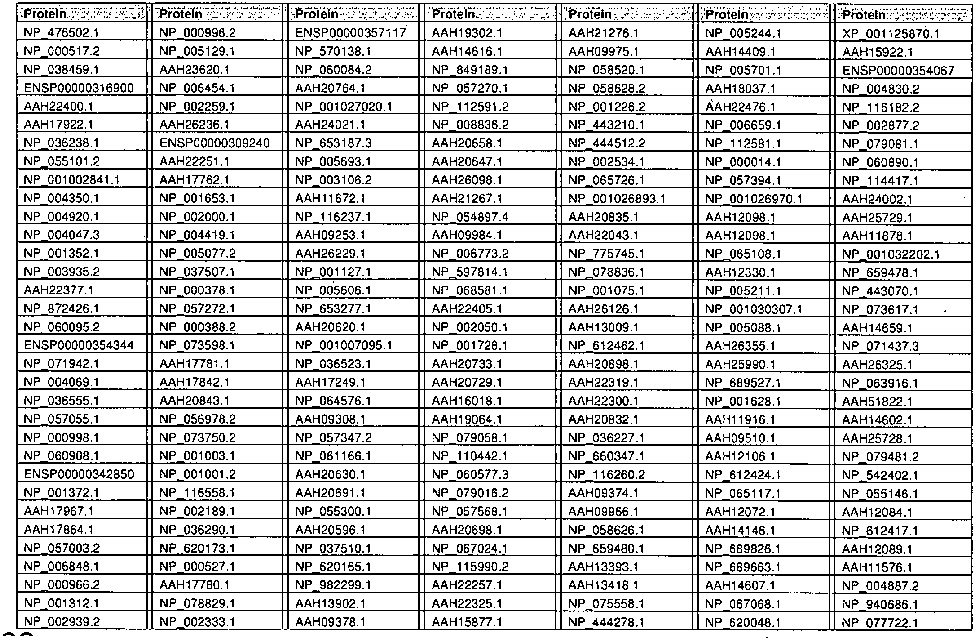

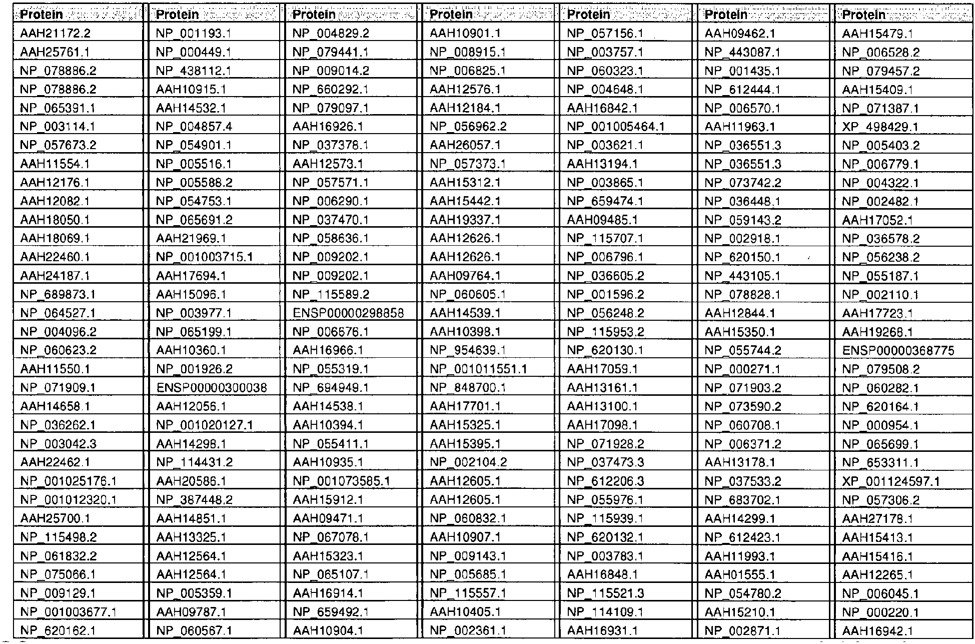

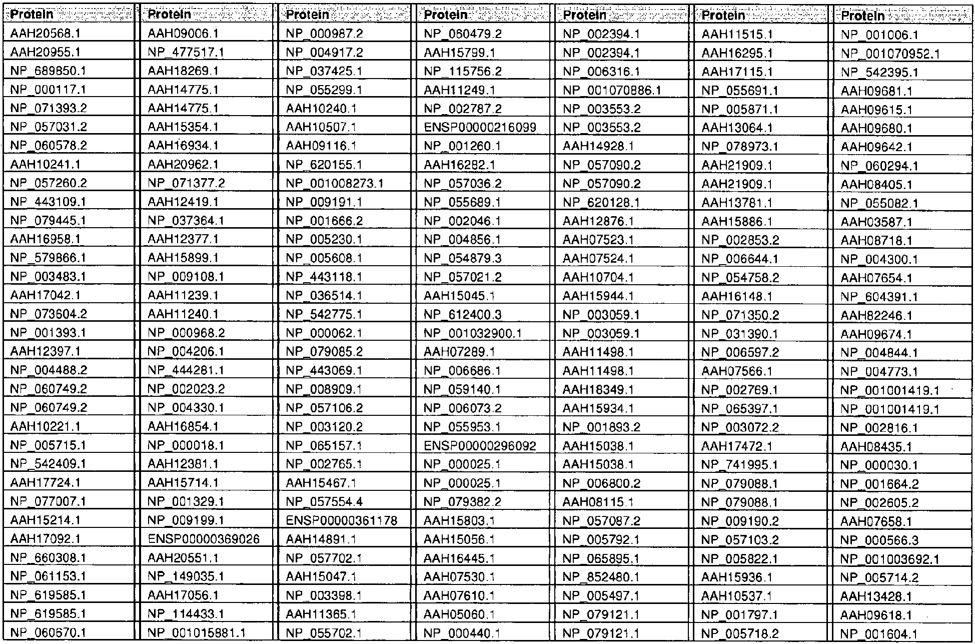

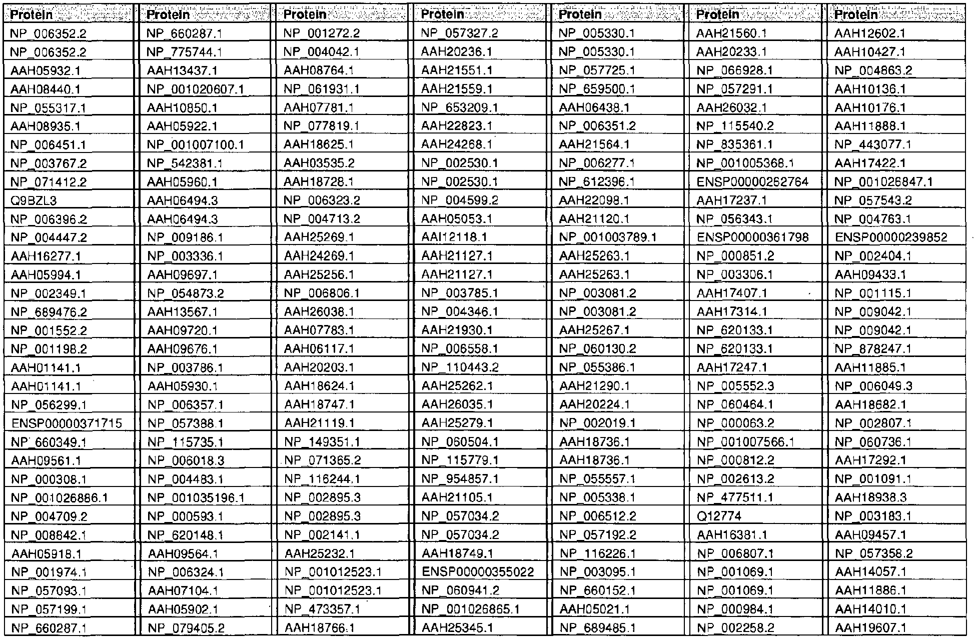

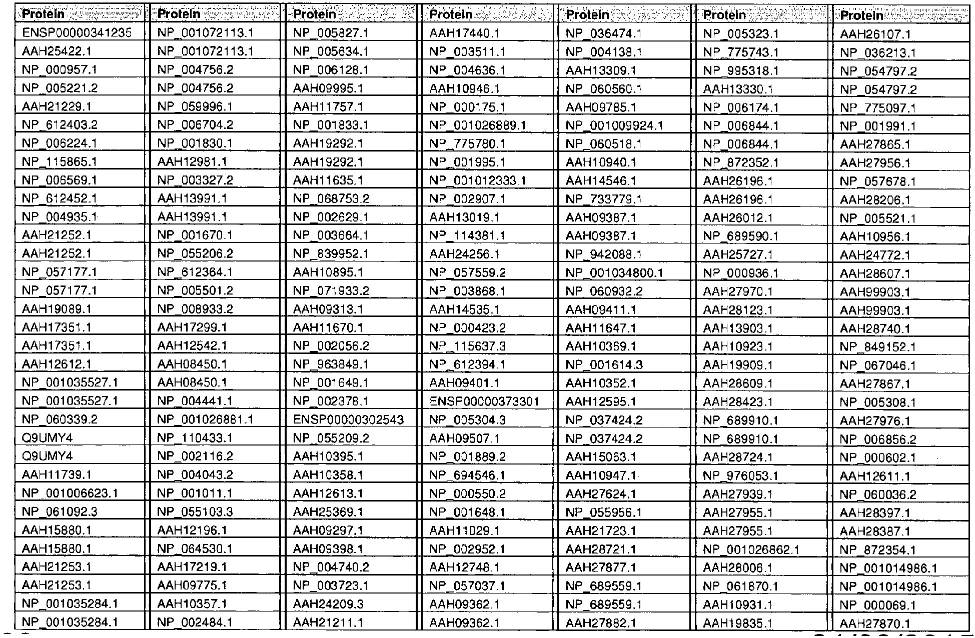

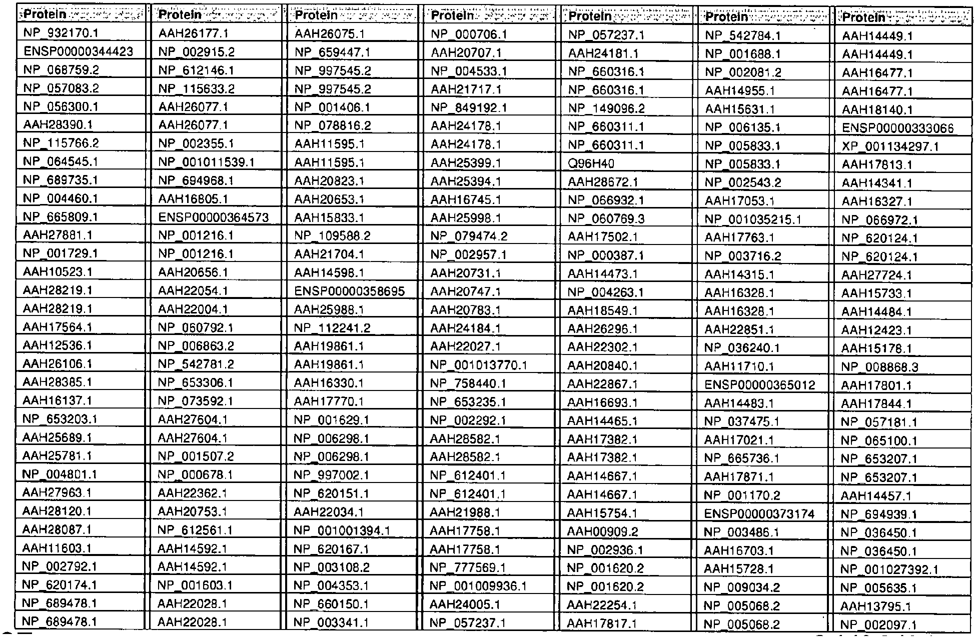

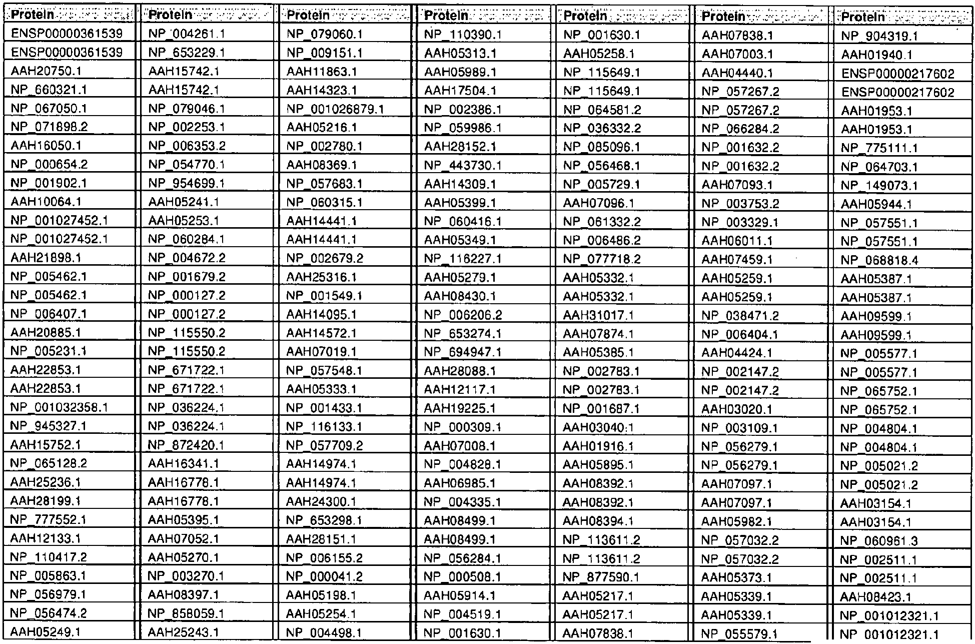

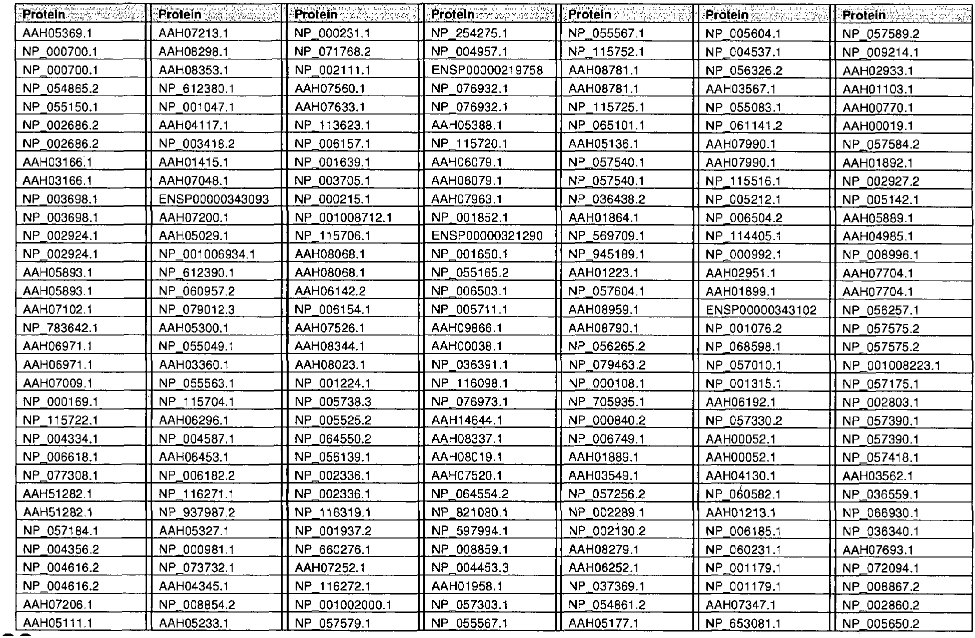

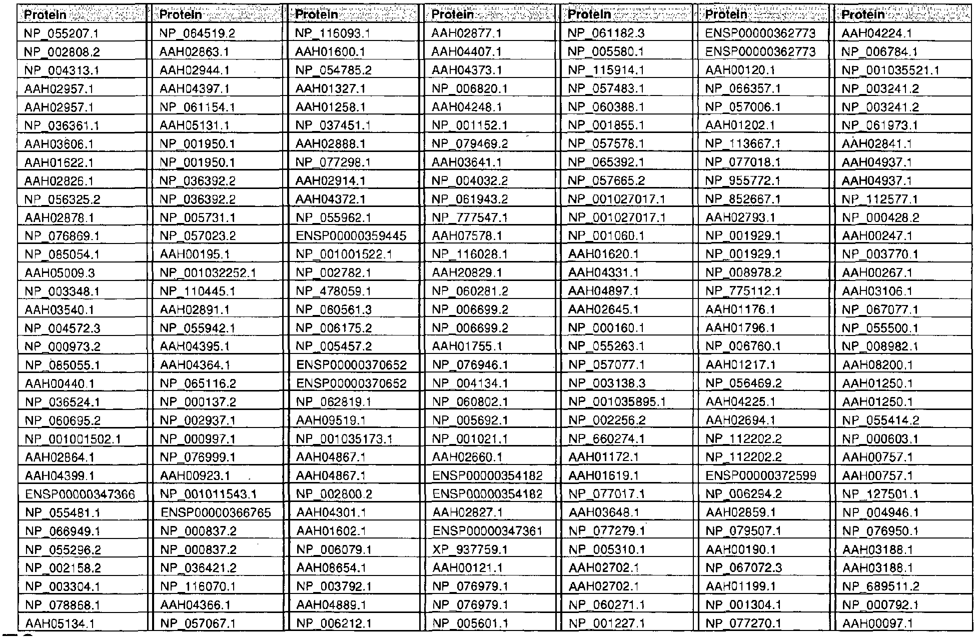

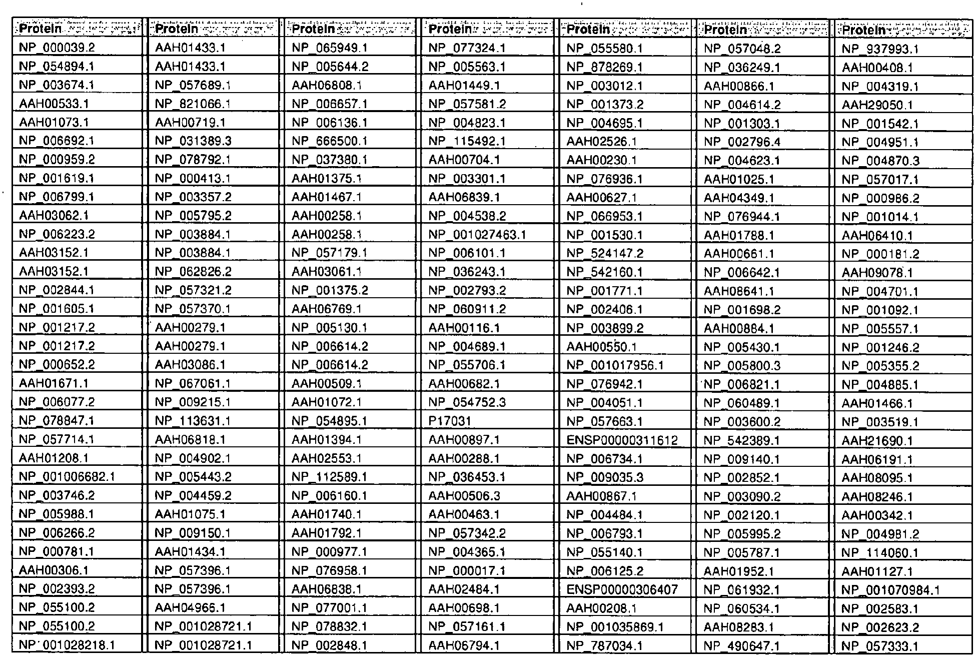

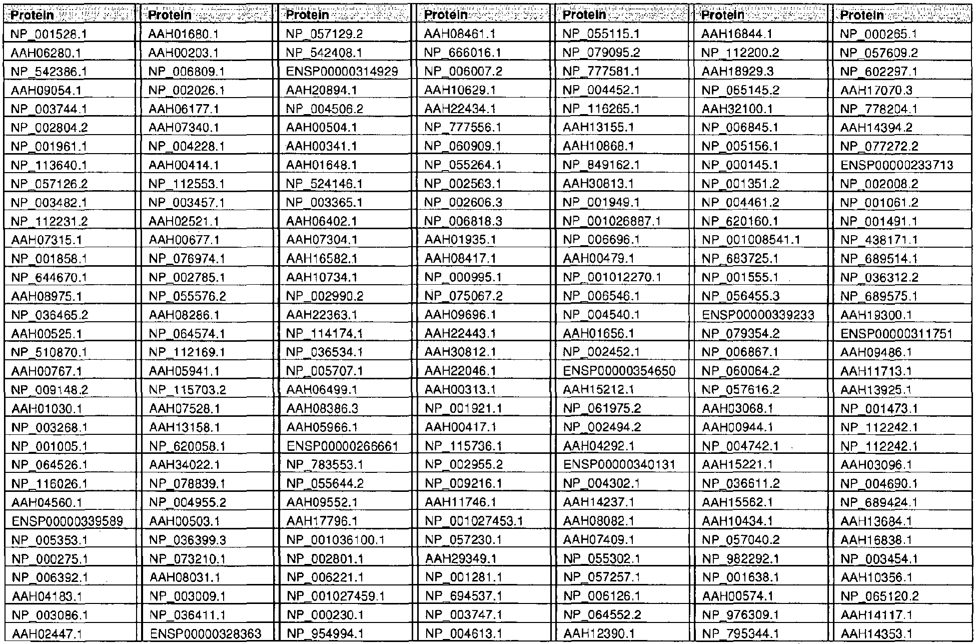

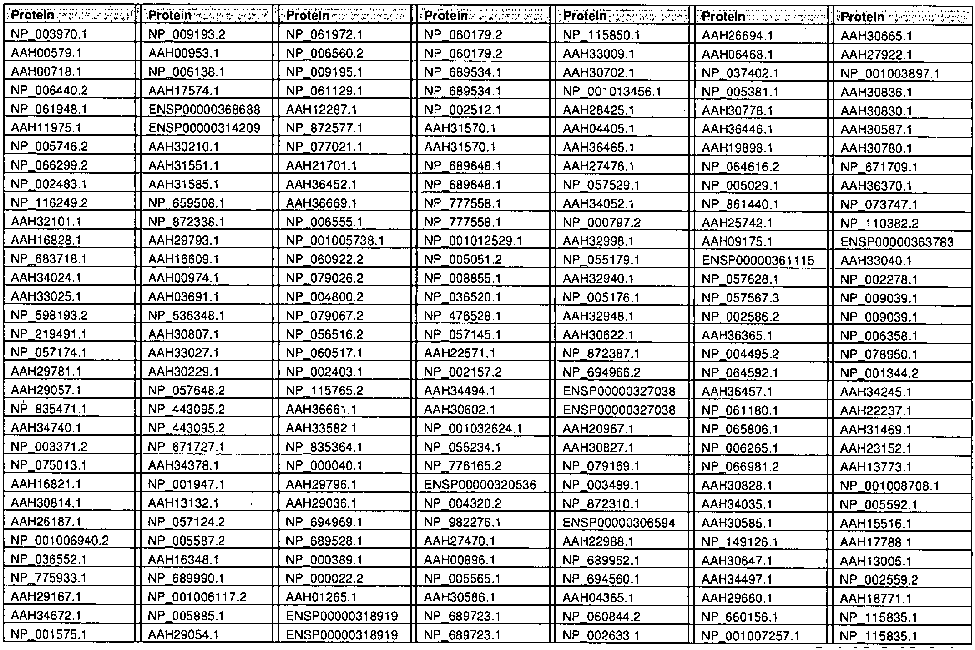

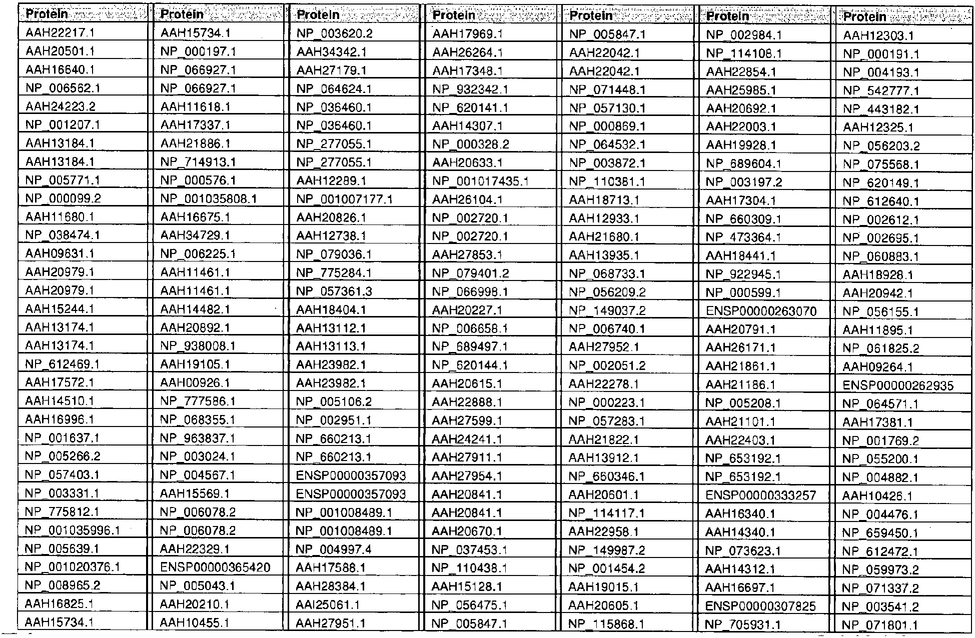

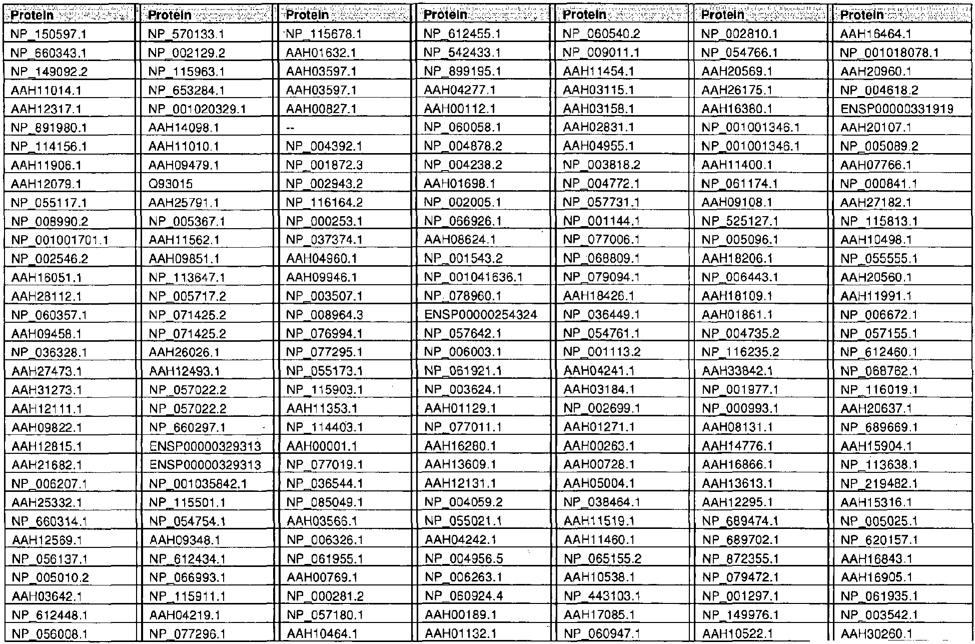

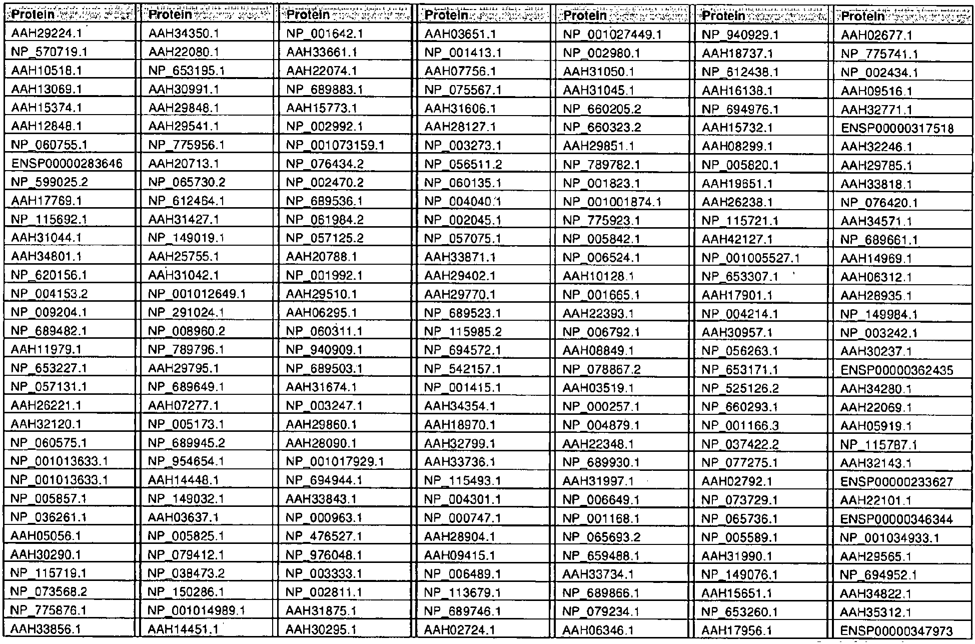

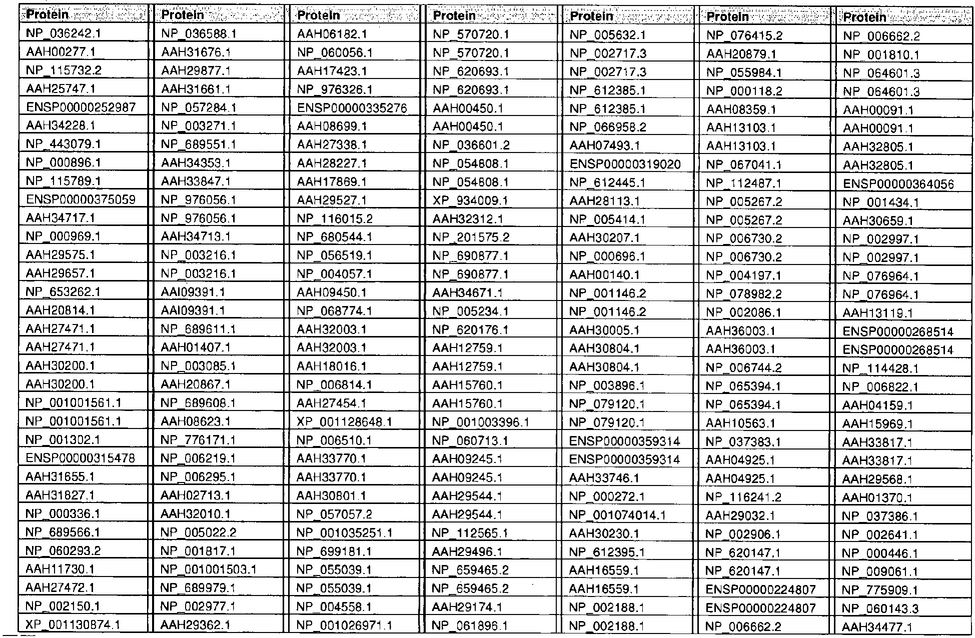

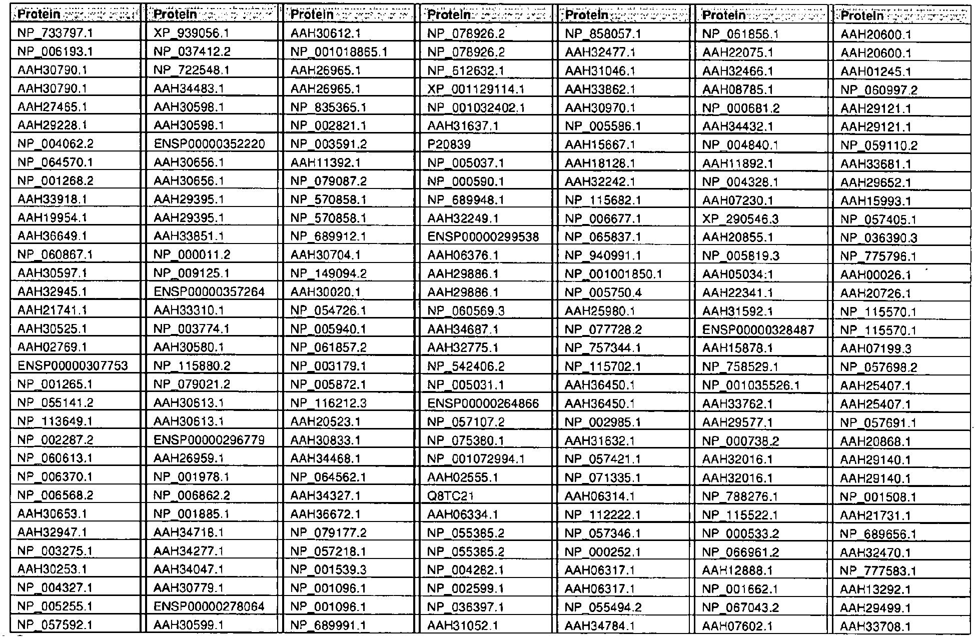

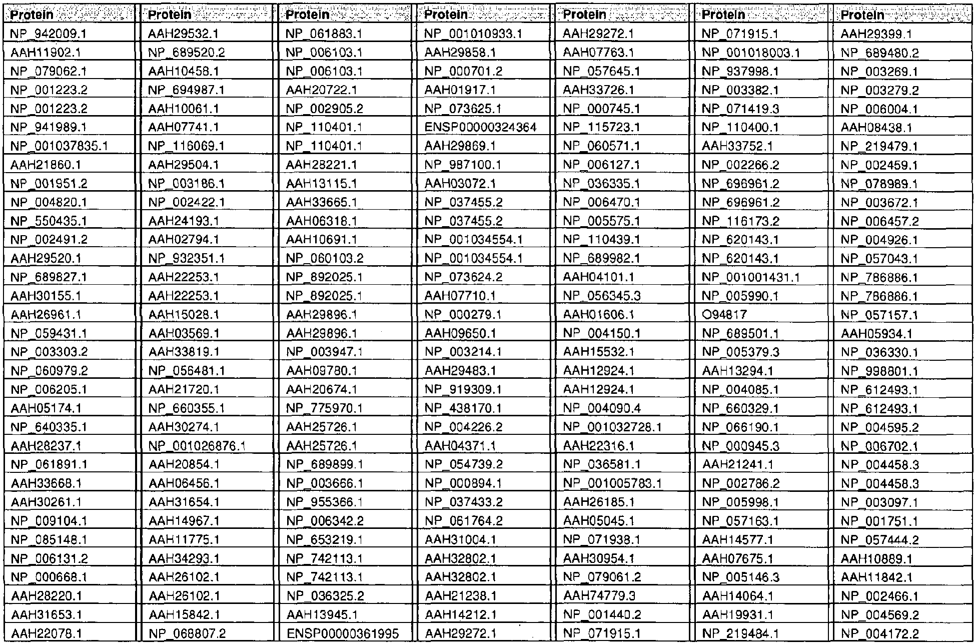

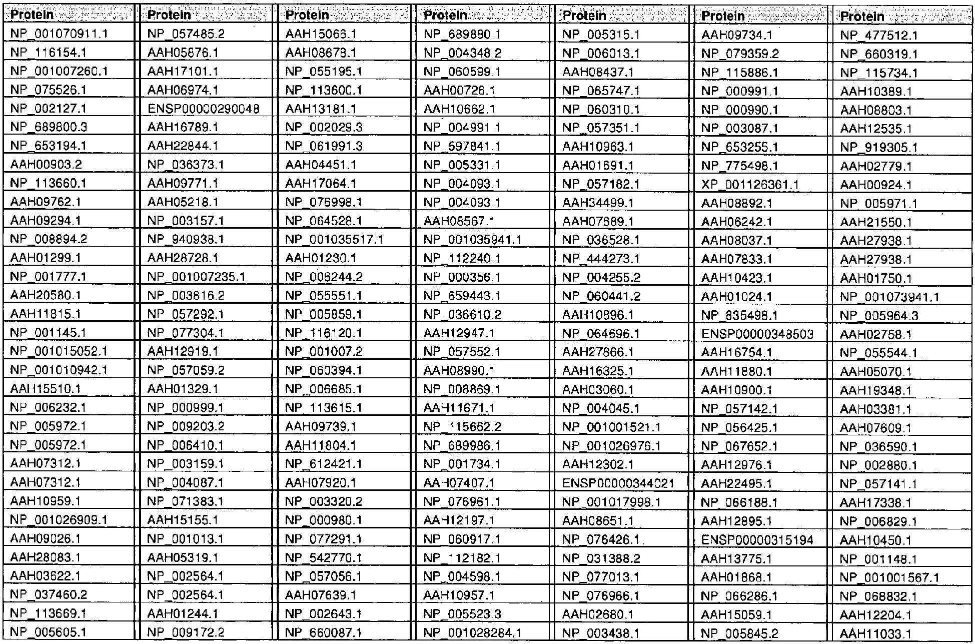

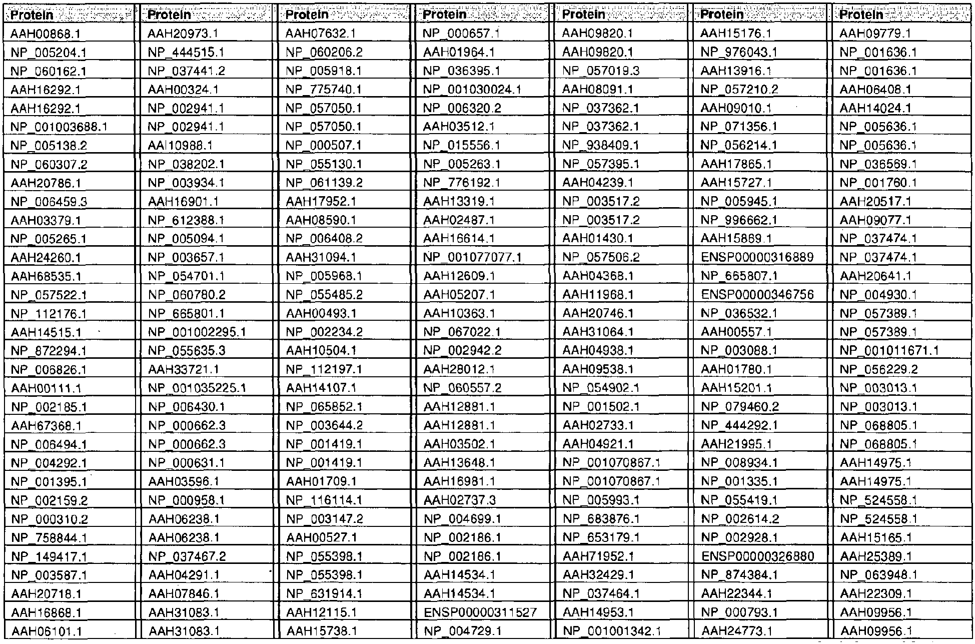

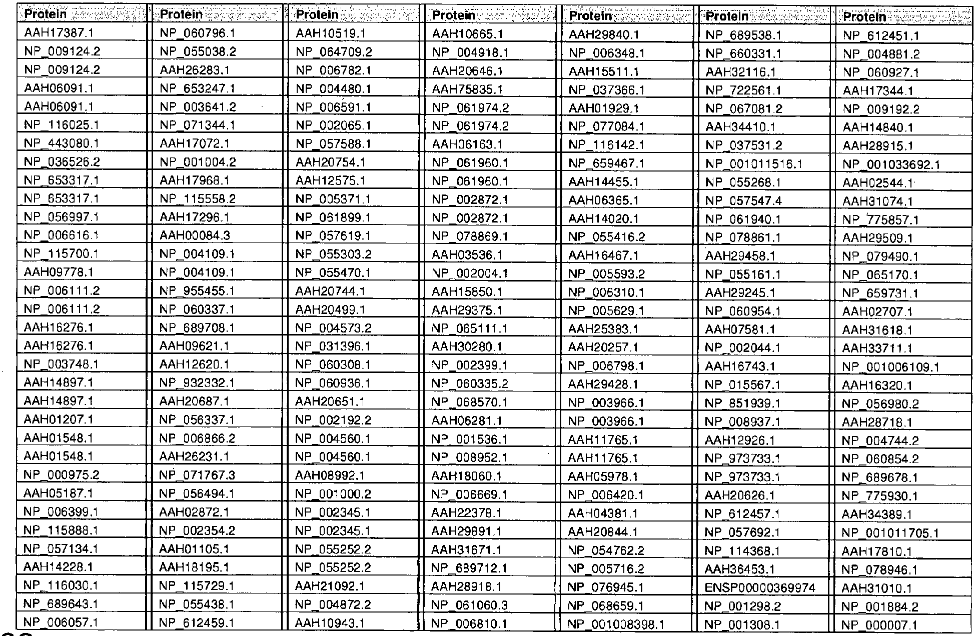

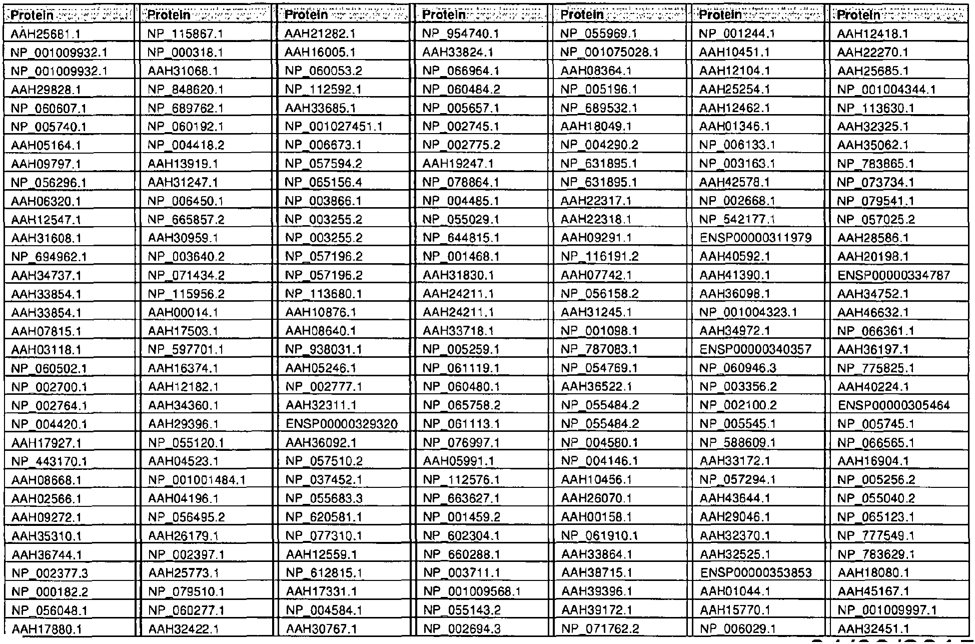

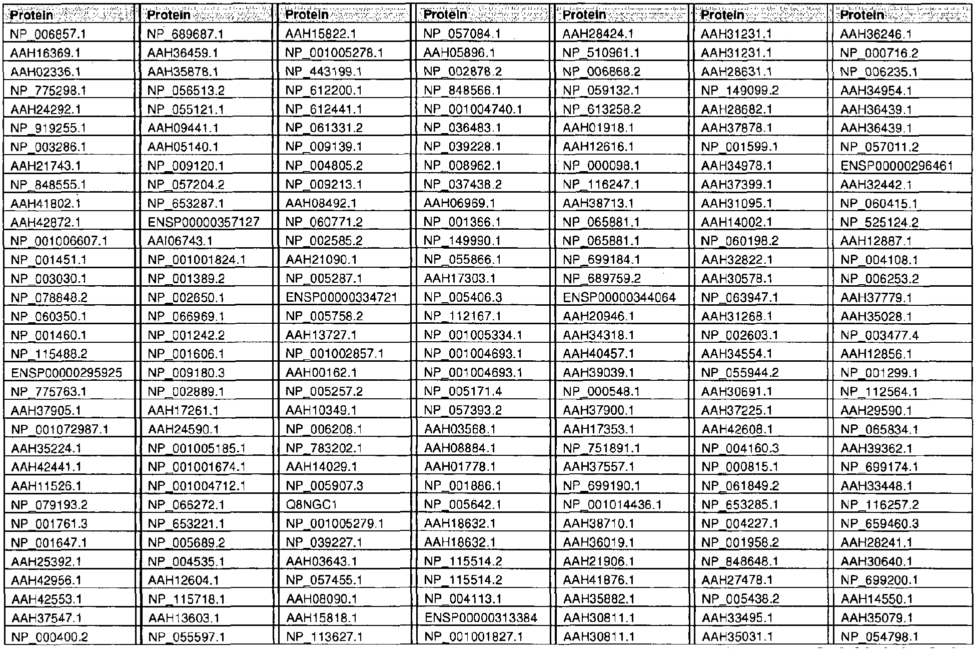

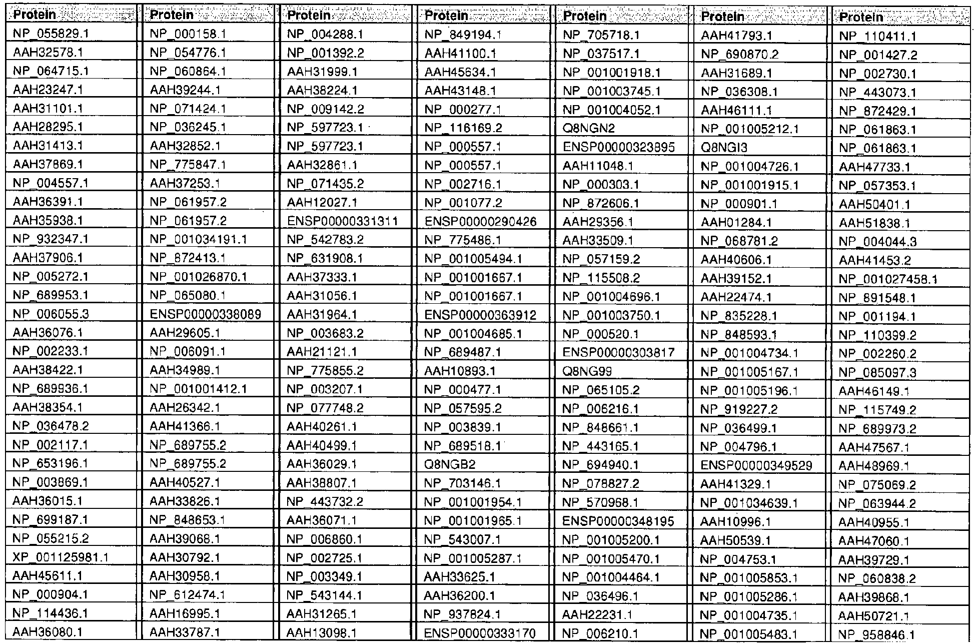

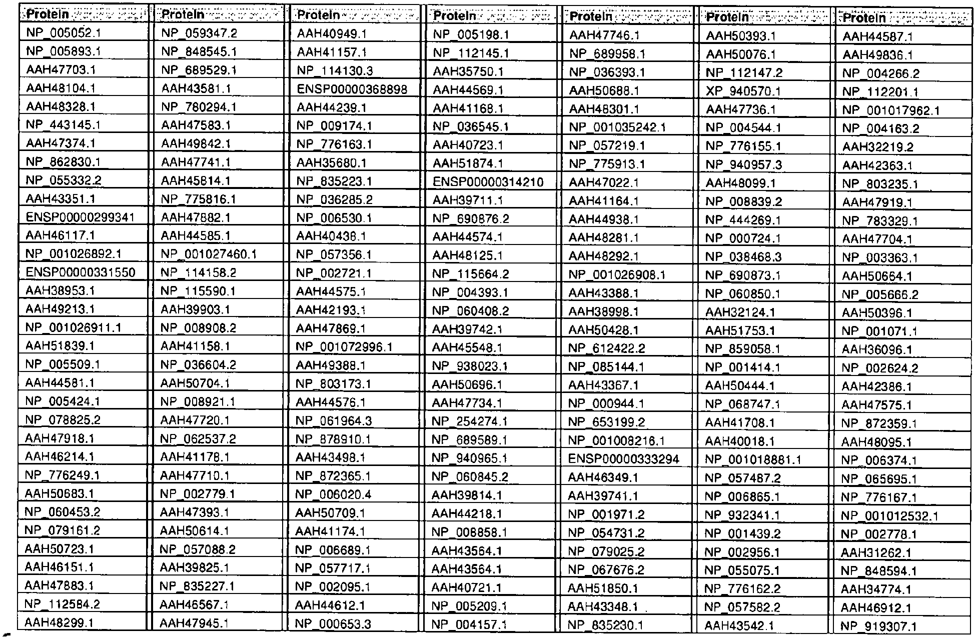

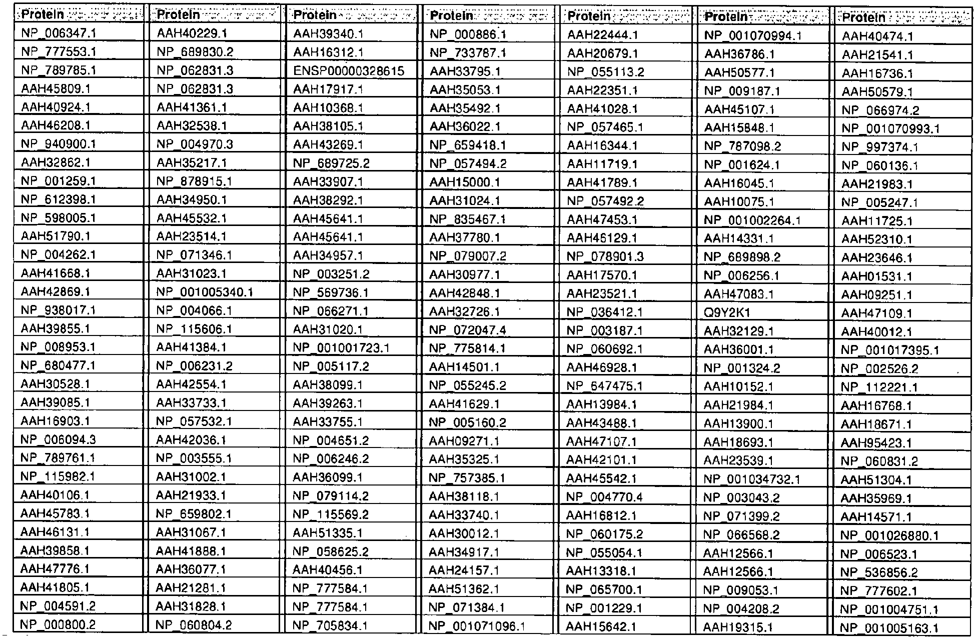

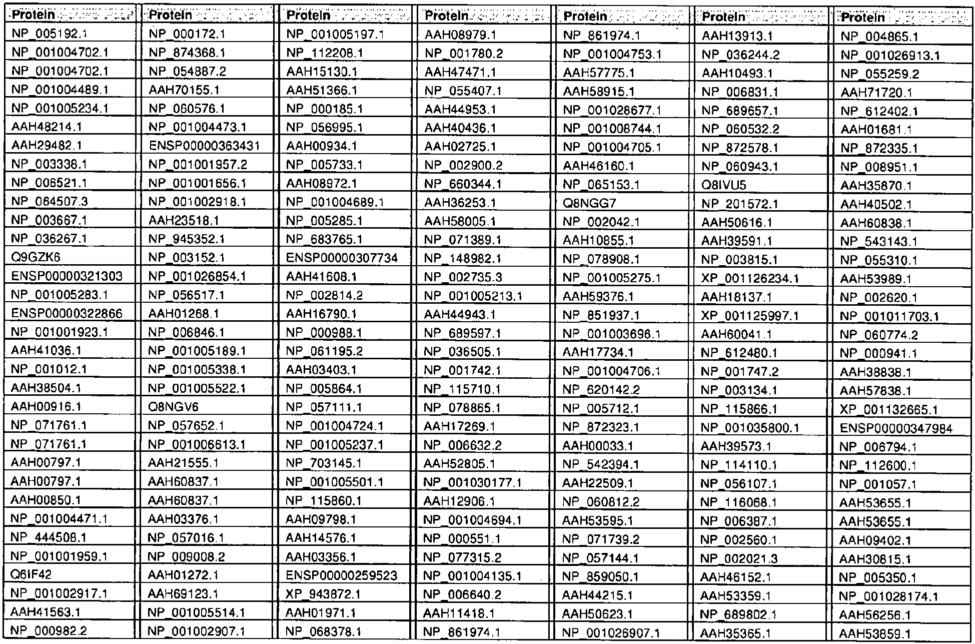

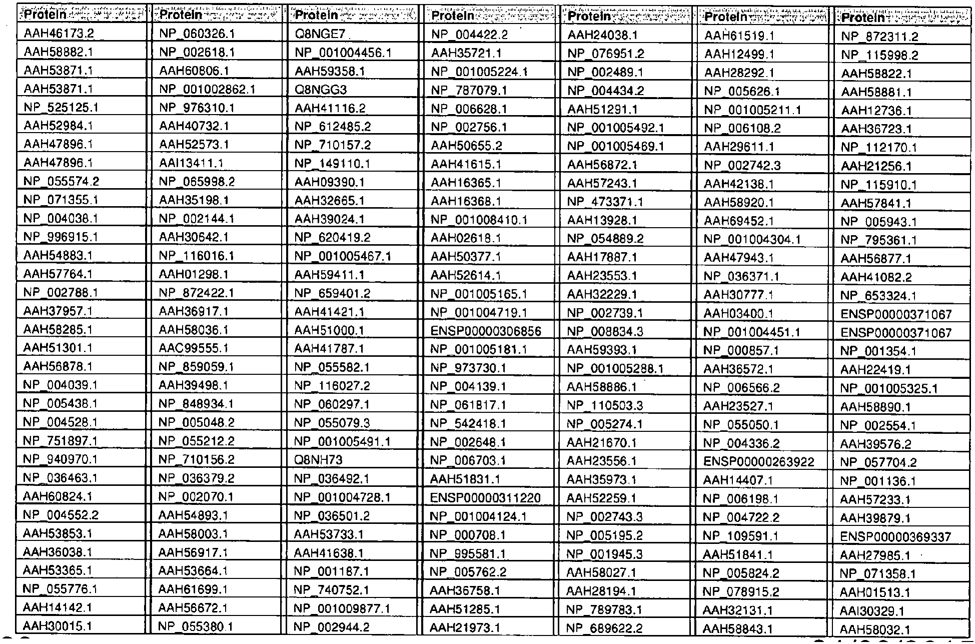

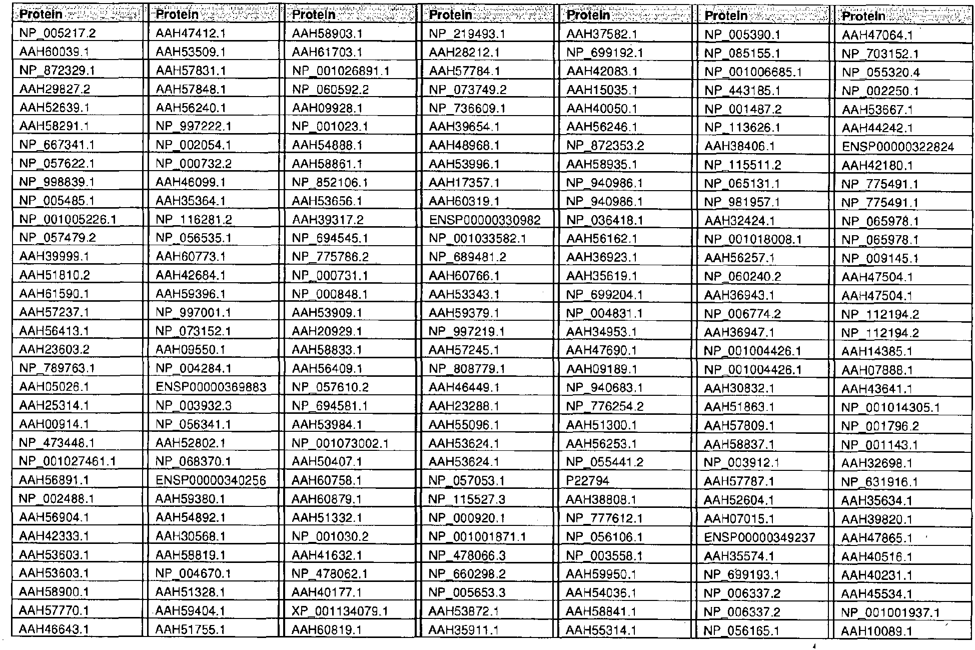

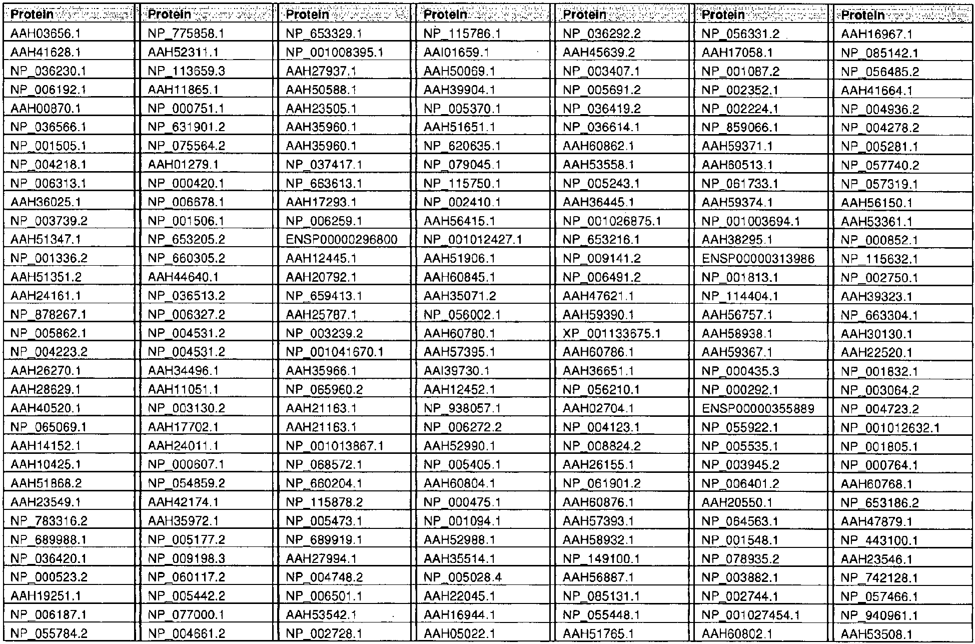

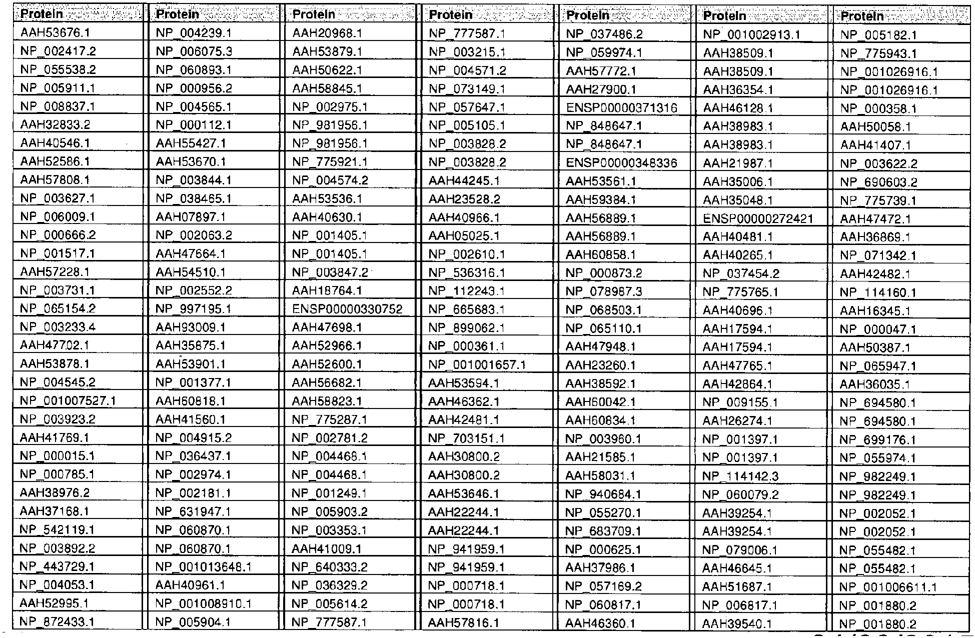

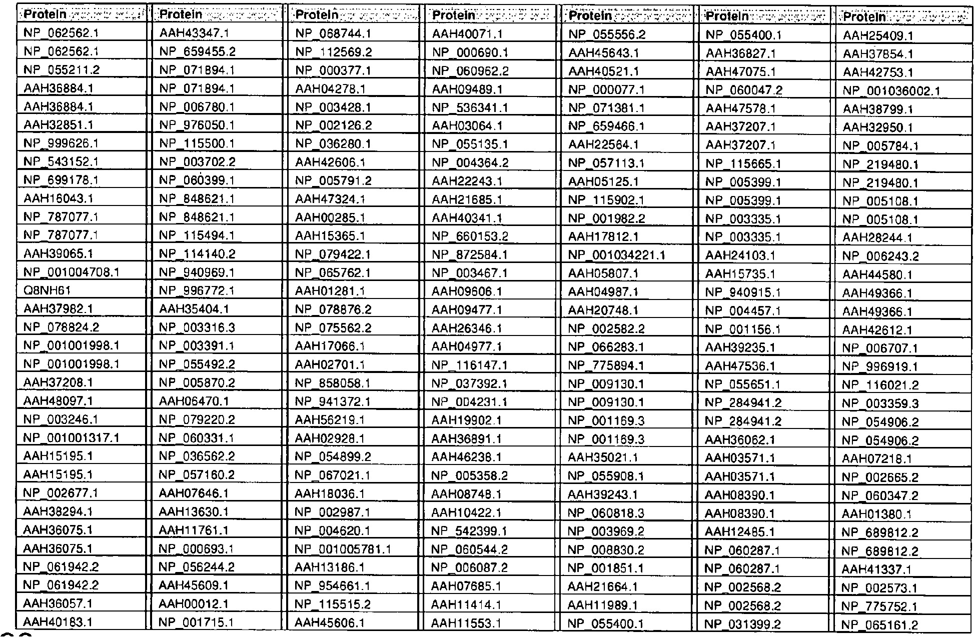

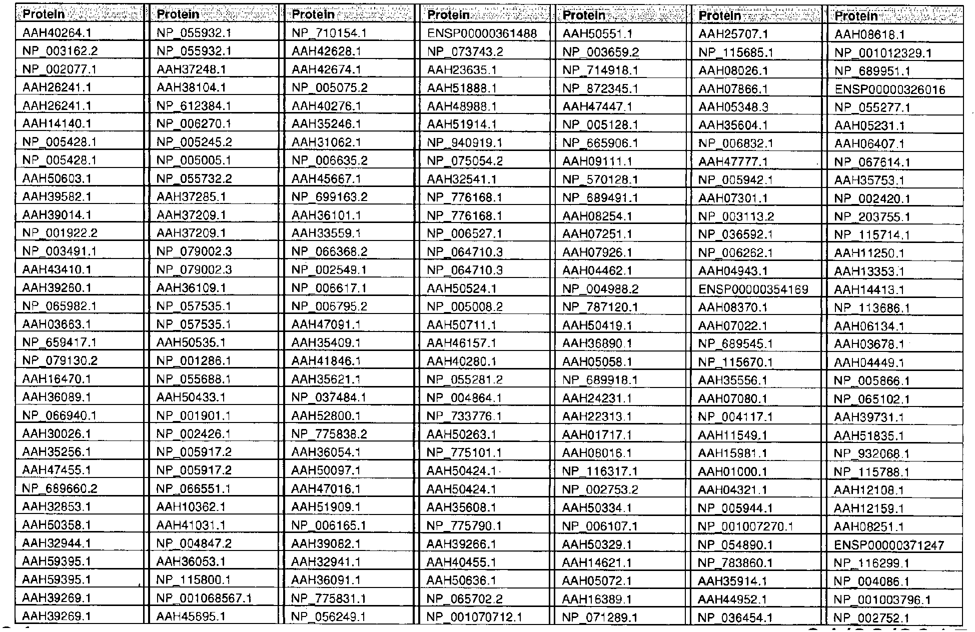

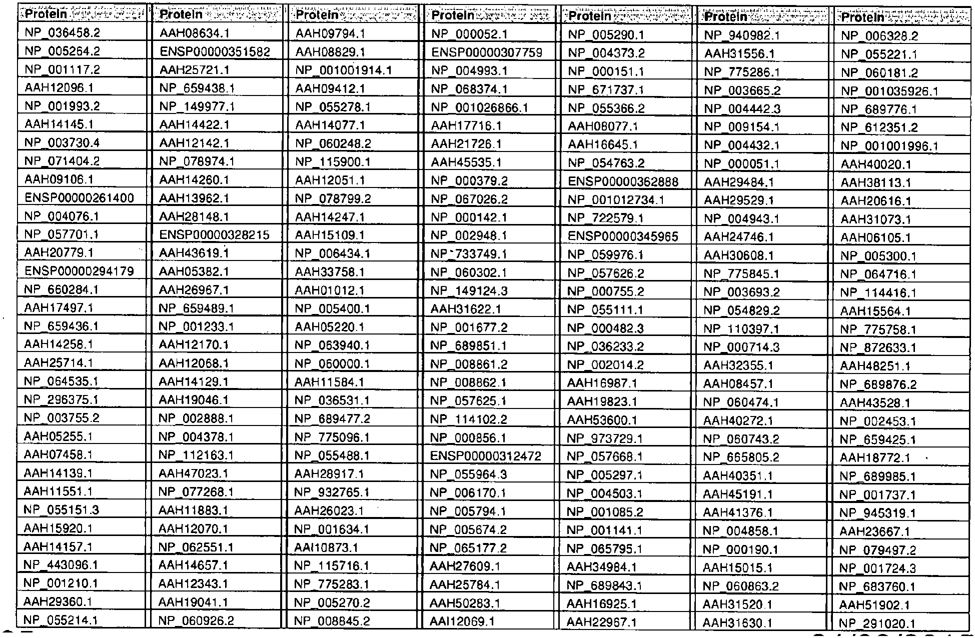

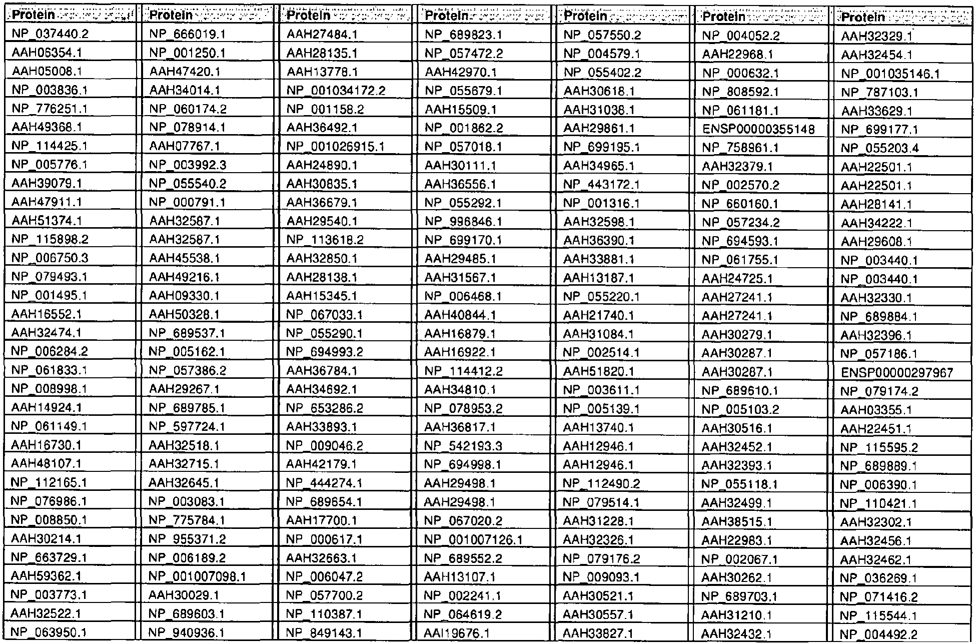

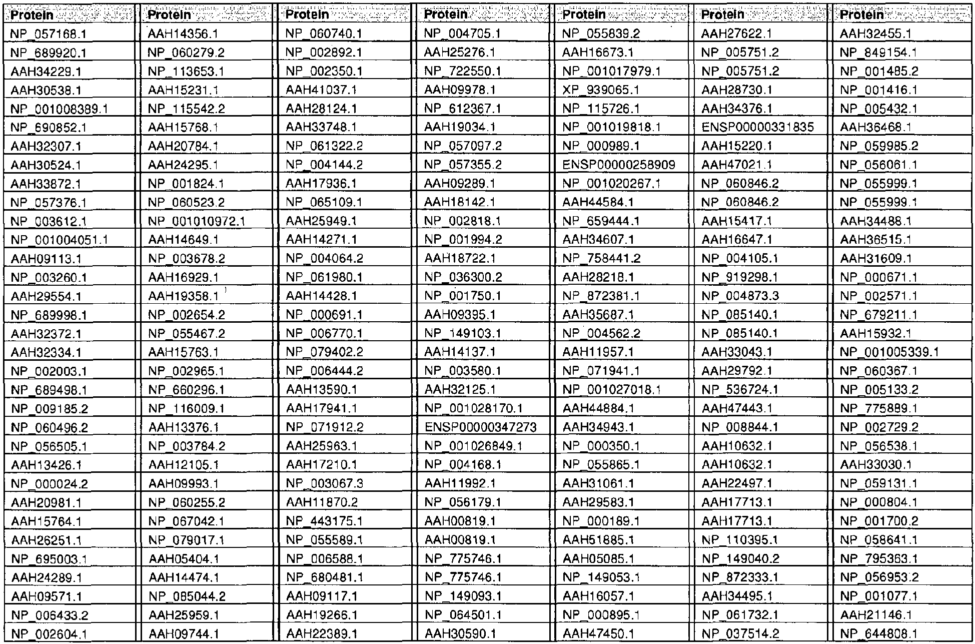

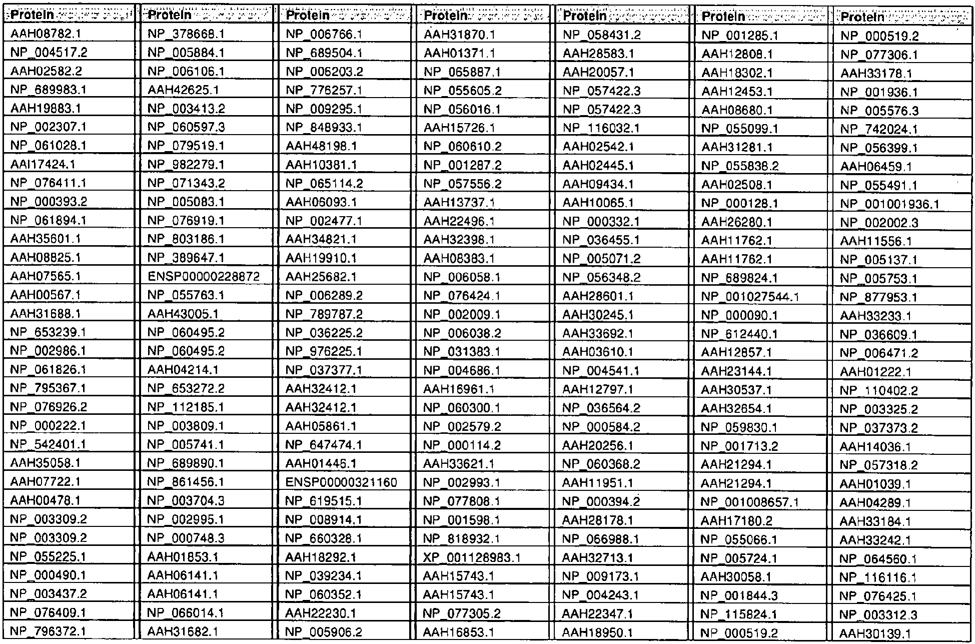

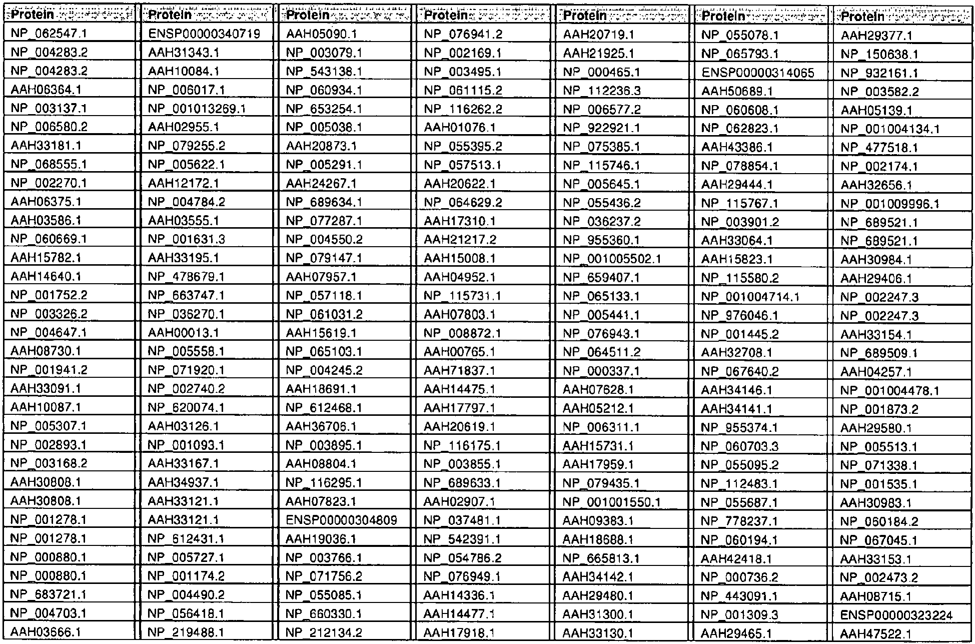

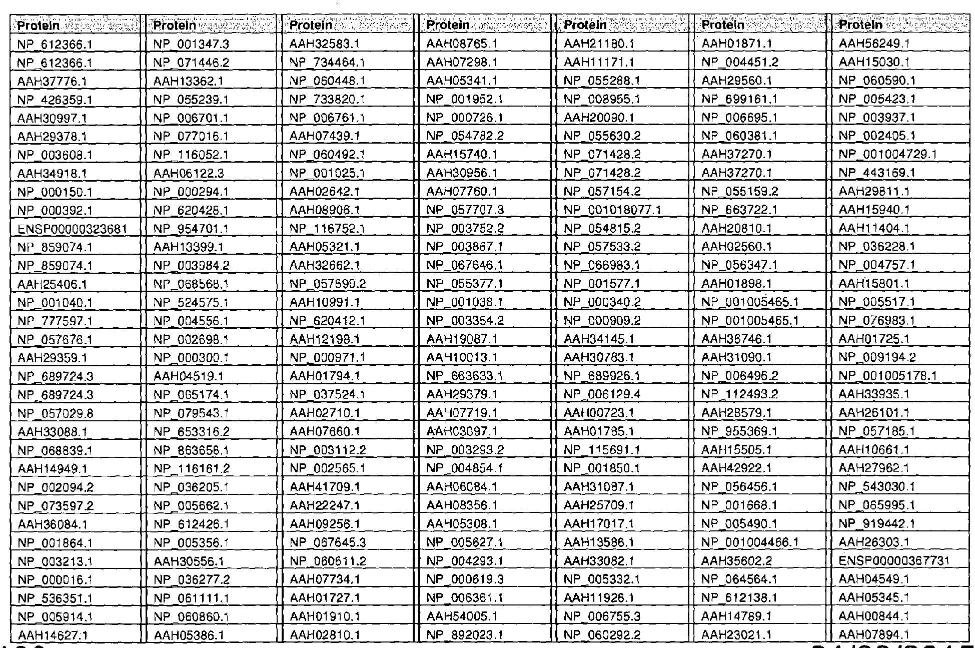

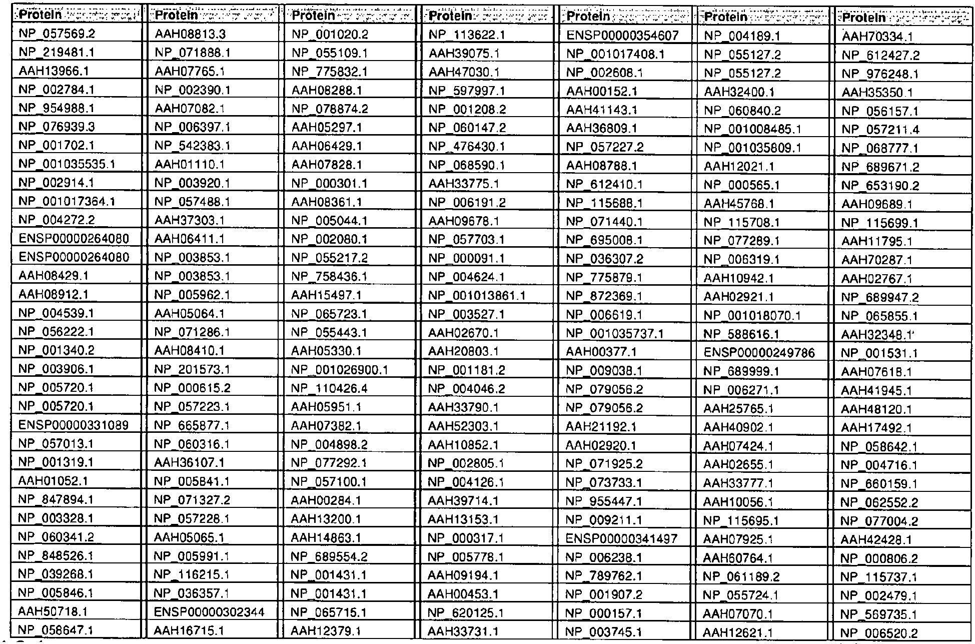

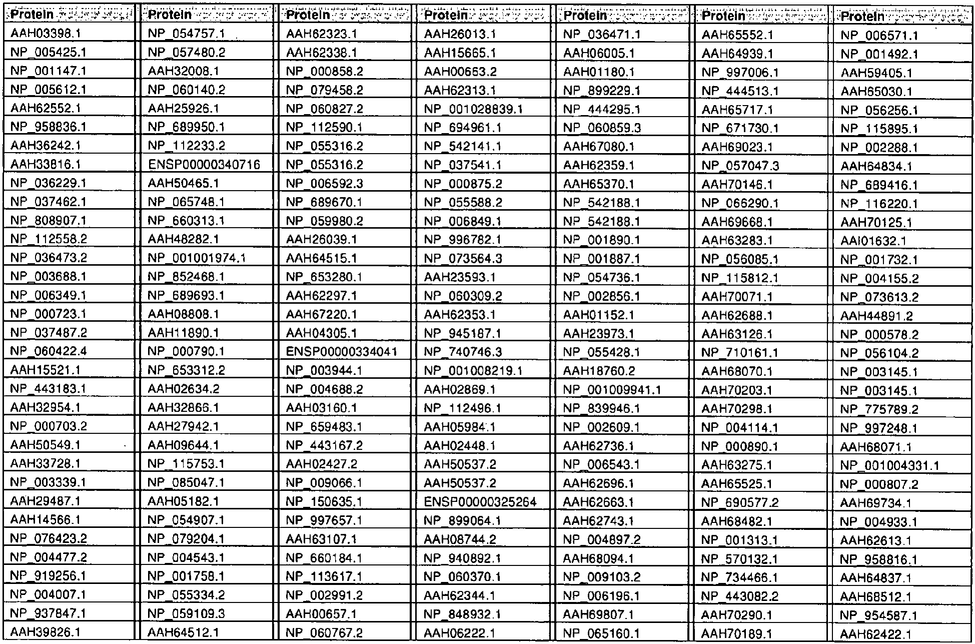

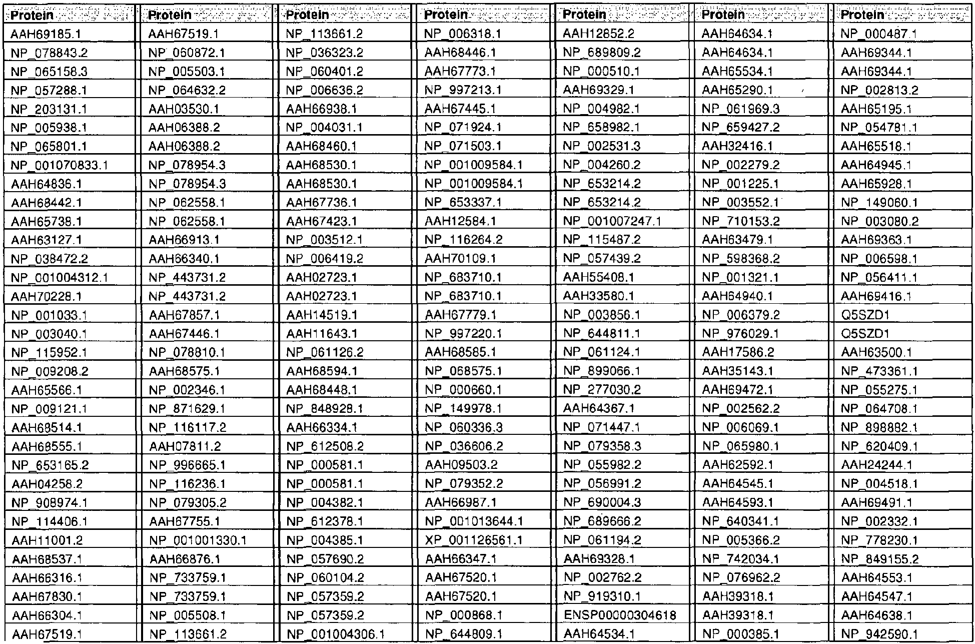

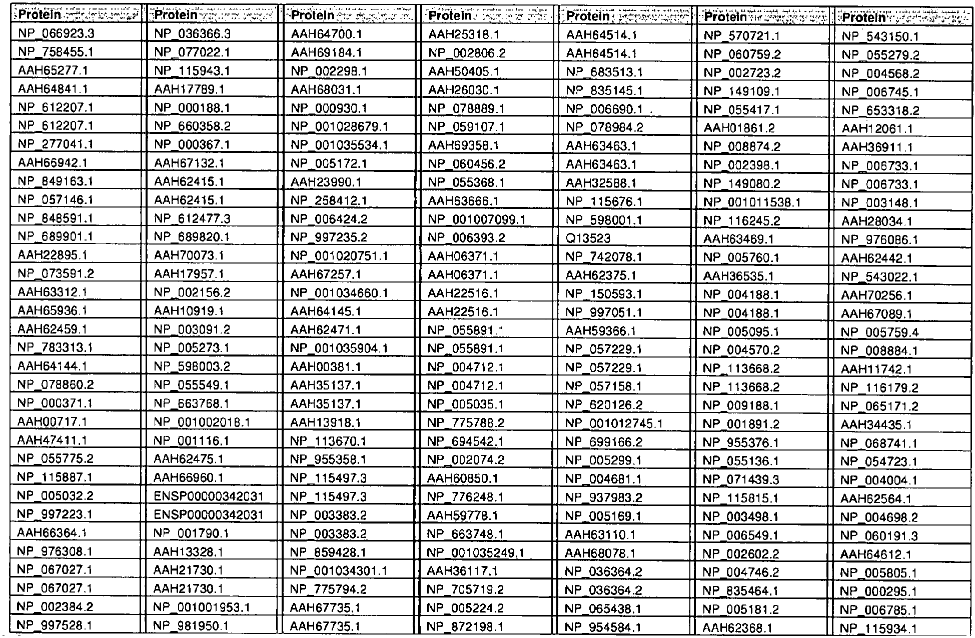

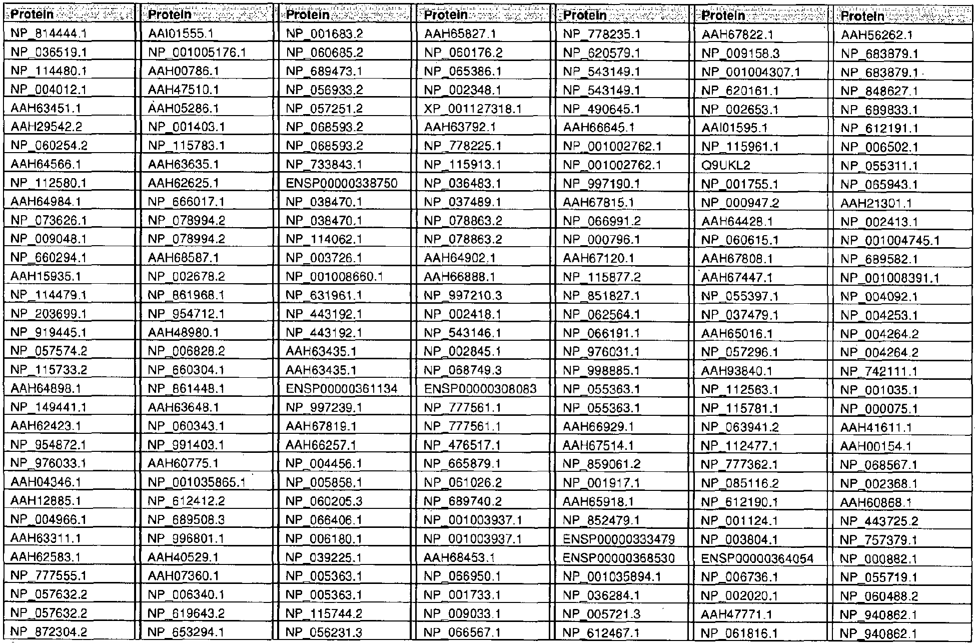

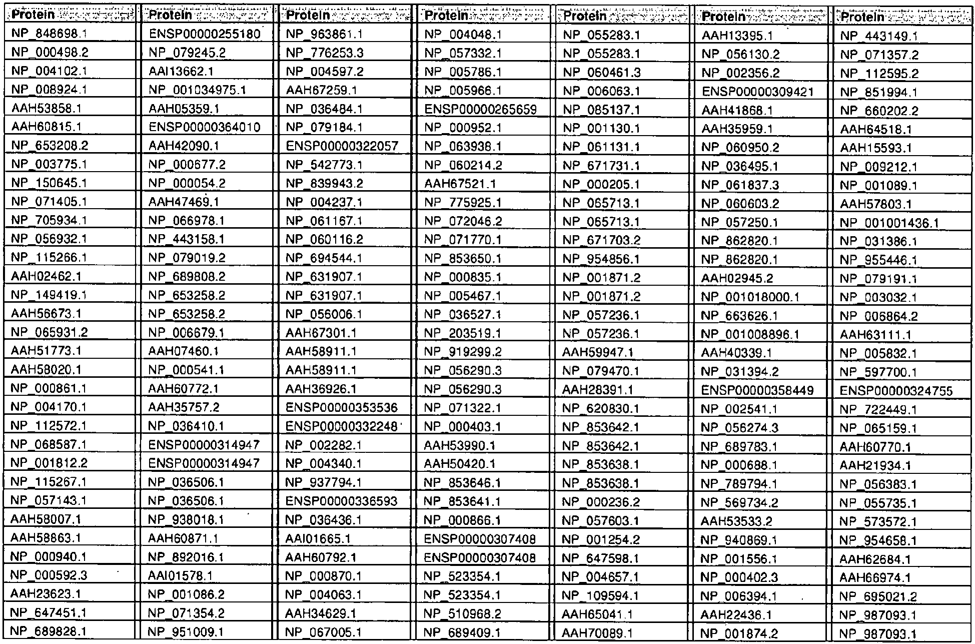

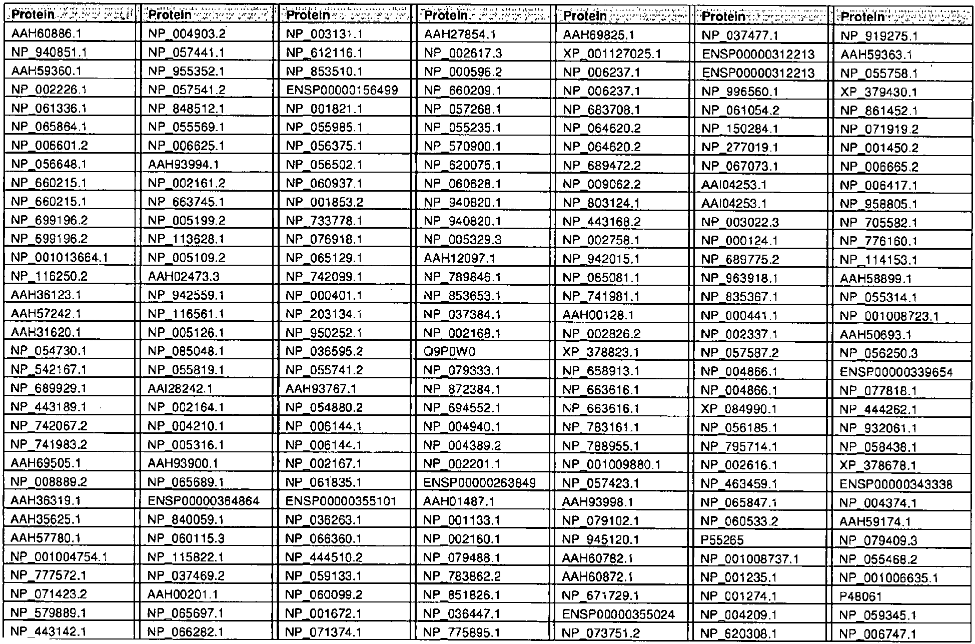

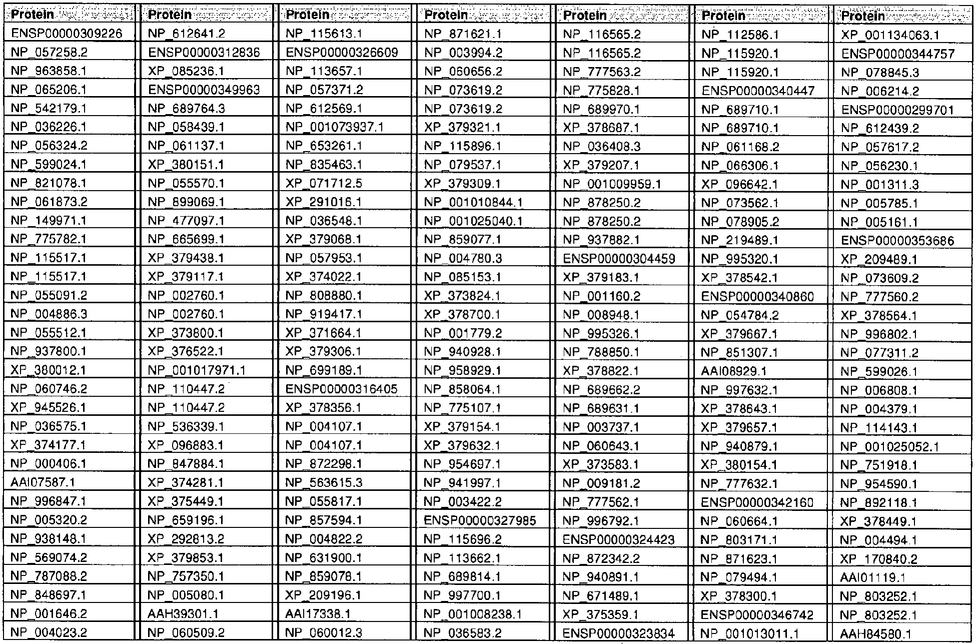

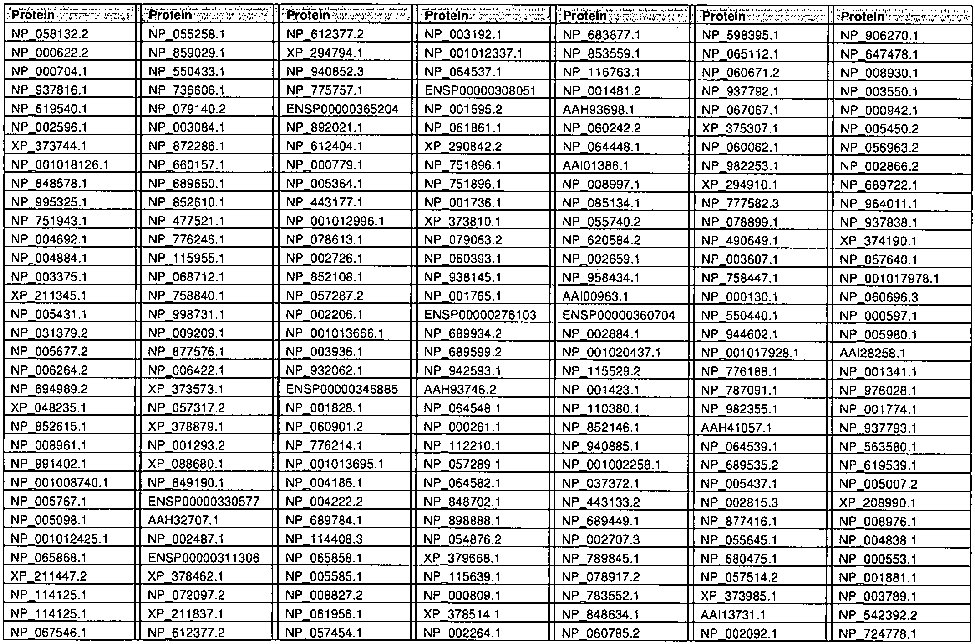

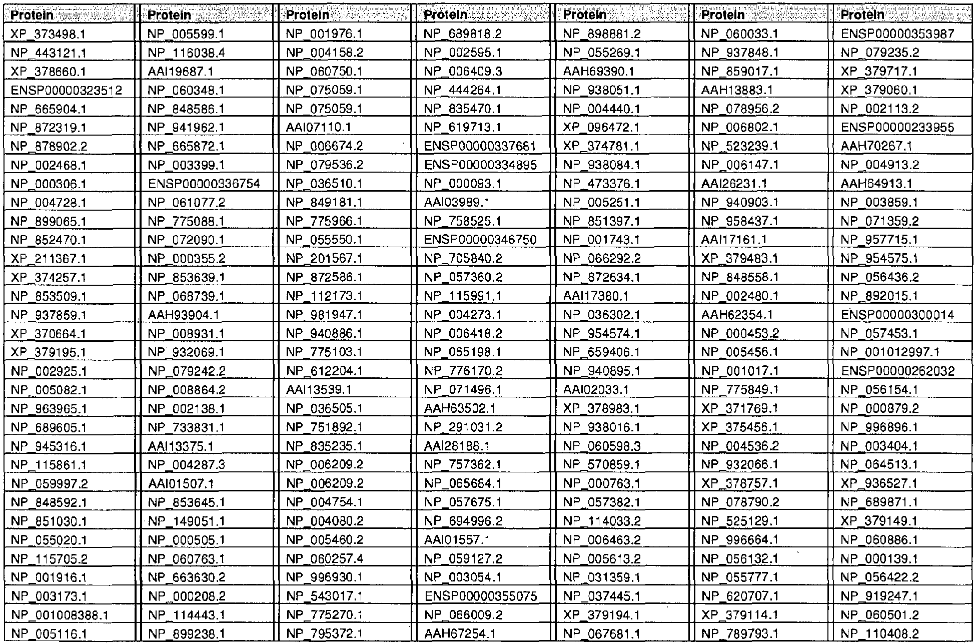

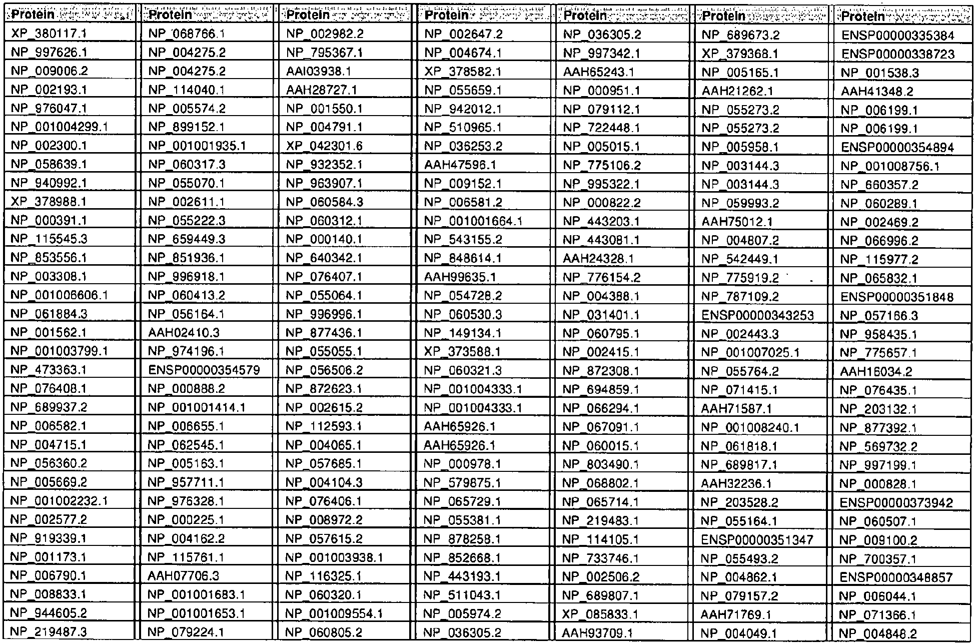

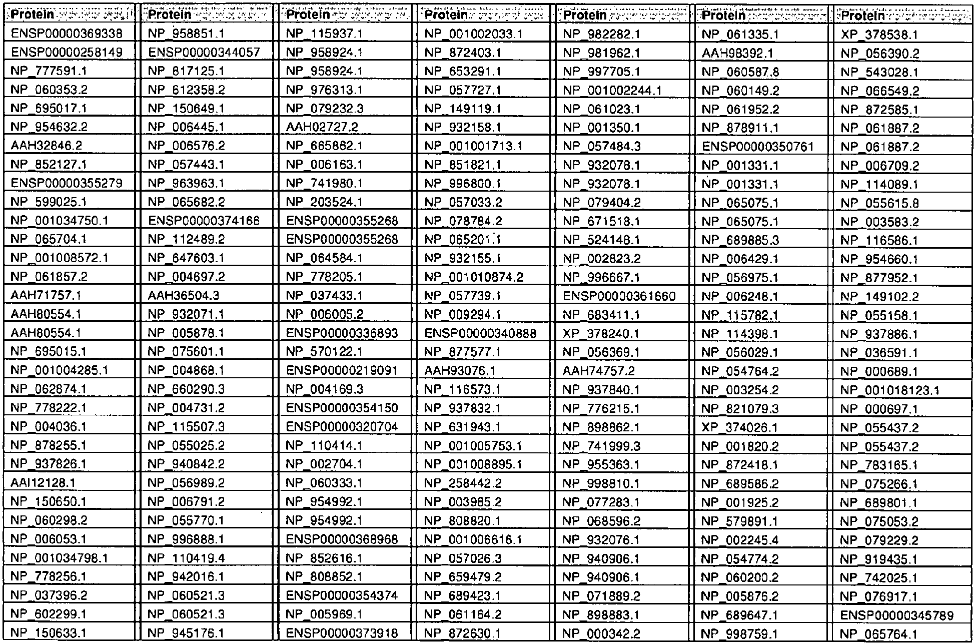

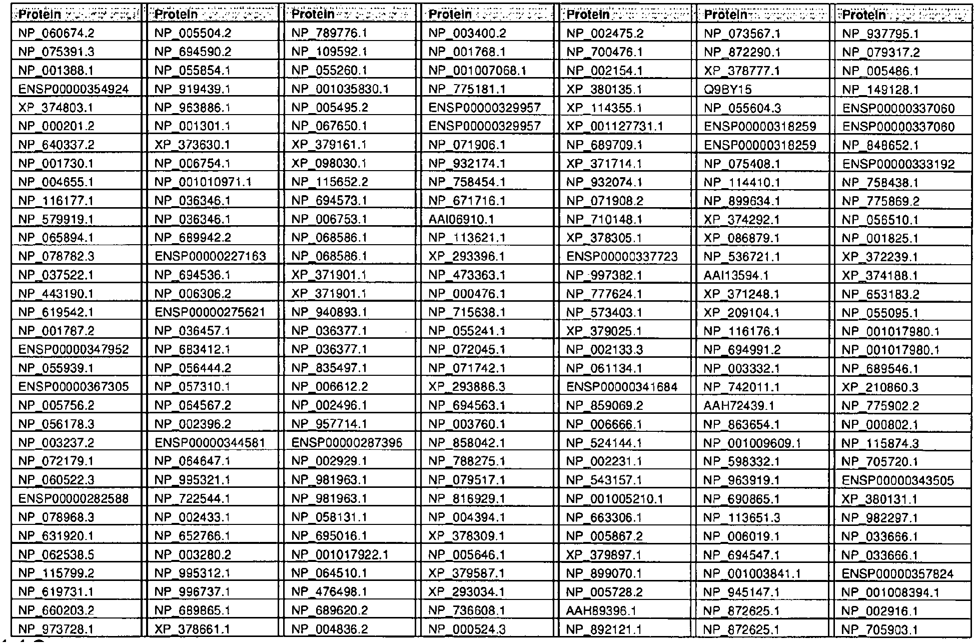

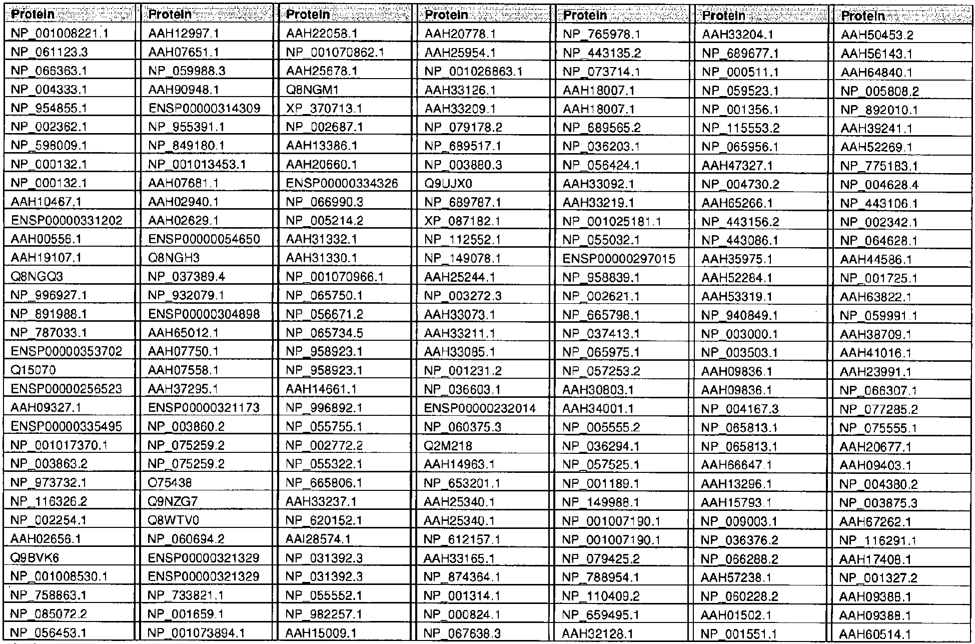

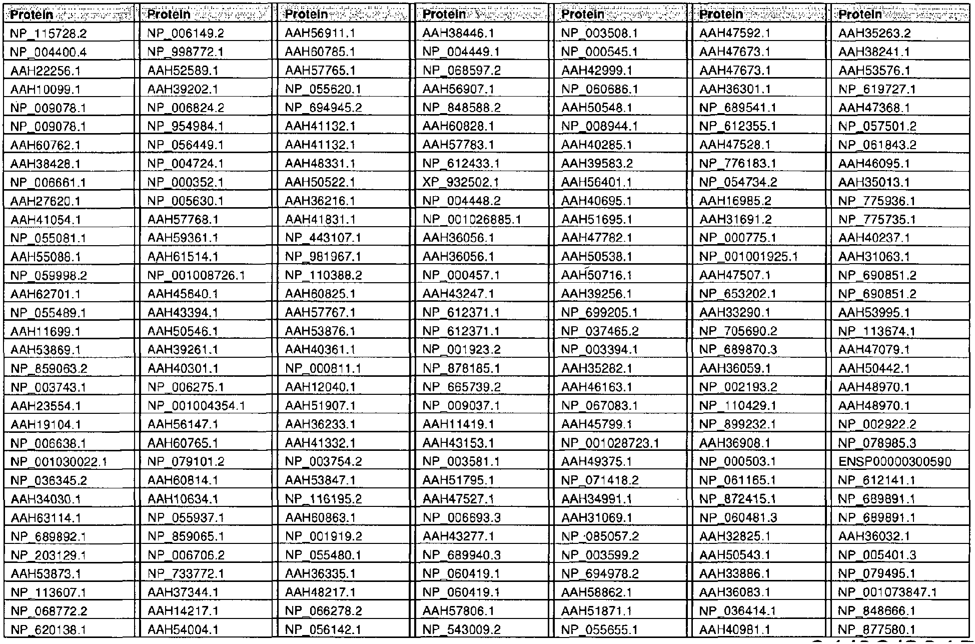

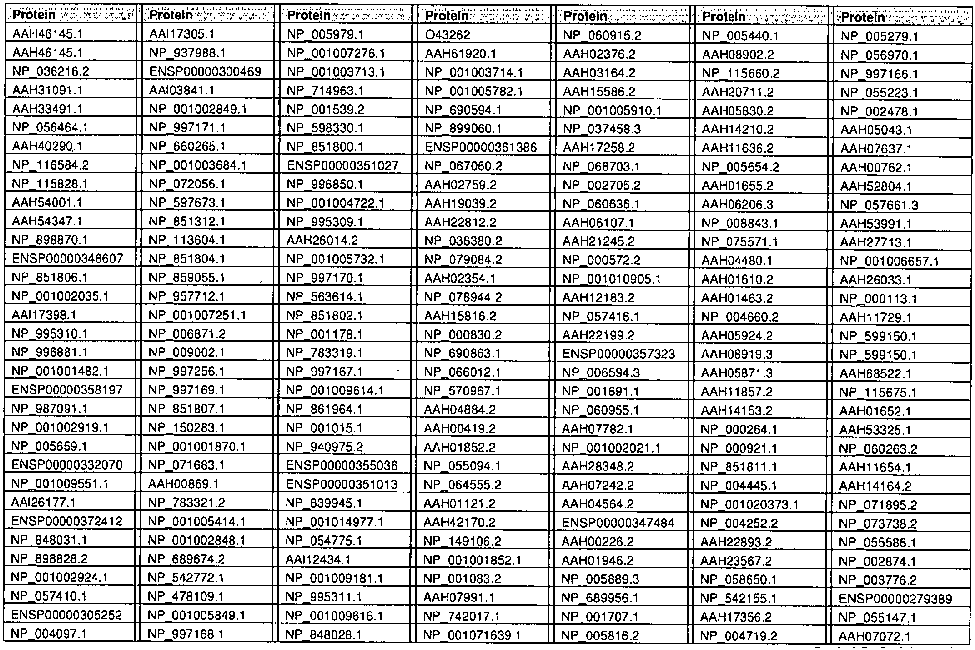

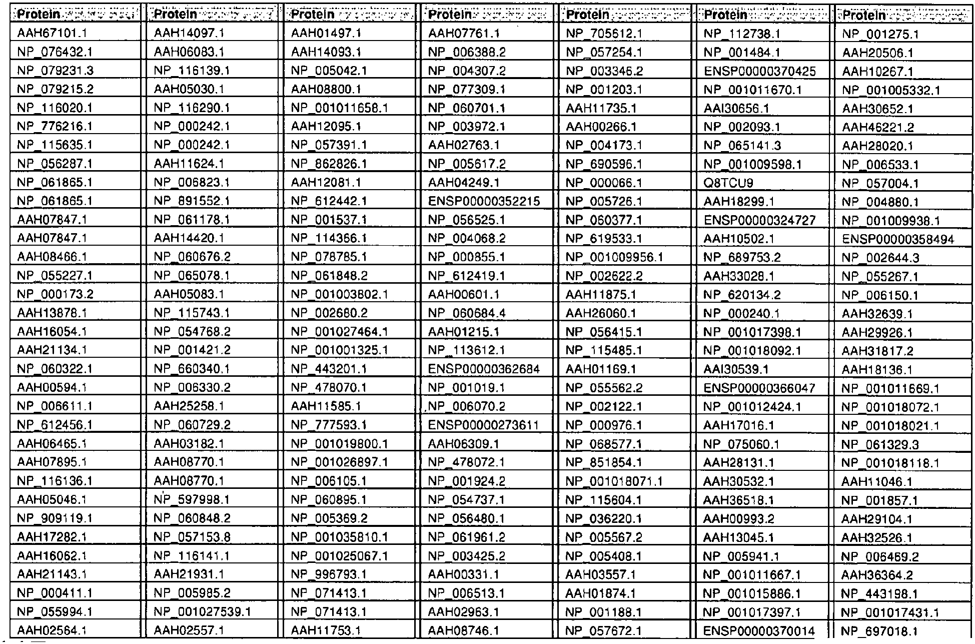

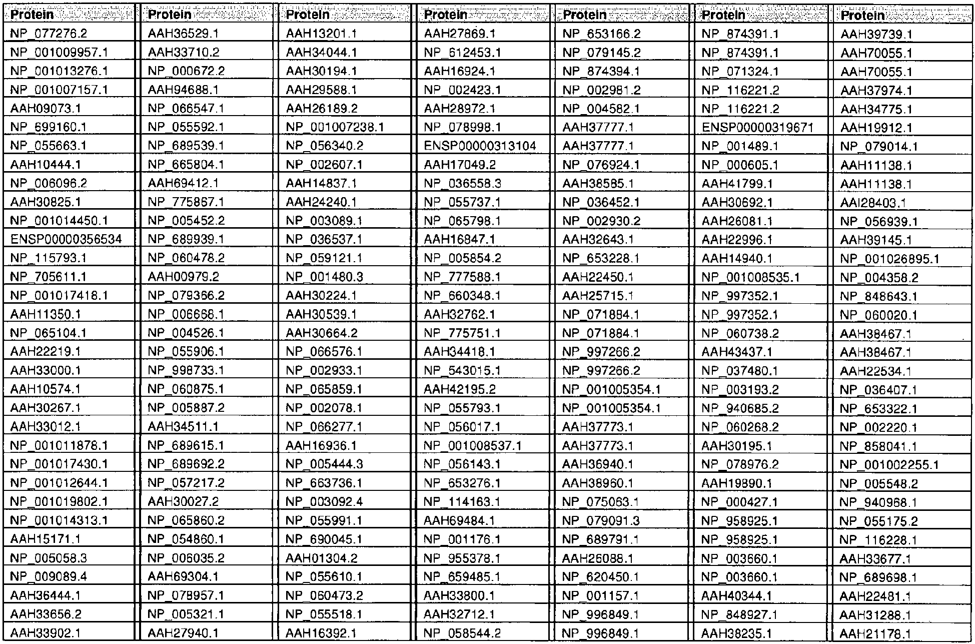

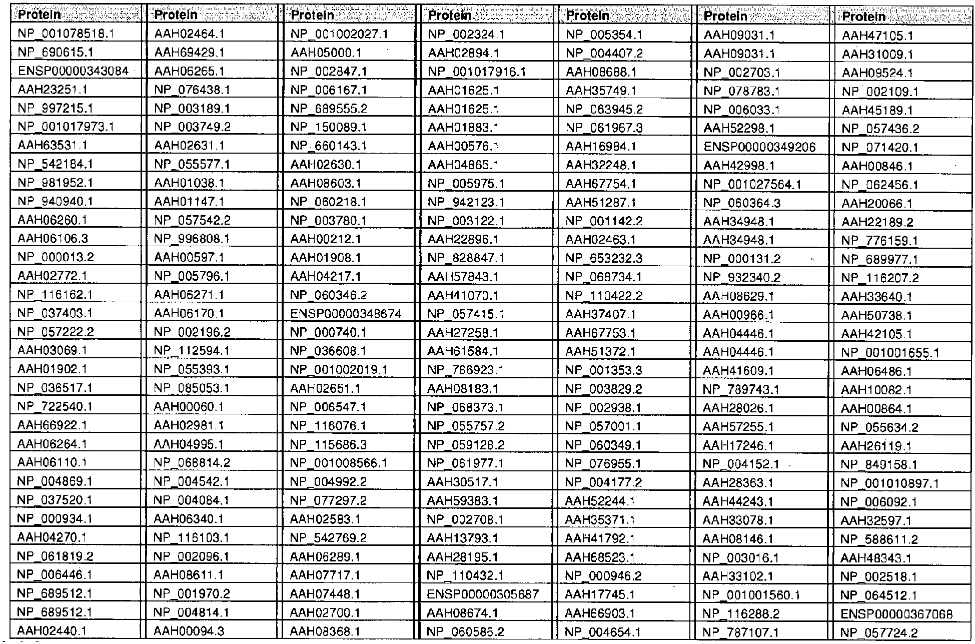

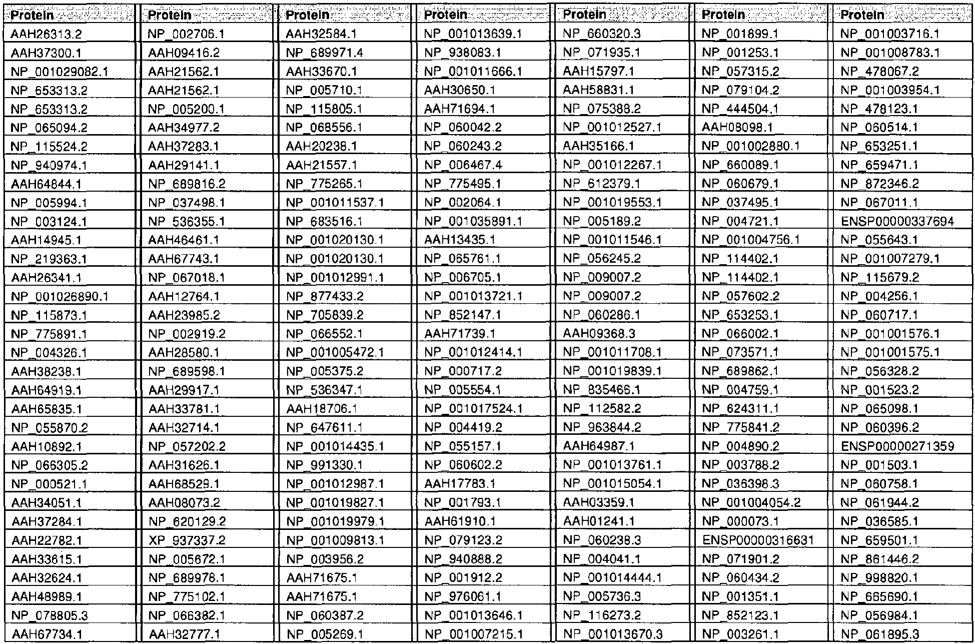

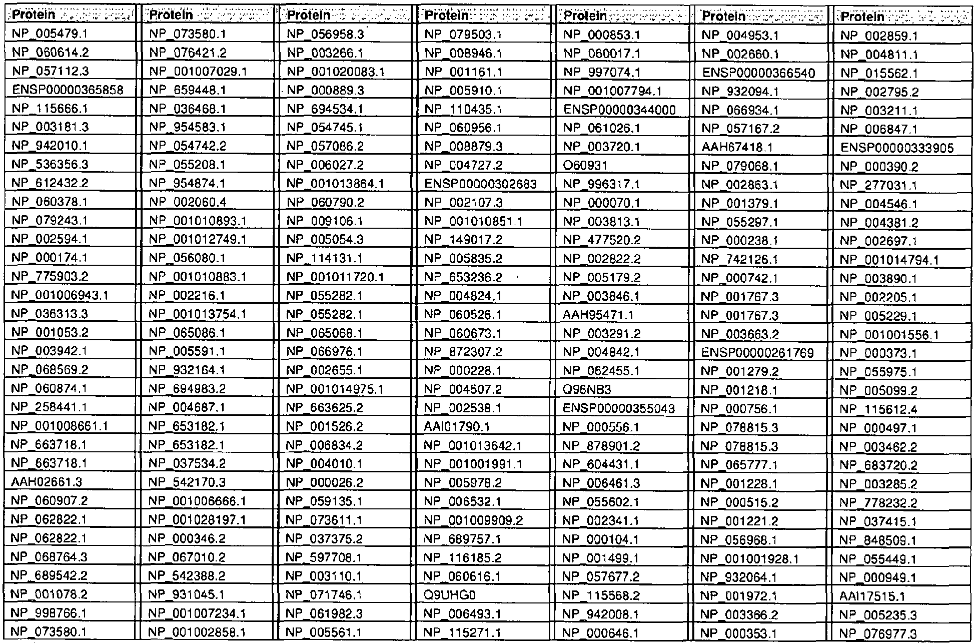

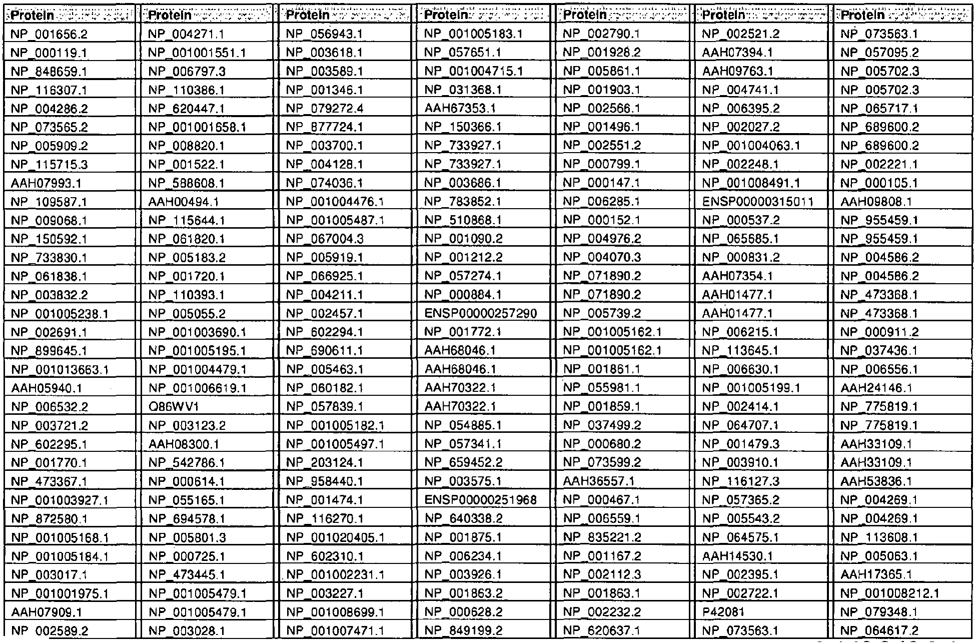

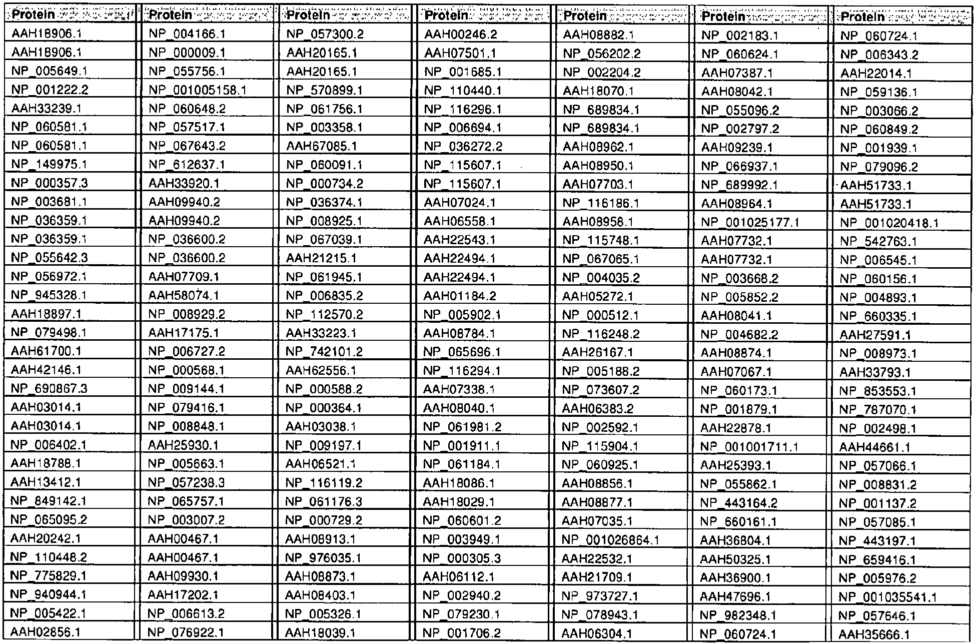

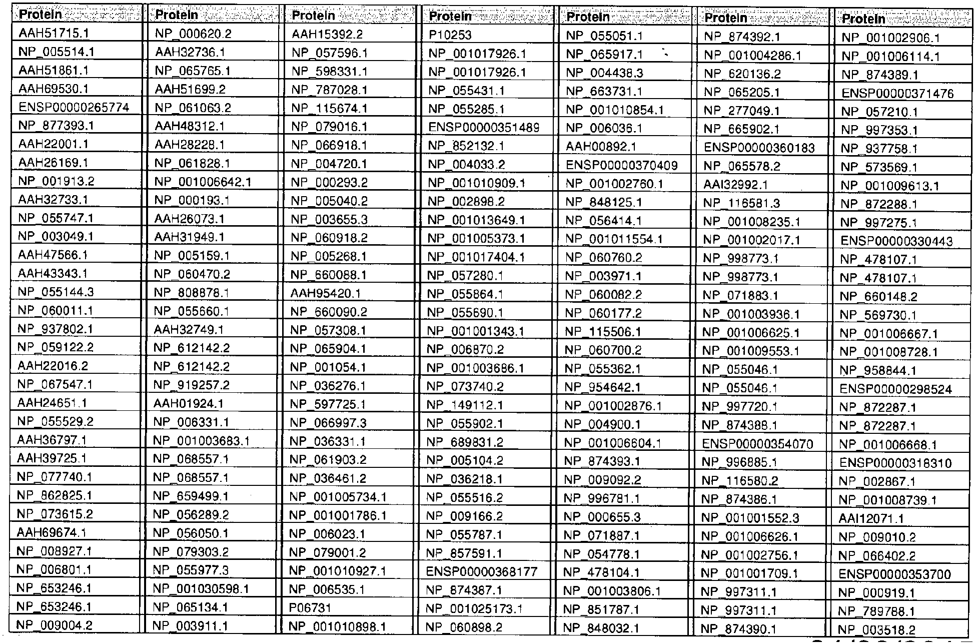

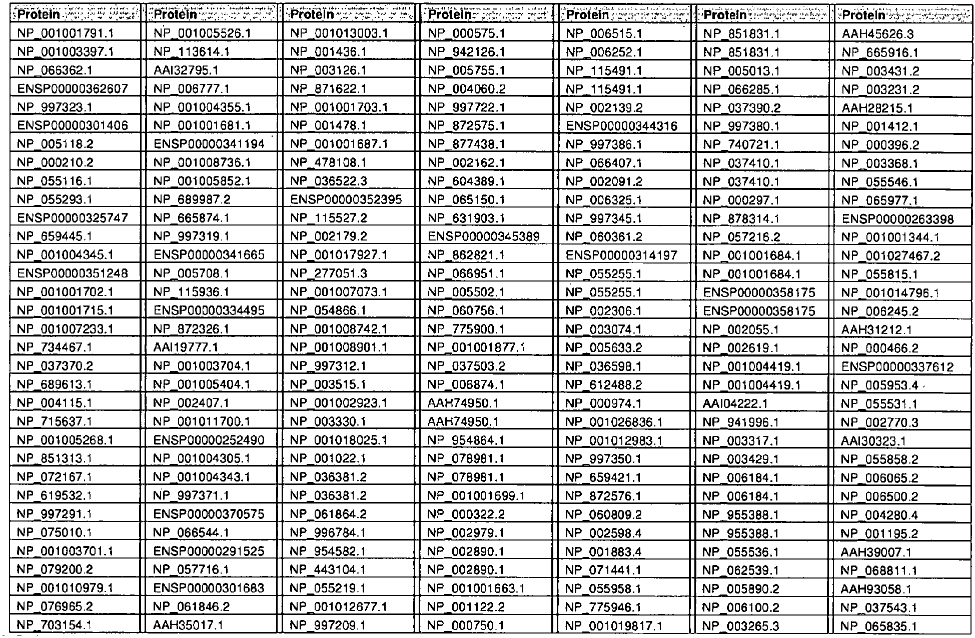

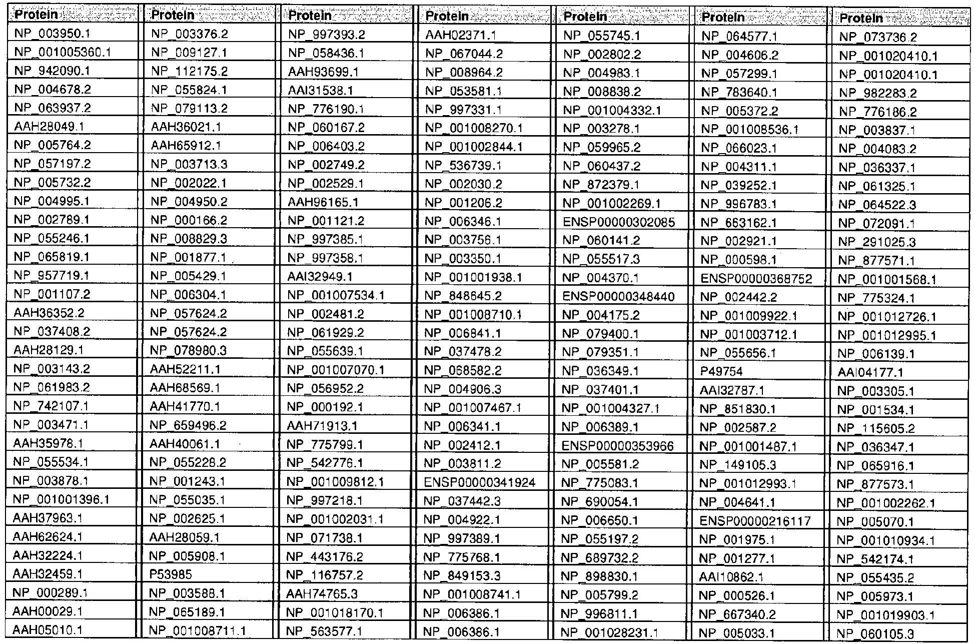

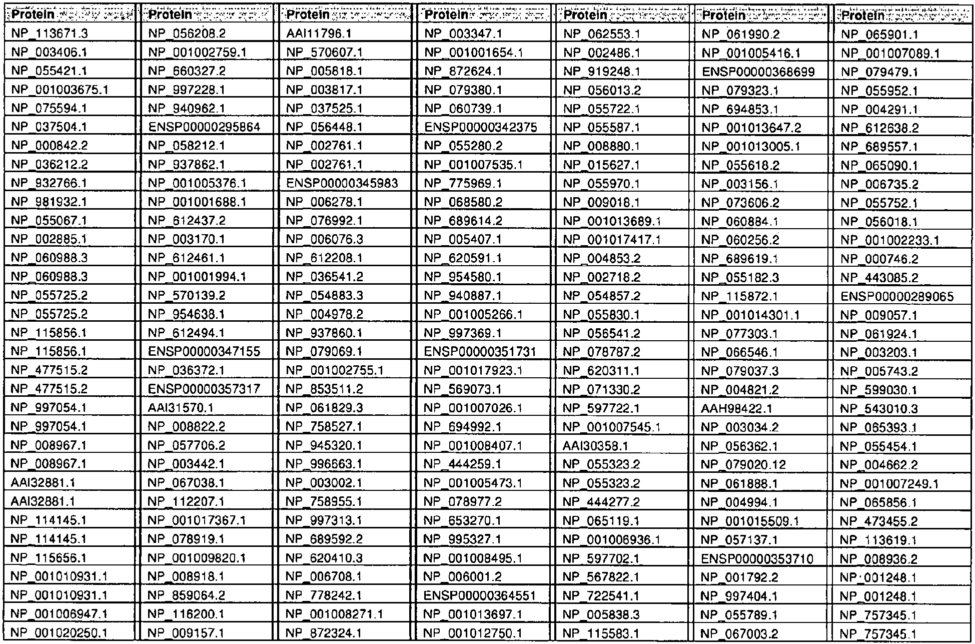

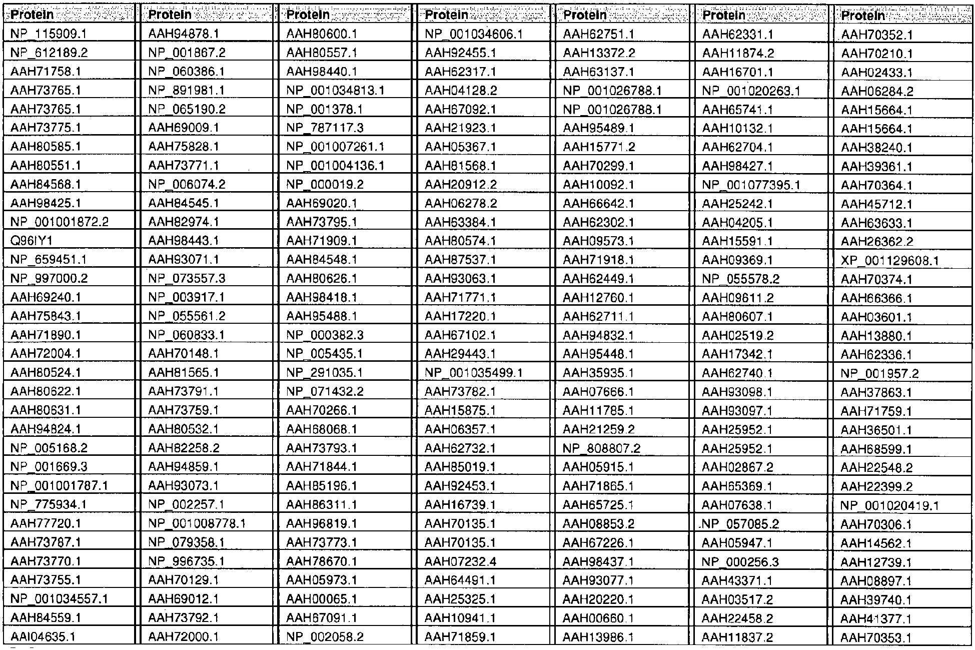

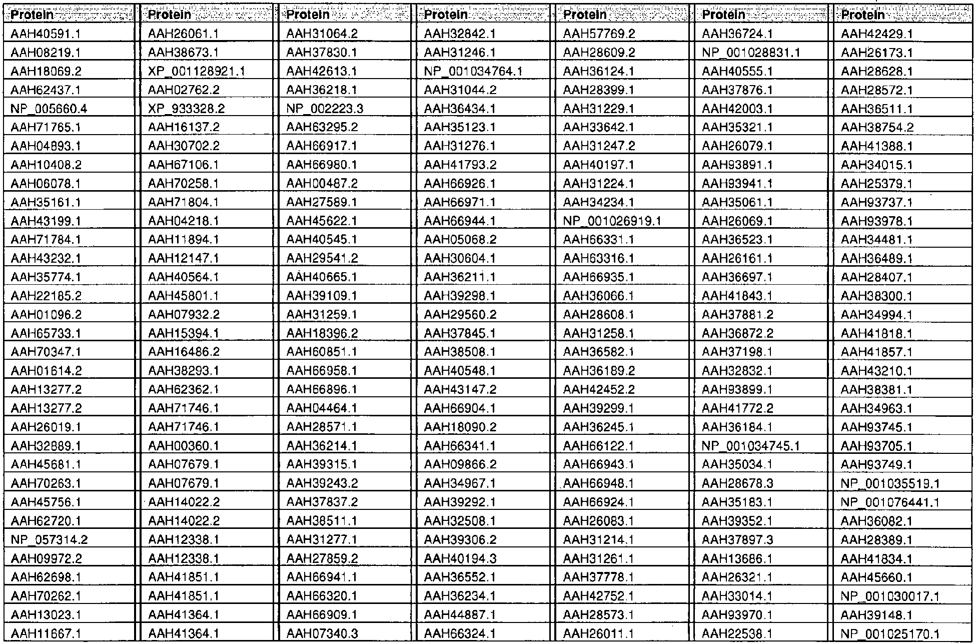

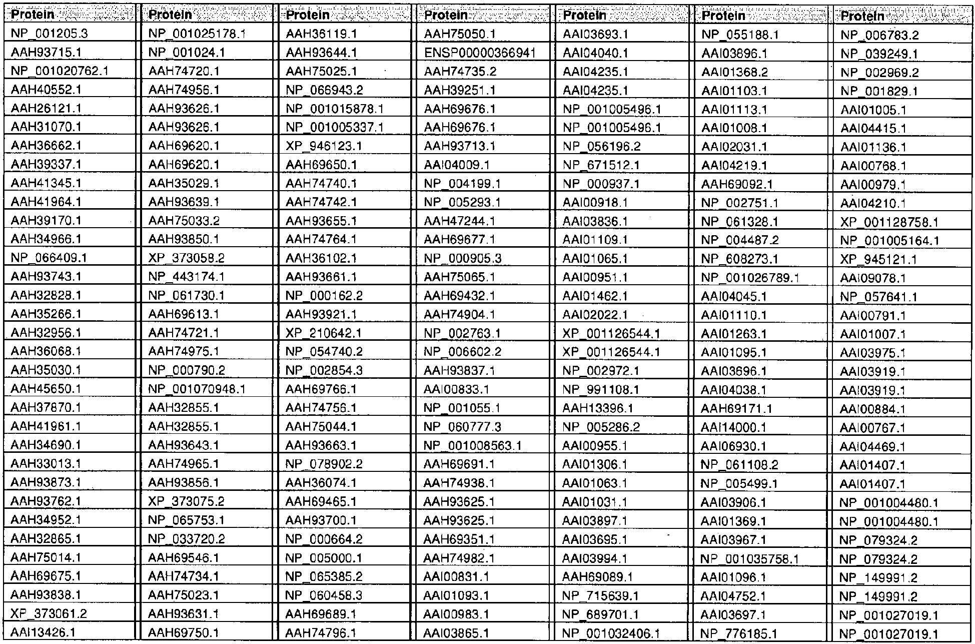

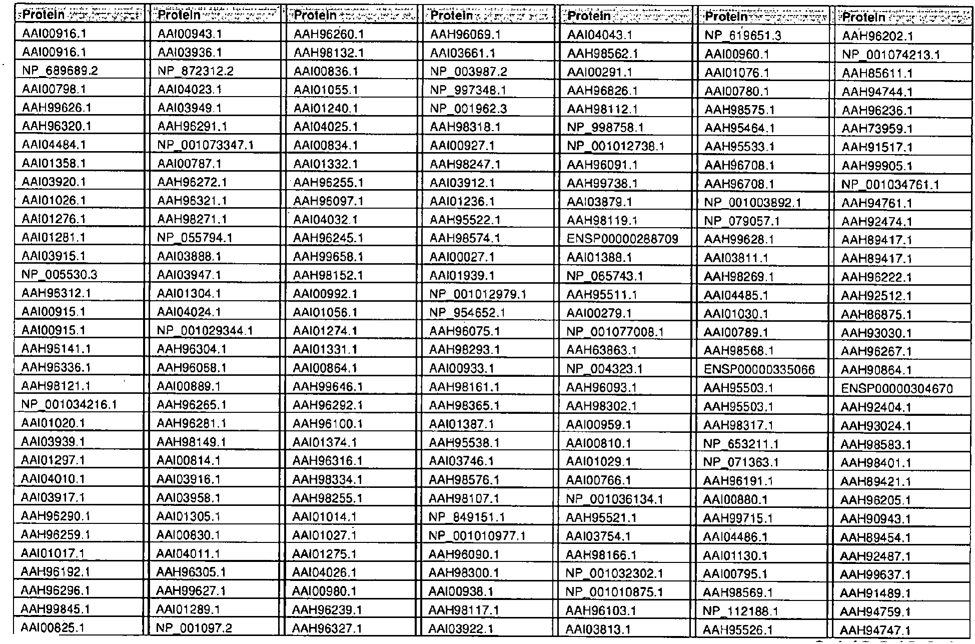

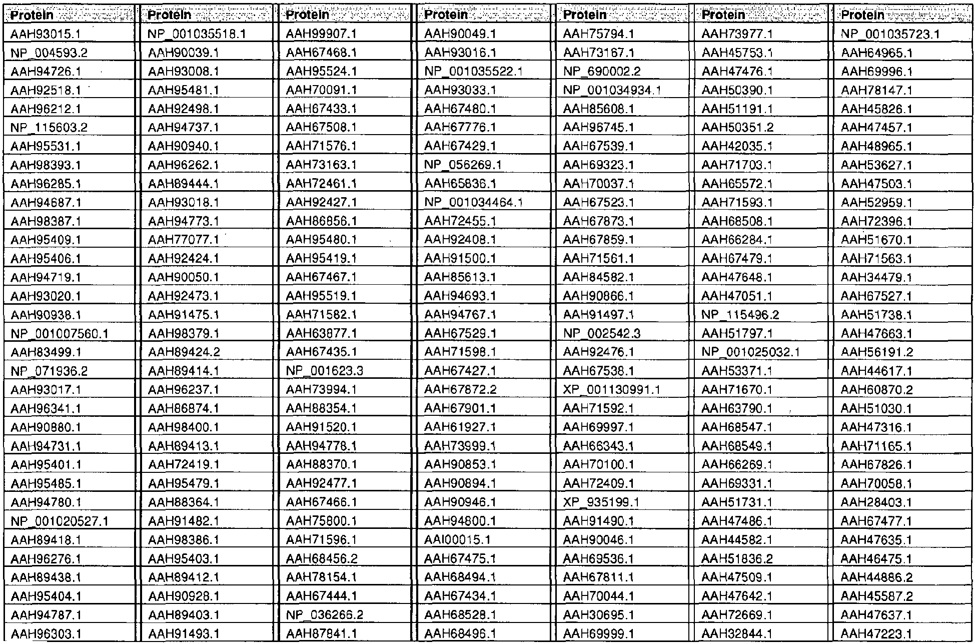

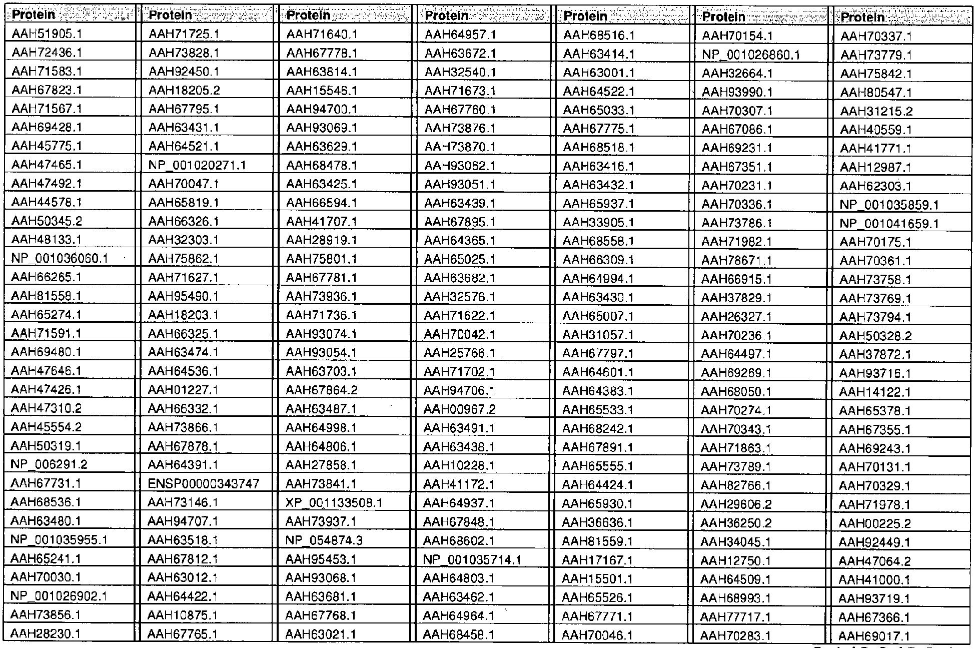

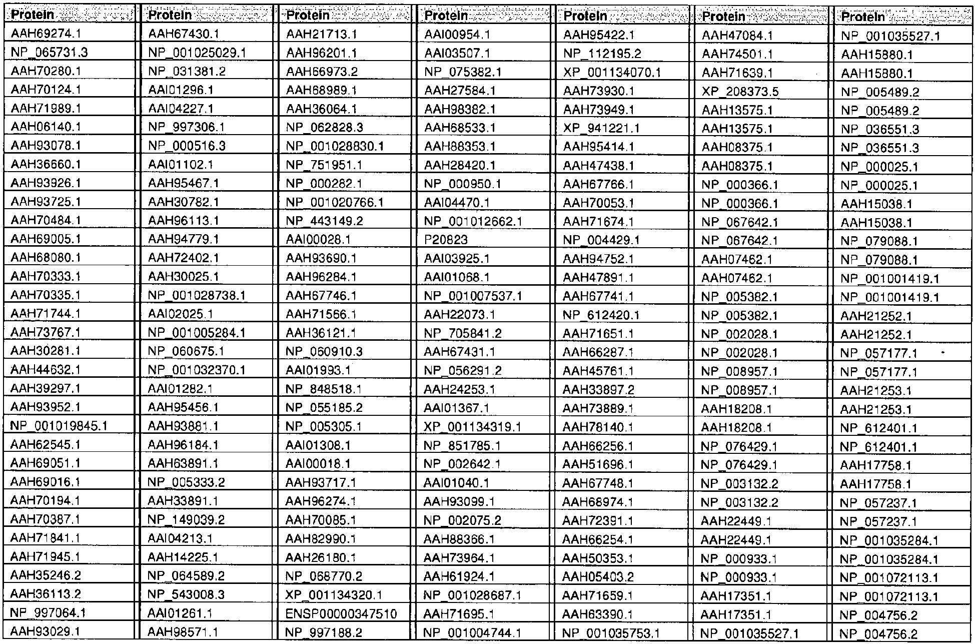

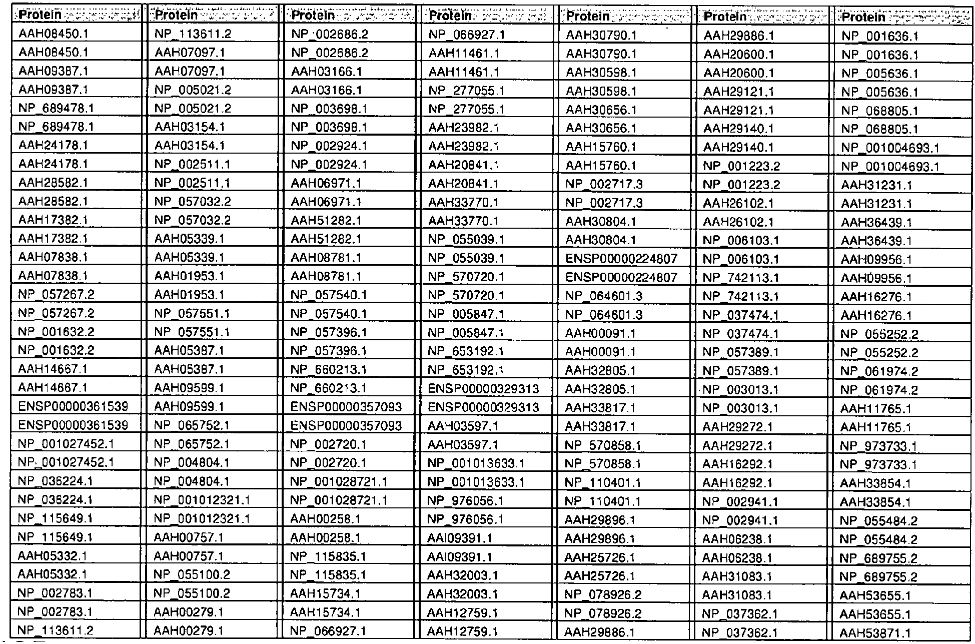

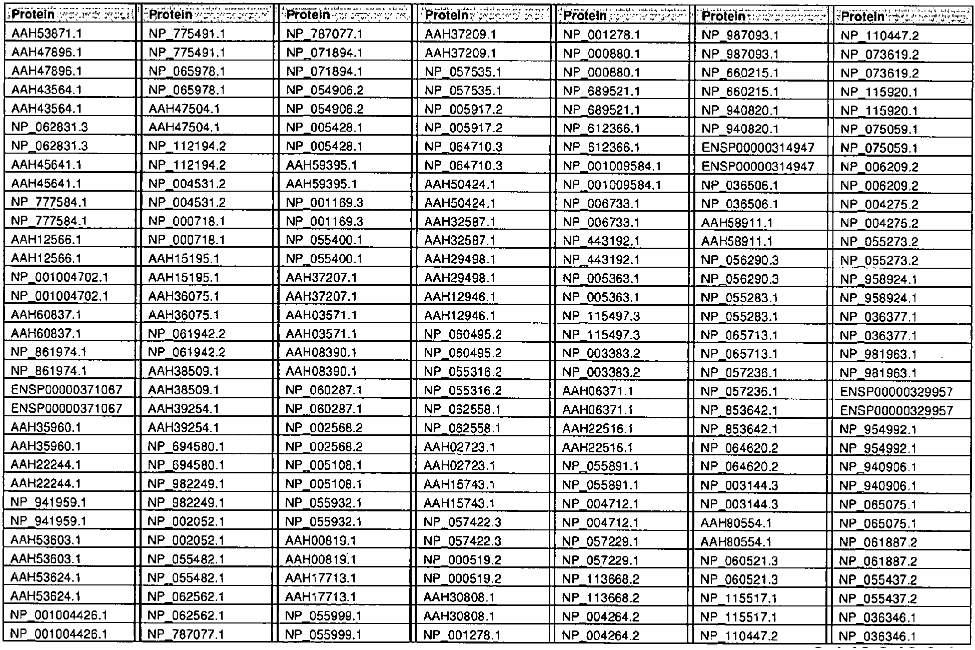

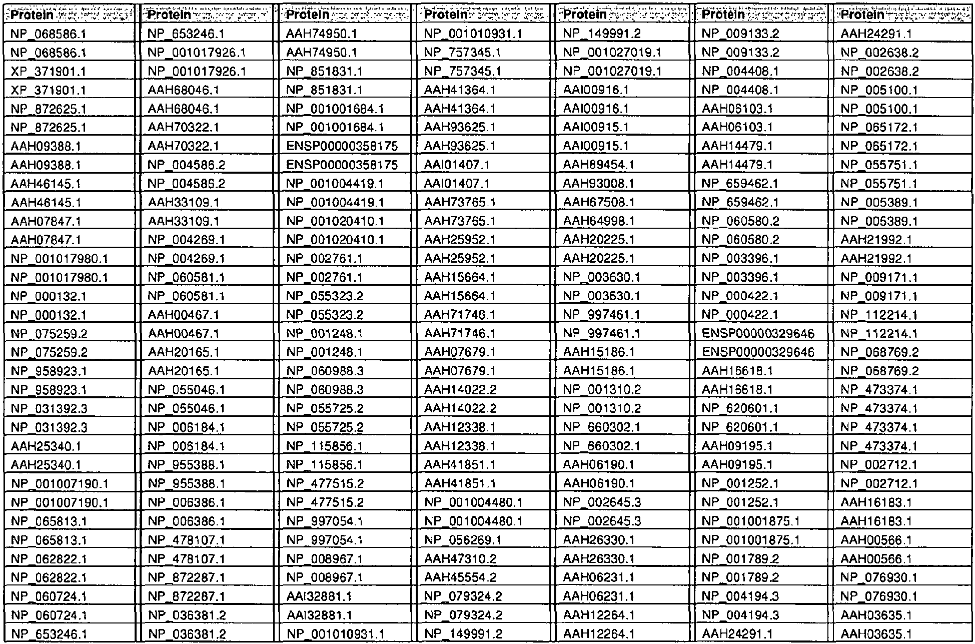

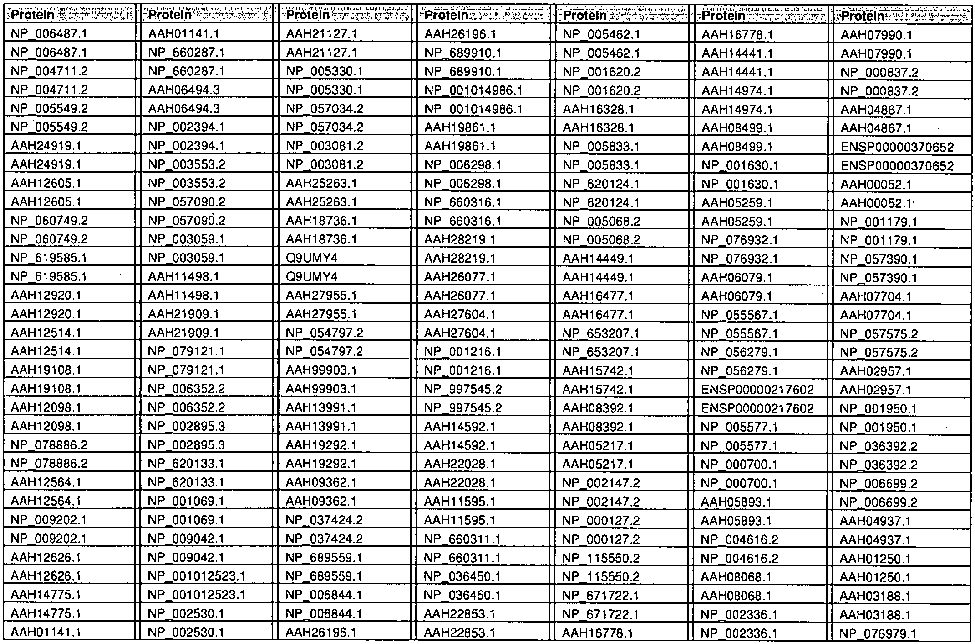

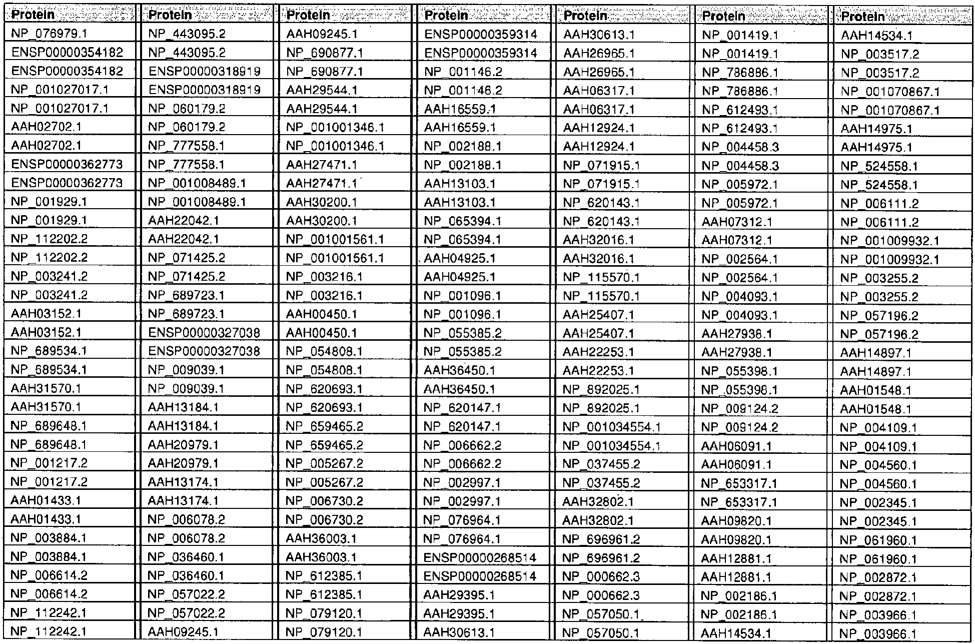

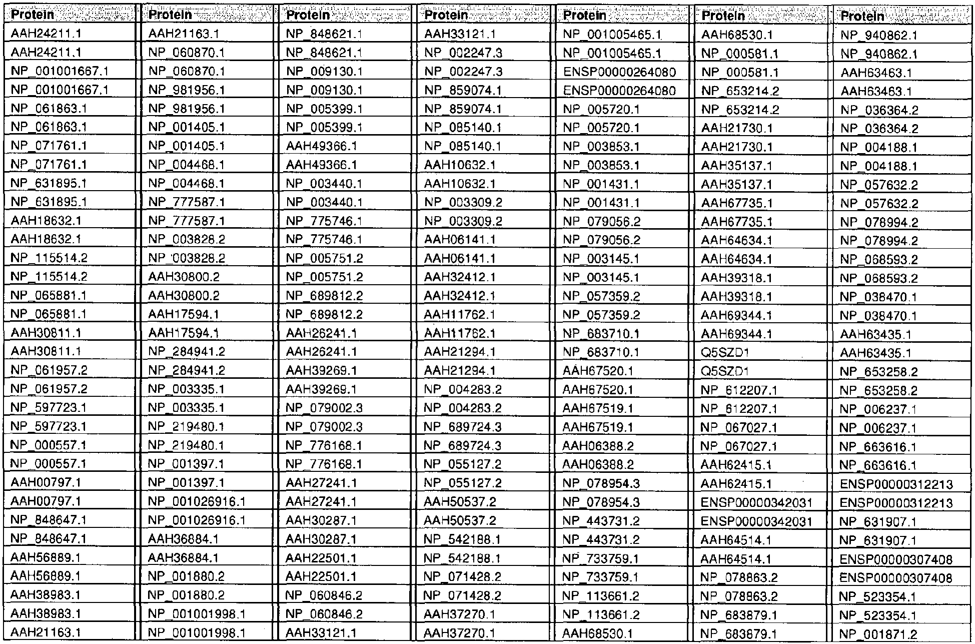

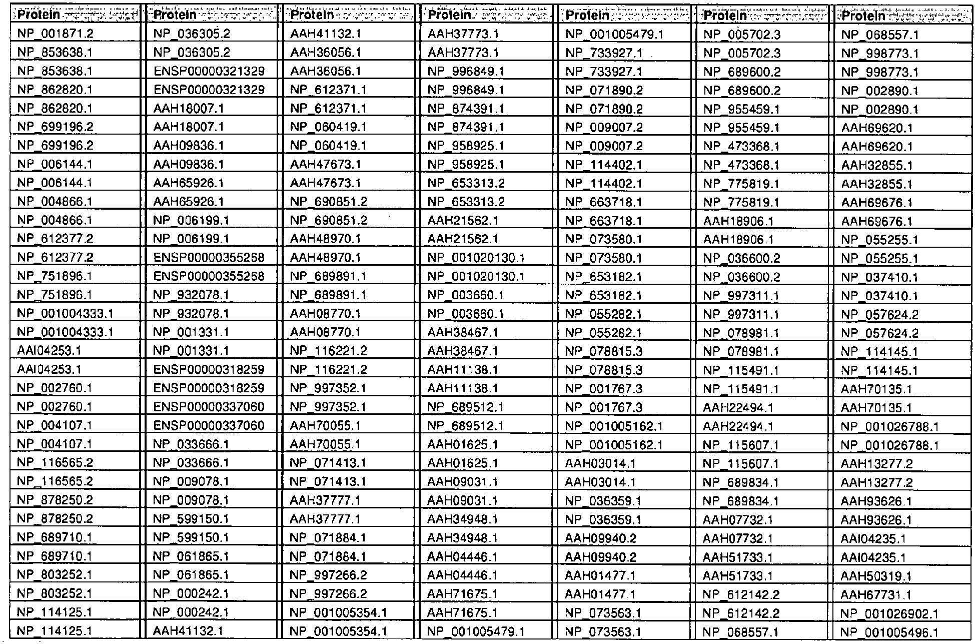

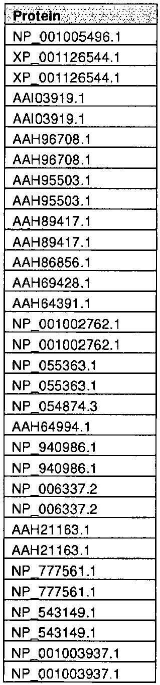

While preferred embodiments of the present invention have been shown and described herein, it will be obvious to those skilled in the art that such embodiments are provided by way of example only. It is intended that the following claims define the scope of the invention.

## Claims

1. A method of producing a library of antibodies comprising a plurality of different antibodies, the method comprising:
(a) immunizing a non-human animal with one or more antigens;
(b) isolating antibody-generating cells from the non-human animal;
(c) isolating the plurality of antibodies from the antibody-generating cells or hybridomas produced from the antibody-generating cells;
(d) screening the plurality of antibodies of (c) with a human proteome array;
(e) selecting an antibody for the library that is monospecific for a single protein on the proteome array, wherein the antibody has an A value of greater than 6 and an S value of greater than 3 for a protein or antigen when incubated on an array comprising the antigens listed in Table 5, wherein the A value is the number of standard deviations above mean signal intensity across an array comprising the antigens listed in Table 5, and the S value is the difference between the top signal and the second highest signal on the array when the signal intensities are rank ordered for the antibody against an array comprising the antigens listed in Table 5; and,
(f) adding the antibody from (e) to the library.

2. The method of claim 1, wherein each antibody of the plurality of antibodies has a binding affinity of at least 10⁻⁷ M (K_{D}) for a protein in an immunoassay.

3. The method of either of claims 1 or 2, wherein the human proteome array comprises at least 0.5% of the human proteins listed in Table 5.

4. The method of any one of claims 1-3, wherein the human proteome array comprises at least 0.5% of the proteins in the human proteome.

5. The method of any one of claims 1-4, wherein the human proteome array comprises at least 11,000 different proteins in the human proteome.

6. The method of any one of claims 1-5, wherein (d) comprises:
(i) contacting the plurality of antibodies of (c) to the human proteome array, and
(ii) determining binding between the plurality of antibodies of (i) and proteins present on the human proteome array.

7. The method of any one of claims 1-6, wherein the array comprises purified recombinant human proteins.

8. The method of any one of claims 1-7, further comprising immobilizing the plurality of antibodies to a substrate.

9. A library of antibodies comprising a plurality of different antibodies, wherein at least 10% of the plurality of antibodies is produced by the method of any one of claims 1-8.

10. A library of antibodies comprising a plurality of different antibodies wherein at least 10% of the plurality of antibodies is monospecific and has an A value of greater than 6 and an S value of greater than 3 for a protein or antigen when incubated on an array comprising the antigens listed in Table 5, wherein the A value is the number of standard deviations above mean signal intensity across an array comprising the antigens listed in Table 5, and the S value is the difference between a top signal and a second highest signal on the array when the signal intensities are rank ordered for the antibody against an array comprising the antigens listed in Table 5.

11. The library of claim 10, wherein each of the monospecific antibodies binds to a single protein on the array.

12. The library of claim 10 or 11, wherein each antibody of the plurality of antibodies individually binds a native form of a protein, is an immunoprecipitating antibody, is a monoclonal antibody, is an IgG antibody, or a combination thereof.

13. The library of any one of claims 10-12, wherein each antibody of the plurality of antibodies has a binding affinity for a protein that is within at least 20% of the binding affinity of another antibody of the plurality of antibodies for a protein.

14. The library of any one of claims 10-13, wherein the plurality of antibodies comprises at least 50 different antibodies.

15. A method comprising:
(a) screening a plurality of different antibodies with a human proteome array; and,
(b) selecting an antibody that is monospecific for a single protein on the proteome array, wherein the antibody has an *A* value of greater than 6 and an S value of greater than 3 for a protein or antigen when incubated on an array comprising the antigens listed in Table 5, wherein the *A* value is the number of standard deviations above mean signal intensity across an array comprising the antigens listed in Table 5, and the S value is the difference between a top signal and a second highest signal on the array when the signal intensities are rank ordered for the antibody against an array comprising the antigens listed in Table 5.

## Patentansprüche

1. Verfahren zum Herstellen einer Bibliothek von Antikörpern, die mehrere verschiedene Antikörper umfasst, wobei das Verfahren Folgendes umfasst:
(a) Immunisieren eines nicht-humanen Tieres mit einem oder mehreren Antigenen;
(b) Isolieren von antikörpererzeugenden Zellen aus dem nicht-humanen Tier;
(c) Isolieren der mehreren Antikörper aus den antikörpererzeugenden Zellen oder Hybridomen, die aus den antikörpererzeugenden Zellen hergestellt wurden;
(d) Screenen der mehreren Antikörper aus (c) mit einem humanen Proteom-Array;
(e) Auswählen eines Antikörpers für die Bibliothek, der monospezifisch für ein einziges Protein auf dem Proteom-Array ist, wobei der Antikörper einen *A-*Wert von mehr als 6 und einen S-Wert von mehr als 3 für ein Protein oder Antigen aufweist, wenn dies auf einem Array, das die in Tabelle 5 aufgeführten Antigene umfasst, inkubiert ist, wobei der *A*-Wert die Zahl der Standardabweichungen oberhalb der mittleren Signalintensität über ein Array ist, das die in Tabelle 5 aufgeführten Antigene umfasst, und der S-Wert die Differenz zwischen dem höchsten Signal und dem zweithöchsten Signal auf dem Array ist, wenn die Signalintensitäten für den Antikörper nach Rangfolge gegen ein Array, das die in Tabelle 5 aufgeführten Antigene umfasst, geordnet sind; und
(f) Hinzufügen des Antikörpers aus (e) zu der Bibliothek.

2. Verfahren nach Anspruch 1, wobei jeder Antikörper der mehreren Antikörper eine Bindungsaffinität von mindestens 10⁻⁷ M (K_{D}) für ein Protein in einem Immunoassay aufweist.

3. Verfahren nach einem der Ansprüche 1 oder 2, wobei das humane Proteom-Array mindestens 0,5 % der humanen Proteine, die in Tabelle 5 aufgeführt sind, umfasst.

4. Verfahren nach einem der Ansprüche 1-3, wobei das humane Proteom-Array mindestens 0,5 % der Proteine in dem humanen Proteom umfasst.

5. Verfahren nach einem der Ansprüche 1-4, wobei das humane Proteom-Array mindestens 11.000 verschiedene Proteine in dem humanen Proteom umfasst.

6. Verfahren nach einem der Ansprüche 1-5, wobei (d) Folgendes umfasst:
(i) Inberührungbringen mehrerer Antikörper aus (c) mit dem humanen Proteom-Array,
und
(ii) Bestimmen der Bindung zwischen den mehreren Antikörpern aus (i) und Proteinen, die auf dem humanen Proteom-Array vorliegen.

7. Verfahren nach einem der Ansprüche 1-6, wobei das Array aufgereinigte rekombinante humane Proteine umfasst.

8. Verfahren nach einem der Ansprüche 1-7, das ferner das Immobilisieren der mehreren Antikörper auf einem Substrat umfasst.

9. Bibliothek von Antikörpern, die mehrere verschiedene Antikörper umfasst, wobei mindestens 10 % der mehreren Antikörper durch das Verfahren nach einem der Ansprüche 1-8 hergestellt werden.

10. Bibliothek von Antikörpern, die mehrere verschiedene Antikörper umfasst, wobei mindestens 10 % der mehreren Antikörper monospezifisch sind und einen *A*-Wert von mehr als 6 und einen S-Wert von mehr als 3 für ein Protein oder Antigen aufweist, wenn dies auf einem Array, das die in Tabelle 5 aufgeführten Antigene umfasst, inkubiert ist, wobei der *A*-Wert die Zahl der Standardabweichungen oberhalb der mittleren Signalintensität über ein Array ist, das die in Tabelle 5 aufgeführten Antigene umfasst, und der *S*-Wert die Differenz zwischen dem höchsten Signal und dem zweithöchsten Signal auf dem Array ist, wenn die Signalintensitäten für den Antikörper nach Rangfolge gegen ein Array, das die in Tabelle 5 aufgeführten Antigene umfasst, geordnet sind.

11. Bibliothek nach Anspruch 10, wobei jeder der monospezifischen Antikörper an ein einziges Protein auf dem Array bindet.

12. Bibliothek nach Anspruch 10 oder 11, wobei jeder Antikörper der mehreren Antikörper individuell eine native Form eines Proteins bindet, ein immunpräzipitierender Antikörper ist, ein monoklonaler Antikörper ist, ein IgG-Antikörper ist oder eine Kombination davon ist.

13. Bibliothek nach einem der Ansprüche 10-12, wobei jeder Antikörper der mehreren Antikörper eine Bindungsaffinität für ein Protein aufweist, die innerhalb mindestens 20 % der Bindungsaffinität eines anderen Antikörpers der mehreren Antikörper für ein Protein liegt.

14. Bibliothek nach einem der Ansprüche 10-13, wobei die mehreren Antikörper mindestens 50 verschiedene Antikörper umfassen.

15. Verfahren, das Folgendes umfasst:
(a) Screenen mehrerer verschiedener Antikörper mit einem humanen Proteom-Array; und
(b) Auswählen eines Antikörpers, der monospezifisch für ein einziges Protein auf dem Proteom-Array ist, wobei der Antikörper einen *A*-Wert von mehr als 6 und einen S-Wert von mehr als 3 für ein Protein oder Antigen aufweist, wenn dies auf einem Array, das die in Tabelle 5 aufgeführten Antigene umfasst, inkubiert ist, wobei der *A*-Wert die Zahl der Standardabweichungen oberhalb der mittleren Signalintensität über ein Array ist, das die in Tabelle 5 aufgeführten Antigene umfasst, und der S-Wert die Differenz zwischen dem höchsten Signal und dem zweithöchsten Signal auf dem Array ist, wenn die Signalintensitäten für den Antikörper nach Rangfolge gegen ein Array, das die in Tabelle 5 aufgeführten Antigene umfasst, geordnet sind.

## Revendications

1. Procédé de production d'une banque d'anticorps comprenant une pluralité d'anticorps différents, le procédé comprenant :
(a) l'immunisation d'un animal non humain avec un ou plusieurs antigènes ;
(b) l'isolement de cellules générant des anticorps à partir de l'animal non humain ;
(c) l'isolement de la pluralité d'anticorps à partir des cellules générant des anticorps ou d'hybridomes produits à partir des cellules générant des anticorps ;
(d) le criblage de la pluralité d'anticorps de (c) avec un ensemble protéome humain ;
(e) la sélection d'un anticorps pour la banque qui est monospécifique pour une protéine unique sur l'ensemble protéome, dans lequel l'anticorps a une valeur *A* supérieure à 6 et une valeur S supérieure à 3 pour une protéine ou un antigène lorsqu'il est incubé sur un ensemble comprenant les antigènes énumérés dans le tableau 5, dans lequel la valeur *A* est le nombre d'écarts-types au-dessus de la moyenne d'intensité de signal à travers un ensemble comprenant les antigènes énumérés dans le tableau 5, et la valeur *S* est la différence entre le signal supérieur et le second signal le plus élevé sur l'ensemble lorsque les intensités de signal sont classées par ordre de rang pour l'anticorps par rapport à un ensemble comprenant les anticorps énumérés dans le tableau 5 ; et,
(f) l'ajout de l'anticorps provenant de (e) à la banque.

2. Procédé selon la revendication 1, dans lequel chaque anticorps de la pluralité d'anticorps a une affinité de liaison d'au moins 10⁻⁷ M(K_{D}) pour une protéine dans un immunodosage.

3. Procédé selon l'une ou l'autre des revendications 1 ou 2, dans lequel l'ensemble protéome humain comprend au moins 0,5 % des protéines humaines énumérées dans le tableau 5.

4. Procédé selon l'une quelconque des revendications 1 à 3, dans lequel l'ensemble protéome humain comprend au moins 0,5 % des protéines du protéome humain.

5. Procédé selon l'une quelconque des revendications 1 à 4, dans lequel l'ensemble protéome humain comprend au moins 11 000 protéines différentes du protéome humain.

6. Procédé selon l'une quelconque des revendications 1 à 5, dans lequel (d) comprend :
(i) la mise en contact de la pluralité d'anticorps de (c) avec l'ensemble protéome humain, et
(ii) la détermination de liaison entre la pluralité d'anticorps de (i) et de protéines présentes sur l'ensemble protéome humain.

7. Procédé selon l'une quelconque des revendications 1 à 6, dans lequel l'ensemble comprend des protéines humaines recombinantes purifiées.

8. Procédé selon l'une quelconque des revendications 1 à 7, comprenant en outre l'immobilisation de la pluralité d'anticorps sur un substrat.

9. Banque d'anticorps comprenant une pluralité d'anticorps différents, dans laquelle au moins 10 % de la pluralité d'anticorps sont produits par le procédé de l'une quelconque des revendications 1 à 8.

10. Banque d'anticorps comprenant une pluralité d'anticorps différents, dans laquelle au moins 10 % de la pluralité d'anticorps sont monospécifiques et ont une valeur *A* supérieure à 6 et une valeur *S* supérieure à 3 pour une protéine ou un antigène lorsqu'ils sont incubés sur un ensemble comprenant les antigènes énumérés dans le tableau 5, dans laquelle la valeur *A* est le nombre d'écarts-types au-dessus de la moyenne d'intensité de signal à travers un ensemble comprenant les antigènes énumérés dans le tableau 5, et la valeur *S* est la différence entre un signal supérieur et un second signal le plus élevé sur l'ensemble lorsque les intensités de signal sont classées par ordre de rang pour l'anticorps par rapport à un ensemble comprenant les anticorps énumérés dans le tableau 5.

11. Banque selon la revendication 10, dans laquelle chacun des anticorps monospécifiques se lie à une protéine unique sur l'ensemble.

12. Banque selon la revendication 10 ou 11, dans laquelle chaque anticorps de la pluralité d'anticorps se lie individuellement à une forme native d'une protéine, est un anticorps d'immunoprécipitation, est un anticorps monoclonal, est un anticorps d'IgG, ou une combinaison de ceux-ci.

13. Banque selon l'une quelconque des revendications 10 à 12, dans laquelle chaque anticorps de la pluralité d'anticorps a une affinité de liaison pour une protéine qui est dans la limite d'au moins 20 % de l'affinité de liaison d'un autre anticorps de la pluralité d'anticorps pour une protéine.

14. Banque selon l'une quelconque des revendications 10 à 13, dans laquelle la pluralité d'anticorps comprend au moins 50 anticorps différents.

15. Procédé comprenant :
(a) le criblage d'une pluralité d'anticorps différents avec un ensemble protéome humain ; et,
(b) la sélection d'un anticorps qui est monospécifique pour une protéine unique sur l'ensemble protéome, dans lequel l'anticorps a une valeur *A* supérieure à 6 et une valeur S supérieure à 3 pour une protéine ou un antigène lorsqu'il est incubé sur un ensemble comprenant les antigènes énumérés dans le tableau 5, dans lequel la valeur *A* est le nombre d'écarts-types au-dessus de la moyenne d'intensité de signal à travers un ensemble comprenant les antigènes énumérés dans le tableau 5, et la valeur S est la différence entre un signal supérieur et un second signal le plus élevé sur l'ensemble lorsque les intensités de signal sont classées par ordre de rang pour l'anticorps par rapport à un ensemble comprenant les anticorps énumérés dans le tableau 5.
